# EUROPÄISCHE PATENTSCHRIFT

(11) **EP 2 104 675 B1**
(45) Veröffentlichungstag und Bekanntmachung des Hinweises auf die Patenterteilung: **31.10.2012**
(21) Anmeldenummer: 07857094.2
(22) Anmeldetag: 21.12.2007
(51) Int. Cl.: C07D 471/04

(54) **SUBSTITUIERTE 5H-PYRIMIDOL[5,4-B]INDOLE, VERFAHREN ZU IHRER HERSTELLUNG UND IHRE VERWENDUNG ZUR BEHANDLUNG VON NICHT-SOLIDEN MALIGNEN TUMOREN DES BLUTBILDENDEN SYSTEMS**
SUBSTITUTED 5H-PYRIMIDOL[5,4-B]INDOLES, METHOD FOR THE PRODUCTION THEREOF, AND USE THEREOF FOR TREATING NON-SOLID MALIGNANT TUMORS OF THE BLOOD-PRODUCING SYSTEM
5H-PYRIMIDOL[5,4-B]INDOLES SUBSTITUÉS, PROCÉDÉS POUR LEUR FABRICATION, ET LEUR UTILISATION POUR LE TRAITEMENT DE TUMEURS MALIGNES NON SOLIDES DU SYSTÈME SANGUIN

(30) Priorität: 22.12.2006 DE 102006062203
(43) Veröffentlichungstag der Anmeldung: 30.09.2009
(73) Patentinhaber: The Medicines Company (Leipzig) GmbH, 04103 Leipzig (DE)
(72) Erfinder: REICHELT, Claudia, 04299 Leipzig (DE); SCHULZE, Alexander, 04886 Grosstreben Zwethau OT Dautzschen (DE); DAGHISH, Mohammed, 04107 Leipzig (DE); LUDWIG, Alexander, 04275 Leipzig (DE); HEINICKE, Jochen, 04229 Leipzig (DE); HERRMANN, Konrad, 04668 Grimma (DE); SCHUSTER, Maj, 04229 Leipzig (DE); LETSCHERT, Sven, 04275 Leipzig (DE); MUGRIDGE, Kenneth, 04109 Leipzig (DE); DeANGELO, Joseph, Carrboro, NC 27510 (US)
(74) Vertreter: Schüssler, Andrea
(86) Internationale Anmeldenummer: PCT/EP2007/011387
(87) Internationale Veröffentlichungsnummer: WO 2008/077631

(56) Entgegenhaltungen:
- MERINO I ET AL: "Synthesis and anti-HIV-1 activities of new pyrimido[5,4-b]indoles" IL FARMACO, ROME, IT, Bd. 54, 1999, Seiten 255-264, XP002388500 ISSN: 0014-827X

## Beschreibung

Die Erfindung betrifft Derivate der allgemeinen Formel 1

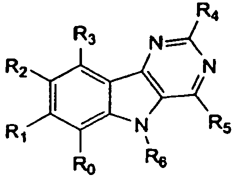

Verfahren zu ihrer Herstellung, pharmazeutische Zubereitungen, die diese Verbindungen und/oder daraus herstellbare physiologisch verträgliche Salze und/oder Solvate enthalten, sowie die pharmazeutische Verwendung dieser Verbindungen, deren Salze oder Solvate als Induktoren der Apoptose bei nicht-soliden malignen Tumoren des blutbildenden Systems, insbesondere bei Leukämien und Lymphomen, ganz besonders bei leukämischen B Lymphozyten.

Nicht-solide maligne Tumoren des blutbildenden Systems sind eine immer häufiger auftretende Krebserkrankung in den Industrienationen geworden.

Zu den nicht-soliden malignen Tumoren des blutbildenden Systems zählen Lymphome und alle Leukämieformen.

Die chronisch lymphatische Leukämie vom B-Zelltyp (B-CLL) ist charakterisiert durch eine fortschreitende maligne Akkumulation kleiner CD5⁺, CD19⁺, CD23⁺ B-CLL Zellen im peripheren Blut, im Knochenmark und in den sekundären lymphatischen Organen.

Die heutige Standardtherapie der B-CLL ist palliativ und wird hauptsächlich mit den Zytostatika Chlorambucil oder Fludarabin durchgeführt. Beim Auftreten von Rezidiven wird oft eine Kombinationstherapie mit Fludarabin, Cyclophosphamid in Kombination mit Rituximab (monoklonaler Antikörper gegen CD20) oder Campath (monoklonaler Antikörper gegen CD52) eingeleitet.

Die "first-line" Standardtherapie der B-CLL ist heute die Chemotherapie mit dem Purin-Analogon Fludarabin (Fludara^{®}), entweder als Mono- oder als Kombinationstherapie. Fludarabin zeigt in klinischen Studien bei CLL-Patienten im Vergleich zu dem bisher gängigen Kombinationsschema Cyclophosphamid, Adriamycin und Predniso-Ion (CAP) signifikant höhere Remissionsraten (60% versus 40%) und längere Überlebenszeit (1300/1000Tage).

Das Nebenwirkungsprofil von Fludarabin ist im Vergleich zu Campath weniger stark ausgeprägt, aber doch als erheblich einzuschätzen.

Alle bis heute zugelassenen Medikamente zur Behandlung von Tumoren des blutbildenden Systems weisen Nachteile auf, insbesondere Lymphopenie, Schüttelfrost, Fieber, Neutropenie, Emesis und dergleichen.

Die Ursachen sind unterschiedlich, lassen sich aber auf eine prinzipielle Unzulänglichkeit der eingesetzten Medikamente zurückführen. Diese besteht darin, dass die Wirkstoffe zu wenig zwischen den malignen Leukämiezellen und den lebensnotwendigen anderen (Blut) Zellen differenzieren können. Das liegt daran, dass die Reaktionswege der Wirkstoffe nicht hinreichend aufgeklärt sind. So kommt es dazu, dass die Medikamente Mutationen in Tumorsuppressorgenen induzieren oder selektionieren und dadurch Resistenzen auslösen und auch nichtleukämische, d.h. gesunde Blutzellen, aber auch Zellen anderen Ursprunges angreifen.

Am Benzoring unsubstituierte (R₀=R₁=R₂=R₃=H) tricyclische Verbindung der Formel (1) sind verschiedentlich in der Literatur beschrieben worden (vgl. hierzu Chemical Abstracts Services, Registry, STN und andere Datenbanken). Des weiteren wurde von Merino et al. die Synthese und anti-HIV Aktivität von Pyrimido [5,4-b] indolen beschrieben (Merino et al., Il Farmaco, Bd.54, 1999, S. 255-264).

Die Erfindung hat das Ziel, neuartige und wirksamere Verbindungen mit einem geringeren Nebenwirkungsprofil für die Behandlung von Patienten mit nicht-soliden malignen Tumoren des blutbildenden Systems, insbesondere von Patienten mit chronischer lymphatischer Leukämie, zur Verfügung zu stellen.

Die Erfindung hat die Aufgabe, Arzneimittel zu entwickeln, die effizient eine Apoptose in den Tumorzellen induzieren und gesunde andere (Blut)Zellen signifikant weniger oder überhaupt nicht schädigen.

Die Aufgabe wird erfindungsgemäß dadurch gelöst, dass Wirkstoffe hergestellt und charakterisiert werden, die der allgemeinen Formel (1) entsprechen.

### Beschreibung der Erfindung

Die Erfindung betrifft Verbindungen der allgemeinen Formel 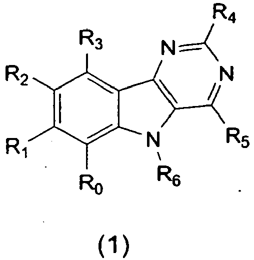 worin bedeuten:
R₀, R₁ bzw. R₂
   - L_{A}-A-L_{B}-B, worin bedeuten:
      L_{A}:
         - Einfachbindung,
         - NR^{#}, O, S, S(O), S(O)₂, S(O)₂-O, O-S(O)₂, -CHR^{§}-, -CH₂-O-, -CH₂-CH₂-O-, -O-CH₂, -O-CH₂-CH₂-, C=O,
      A:
         - Wasserstoff,
         - C₁₋₆Alkyl (ggf. mit R⁵ substituiert),
         - C₂₋₆Alkenyl (ggf. mit R^{§} substituiert),
         - C₂₋₆Alkinyl (ggf. mit R^{§} substituiert),
         - Chlor, Brom, lod,
         - Azido, Hydrazino,
         - Phenyl, ggf. ein-, zwei- oder dreifach mit unabhängig voneinander ausgewählten Resten R^{§} substituiert
         - ein mono- oder bicyclischer gesättigter oder ein- oder mehrfach ungesättigter Heterocyclus mit 4 - 14 Ringatomen, darunter 1 - 5 Heteroatome (vorzugsweise N, O, S), der ggf. einzwei- oder dreifach mit unabhängig voneinander ausgewählten Resten R^{§} und/ oder ein oder mehreren Sauerstoffatomen substituiert sein kann.
         und für den Fall, dass A nicht weiter substituierbar ist, L_{B} und B gegenstandslos sind,
      L_{A}-A sowie L_{A}-A-L_{B} jeweils gemeinsam auch eine Einfachbindung bedeuten können
      L_{B}:
         - Einfachbindung,
         - NR^{#}, O, S, S(O), S(O)₂, S(O)₂-O, O-S(O)₂, -CHR^{§}-, -CH₂-O-, -CH₂-CH₂-O-, -O-CH₂, -O-CH₂-CH₂-, C=O,
         - folgende funktionelle Gruppen: 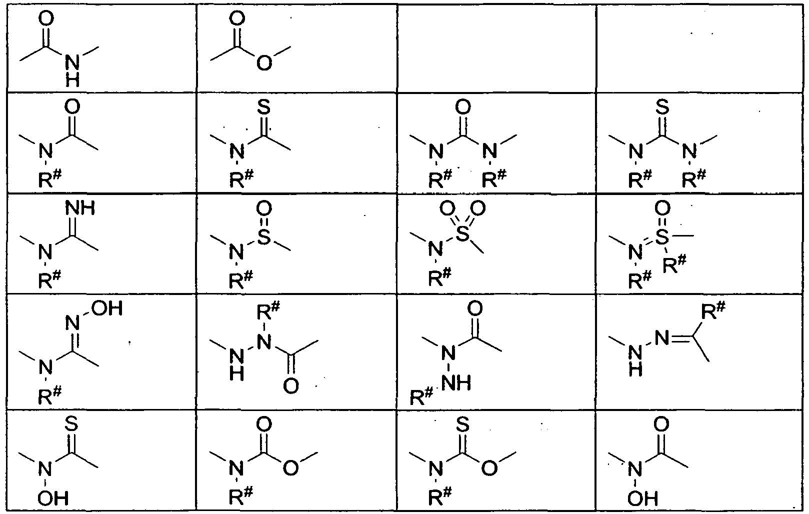
      B:
         - Wasserstoff
         - Alkyl, ggf. mit R^{§} substituiert
         - C₂₋₆Alkenyl (ggf. mit R^{§} substituiert),
         - C₂₋₆Alkinyl (ggf. mit R^{§} substituiert),
         - Arylalkyl- mit C₆₋₁₂Aryl und C₁₋₅ Alkyl, (Alkyl und/oder Aryl ggf. mit R^{§} substituiert),
         - Alkyl, ein- oder mehrfach substituiert mit mono- oder bicyclischen gesättigten oder ein- oder mehrfach ungesättigten Heterocyclen mit 4 - 14 Ringatomen darunter 1 - 5 Heteroatome (vorzugsweise N, O, S), die ggf. ein- zwei- oder dreifach mit unabhängig voneinander ausgewählten Resten R^{§} und/ oder ein oder mehreren Sauerstoffatomen substituiert sein können.
         - Aryl, insb. Phenyl, ggf. mit R^{§} substituiert
         - ein mono- oder bicyclischer gesättigter oder ein- oder mehrfach ungesättigter Heterocyclus mit 4 - 14 Ringatomen, darunter 1 - 5 Heteroatome (vorzugsweise N, O, S), der ggf. ein- zwei- oder dreifach mit unabhängig voneinander ausgewählten Resten R^{§} und/ oder ein oder mehreren Sauerstoffatomen substituiert sein kann
      R^{#} - Wasserstoff, Alkyl (ggf. mit R^{§} substituiert)
R₃
   - Wasserstoff,
   - C₁₋₆Alkyl, geradkettig, verzweigt oder C₃₋₆Alkyl auch cyclisch sowie ggf. ein-, zwei- oder dreifach mit unabhängig voneinander ausgewählten Resten R^{§} substituiert,
   - R^{§}
   - C₁₋₆ Alkoxy, geradkettig, verzweigt oder C₃₋₆Alkoxy auch cyclisch sowie ggf. ein-, zwei- oder dreifach mit unabhängig voneinander ausgewählten Resten R^{§} substituiert,
   - ein monocyclischer gesättigter oder ein- oder mehrfach ungesättigter Heterocyclus mit 4 - 8 Ringatomen darunter 1-3 Heteroatomen, vorzugsweise N, O und S
R₄
   - NR₇R₈, worin dieser Substituent insgesamt bedeutet:
      - Morpholino, Thiomorpholino, Thiomorpholino-S,S,-dioxid, Pyrrolidino, Piperidino, Piperazin-1-yl, 1-Homopiperazinyl, 4-C₁₋₆-Alkyl-Piperazin-1-yl, 4-Aryl-1-Piperazin-1-yl, 4-Bn-Piperazin-1-yl (ggf. mit R^{§} am heterocycloaliphatischen Ring substituiert),
      - weitere Amino-Reste sekundärer, mono- oder polycyclischer Amine mit insgesamt 4-14 Ringatomen, einschließlich der am C-Skelett mit R^{§} substituierten Vertreter
R₅
   - mono-, bi- oder tricyclischer gesättigter oder ein- oder mehrfach ungesättigter heterocyclischer Rest mit insgesamt 4-14 Ring-Atomen, davon 1-5 Heteroatomen (vorzugsweise N, O und S), ggf. mit R^{§} substituiert,
   - C₁₋₆ Alkyl, C₁₋₆ Alkoxy, C₁₋₆ Alkylthio, jeweils geradkettig, verzweigt oder cyclisch ggf. mit R^{§} substituiert
   - ein über ein exocyclisches Atom aus der Gruppe O, N, S gebundener gesättigter oder ein- oder mehrfach ungesättigter heterocyclischer Rest mit insgesamt 4-10 Ring-Atomen, davon 1-5 Heteroatomen (vorzugsweise N, O und S), ggf. mit R^{§} substituiert,
   - ein über ein am Tricyclus gebundenes Atom aus der Gruppe O, N oder S und eine nachfolgend angeordnete C₁₋₆ Alkylen-Gruppe gebundener gesättigter oder ein- oder mehrfach ungesättigter heterocyclischer Rest mit insgesamt 4-10 Ring-Atomen, davon 1-5 Heteroatomen (vorzugsweise N, O und S), ggf. mit R^{§} substituiert,
   - Hydroxy, Halogen (Cl, Br, I)
R₆
   - Wasserstoff,
   - C₁₋₆Alkyl, geradkettig, verzweigt oder C₃₋₆Alkyl auch cyclisch sowie ggf. ein-, zwei- oder dreifach mit unabhängig voneinander ausgewählten Resten R^{§} substituiert
   - C₂₋₆Alkenyl (ggf. mit R^{§} substituiert)
   - Alkyl, einfach oder mehrfach, mit mono- oder bicyclischen gesättigten oder ein- oder mehrfach ungesättigten Heterocyclen mit 4 - 14 Ringatomen darunter 1 - 5 Heteroatome, die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sein können, substituiert sowie ggf. mit R^{§} substituiert
   - Carbonyl oder Sulfonyl, jeweils substituiert mit
      - Wasserstoff, C₁₋₆Alkyl (ggf. mit R^{§} substituiert),
      - C₂₋₆Alkenyl (ggf. mit R^{§} substituiert),
      - C₂₋₆Alkinyl (ggf. mit R^{§} substituiert),
      - Aryl, insb. Phenyl (ggf. mit R^{§} substituiert),
      - einem mono- oder bicyclischen gesättigten oder ein- oder mehrfach ungesättigten Heterocyclus mit 4 - 14 Ringatomen darunter 1 - 5 Heteroatomen (vorzugsweise N, O und S) der ggf. ein-, zwei- oder dreifach mit unabhängig voneinander ausgewählten Resten R^{§} und/ oder einem oder mehreren Sauerstoffatomen substituiert sein kann,
R^{§}
   - OH, -SH, -O-C₁₋₈Alkyl, -O-C₆₋₁₄ Aryl, -S-C₁₋₄ Alkyl, -S-C₆₋₁₄Aryl,
   - SO-C₁₋₄Alkyl, -SO-C₆₋₁₄Aryl, -SO₂-C₁₋₄Alkyl, -SO₂-C₆₋₁₄Aryl, -SO₃H,
   - OSO₂C₁₋₈Alkyl, -OSO₂C₆₋₁₄Aryl, -COOH, -COOC₁₋₈Alkyl,
   - (CO)C₁₋₈Alkyl,
   - COOH, -CONH₂, -CONHC₁₋₆Alkyl, -CON(C₁₋₆Alkyl)₂,
   - NH₂, -NHC₁₋₆Alkyl, -N(C₁₋₆Alkyl)₂, -NHC₆₋₁₄Aryl, -NH-Hetaryl,
   - N(C₆₋₁₄Aryl)₂, -N(C₁₋₆Alkyl)(C₆₋₁₄Aryl),
   - C₁₋₆Alkyl, -C₂₋₁₂ Alkenyl, -C₂₋₁₂ Alkinyl, jeweils geradkettig, verzweigt oder cyclisch sowie ggf. ein-, zwei- oder dreifach unabhängig voneinander mit Halogen substituiert,
   - Halogen (-F, -Cl, -Br, -I),
   - CH₂CH₂OH, -CH₂CH₂SH, -CH₂CH₂SCH₃,
   - Sulfamoyl, Alkylsulfamoyl, Dialkylsulfamoyl mit Alkyl C₁₋₅ ggf. substituiert mit Methoxy,
   - Amidino, Hydroxyamidino
   - Sulfo, Phosphono,
   - -CN, -NO₂, und -SCN
sowie pharmazeutisch verträgliche Salze, Solvate, aktive Metabolite, Tautomere und Prodrugs dieser Verbindungen,

Ausgenommen sind Verbindungen mit:

- R₀=R₁=R₂=R₃=H

Die Begriffe "Alkyl, Alkenyl, Alkinyl, Alkoxy, usw.", auch in Wortzusammensetzungen wie Alkylsulfonyl, Alkylamino oder Alkoxycarbonyl usw. bedeuten sowohl die unverzweigten wie auch die verzweigten möglichen Verbindungen. Ebenso bedeuten "Alkenyl und Alkinyl" die entsprechend möglichen einfach oder mehrfach ungesättigten Verbindungen. Das gleiche gilt auch für die entsprechenden cyclischen Verbindungen.

"Aryl" bedeutet ein aromatisches monocyclisches oder mehrcyclisches Ringsystem mit 6 bis 14 Kohlenstoffatomen, vorzugsweise 6 bis 10 Kohlenstoffatomen. Die Arylgruppe kann ggf. mit einem oder mehreren Ringsubstituenten subtituiert sein. Bevorzugte Arylgruppen sind Phenyl oder Naphthyl.

"Halogen" bedeutet Fluor, Chlor, Brom oder Jod.

Unter "mono- oder bicyclischen gesättigten oder ein- oder mehrfach ungesättigte Heterocyclen mit 5-14 Ringatomen darunter 1 - 5 Heteroatome, die vorzugsweise N, O und S sind, ggf. ein-, zwei- oder dreifach mit unabhängig voneinander ausgewählten Resten R^{§} substituiert", sind vorzugsweise die folgenden Gruppen zu verstehen:
Thienyl, Pyridinyl, Pyrimidinyl, Piperazinyl, Pyridyl, Isoxazolyl, Piperidinyl, Pyrazinyl, Morpholino, Pyrrolyl, Triazinyl, Tetrazolyl, Oxazolyl, Benzo[d][1,3]dioxolyl, Indolyl, Imidazolyl, Pyrazolyl, Furanyl.

"Heteroaryl" (manchmal "Hetaryl" genannt) bedeutet ein aromatisches moncyclisches pder mehrcyclisches Ringsystem mit 5 bis 14 Ringatomen, vorzugsweise 5 bis 10 Ringatomen, bei dem 1 oder mehrere Ringatome ein anderes Element als Kohlenstoff ist, z.B. N, O, oder S, allein oder in Kombination. Bevorzugte Hetaryle enthalten 5 oder 6 Ringatome. Die Hetaryle können ggf. an einem oder mehreren Ringsystem substituiert sein. Beispiele geeigneter Hetaryle sind: Pyridyl, Pyrazinyl, Pyridinyl, Furanyl, Thienyl, Pyrimidinyl, Isoxazolyl, Isothiazolyl, Oxazolyl, Thiazolyl, Pyrazolyl, Furazanyl, Pyrrolyl, Triazolyl, 1,2,4-Thiadiazolyl, Pyrazinyl, Pyridazinyl, Benzofurazanyl, Indolyl, Azaindolyl, Benzoimidazolyl, Benzothienyl, Quinolinyl, Imidazolyl, Thienopyridinyl, 1,2,4-Triazinyl, Benzothiazolyl oder Benzoazaindolyl .

Im Sinne der Erfindung gelten alle Reste als miteinander kombinierbar, soweit bei der Definition der Reste nichts anderes angegeben ist. Es sollen alle denkbaren Untergruppierungen daraus als offenbart gelten.

Die Erfindung betrifft auch physiologisch verträgliche Salze der Verbindungen der allgemeinen Formel (1). Die physiologisch verträglichen Salze werden auf übliche Weise durch Umsetzung basischer Verbindungen der allgemeinen Formel (1) mit anorganischen oder organischen Säuren, ggf. auch bei Vorliegen von Verbindungen mit aciden Eigenschaften, wenn z.B. einer der Substituenten R¹, R², R³ oder R⁴ in diesen Verbindungen -COOH bzw. -SO₃H bedeutet, durch Neutralisation mit anorganischen oder organischen Basen, erhalten.

Als anorganische Säuren kommen vorzugsweise Salzsäure, Schwefelsäure, Salpetersäure oder Bromwasserstoffsäure, als organische Säuren zum Beispiel Ameisensäure, Essigsäure, Propionsäure, Glykolsäure, Milchsäure, Mandelsäure, Weinsäure, Äpfelsäure, Zitronensäure, Malonsäure, Maleinsäure, Fumarsäure, Succinsäure, Alginsäure, Benzoesäure, 2-, 3- und 4-Alkyloxy- und Acyloxy-benzoesäuren, Ascorbinsäure, C₁-C₃,Alkylsulfonsäuren, Benzolsulfonsäure, Nicotinsäure, Isonicotinsäure und Aminosäuren zur Anwendung.

Als anorganische Basen kommen zum Beispiel Ammoniak, Natron- und Kalilauge sowie als organische Basen Alkylamine, C₁-C₃, Pyridin, Chinolin, Isochinolin, Piperazin und -Derivate, Picoline, Chinaldin oder Pyrimidin zur Anwendung.

Weiterhin können physiologisch verträgliche Salze der Verbindungen gemäß der allgemeinen Formel (1) dadurch gewonnen werden, dass jene Substanzen, die als Substituenten eine tertiäre Amino-Gruppe besitzen, in prinzipiell bekannter Weise mit alkylierenden Agentien - wie zum Beispiel Alkyl- oder Aralkylhalogeniden - in die entsprechenden quaternären Ammoniumsalze übergeführt werden können.

Die Erfindung betrifft auch Solvate der Verbindungen, einschließlich der pharmazeutisch akzeptablen Salze, Säuren, Basen und Ester sowie deren aktive Metabolite und gegebenenfalls deren Tautomere gemäß der allgemeinen Formel (1) einschließlich Prodrug-Formulierungen. Prodrug-Formulierungen umfassen hierbei alle jene Substanzen, die durch einfache Transformation einschließlich Hydrolyse, Oxidation, oder Reduktion entweder enzymatisch, metabolisch oder auf andere Art und Weise entstehen. Ein geeignetes Prodrug enthält beispielsweise eine Substanz der allgemeinen Formel (1), die über einen enzymatisch spaltbaren Linker (z.B. Carbamat, Phosphat, N-Glycosid oder eine Disulfidgruppe) an eine lösungsverbessemde Substanz (z.B. Tetraethylenglykol, Saccharide, Aminosäuren oder Glucuronsäure, etc.) gebunden ist. Ein solches Prodrug einer erfindungsgemäßen Verbindung kann einem Patienten appliziert werden, und dieses Prodrug kann in eine Substanz der allgemeinen Formel (1) transformiert werden, wodurch der gewünschte pharmakologische Effekt erzielt wird.

Die erfindungsgemäßen Verbindungen können auf verschiedenen Wegen verabreicht werden, z.B. oral, parenteral, kutan, subkutan, intravenös, intramuskulär, rektal oder inhalativ. Bevorzugt ist die intravenöse Verabreichung. Die Verbindung wird einem Patienten, der eine Therapie einer unter das Indikationsspektrum der erfindungsgemäßen Verbindungen fallenden Krankheit bedarf, über einen vom Arzt zu bestimmenden Zeitraum verabreicht. Die Verbindung kann sowohl Menschen als auch anderen Säugern verabreicht werden.

Die Dosierung der erfindungsgemäßen Verbindungen wird vom Arzt anhand der patientenspezifischen Parameter wie z.B. Alter, Gewicht, Geschlecht, Schwere der Erkrankung, etc. bestimmt. Bevorzugt beträgt die Dosierung zwischen 0,001 mg/kg bis 1000 mg/kg Körpergewicht, bevorzugt 0,01 bis 500 mg/kg Körpergewicht und ganz bevorzugt 0,1 bis 100 mg/kg Körpergewicht.

Entsprechend der Art der Verabreichung wird das Medikament in geeigneter Weise formuliert, z.B. in Form von Lösungen bzw. Suspensionen, einfachen oder dragierten Tabletten, Hart- oder Weichgelatinekapseln, Suppositorien, Ovula, Injektionspräparaten, die nach üblichen galenischen Verfahren hergestellt werden.

Die erfindungsgemäßen Verbindungen können gegebenenfalls zusammen mit weiteren Wirkstoffen und mit in pharmazeutischen Zusammensetzungen üblichen Exzipientien formuliert werden, z.B. je nach herzustellendem Präparat Talk, Gummi arabicum, Lactose, Stärke, Magnesiumstearat, Kakaobutter, wäßrige und nichtwäßrige Träger, Fettkörper mit tierischem oder pflanzlichem Ursprung, Paraffinderivate, Glykole (insbesondere Polytethylenglykol), verschiedene Weichmacher, Dispergiermittel oder Emulgatoren, pharmazeutisch verträgliche Gase (z.B. Luft, Sauerstoff, Kohlendioxid usw.), Konservierungsstoffe.

Zur Herstellung flüssiger Präparate können Additive wie Natriumchloridlösung, Ethanol, Sorbit, Glycerin, Olivenöl, Mandelöl, Propylenglycol oder Ethylenglycol verwendet werden.

Bei der Verwendung von Infusions- oder Injektionslösungen sind diese sind bevorzugt wäßrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Geringe Mengen an Antibiotika können auch zugesetzt werden, um die Beibehaltung der Sterilität zu gewährleisten.

Die Erfindung betrifft auch pharmazeutische Zubereitungen, die eine therapeutisch wirksame Menge des aktiven Inhaltsstoffs (erfindungsgemäße Verbindung der Formel (1)) zusammen mit organischen oder anorganischen festen oder flüssigen pharmazeutisch verträglichen Trägem, die für die beabsichtigte Verabreichung geeignet sind, und die mit den aktiven Inhaltsstoffen nicht nachteilig wechselwirken, enthalten.

Bevorzugte erfindungsgemäße Substanzen sind:
7-Brom-4-ethoxy-9-fluor-2-(piperazin-1-yl)-5*H*-pyrimido[5,4-b]indol
7-Brom-4-ethoxy-2-(piperazin-1-yl)-5*H*-pyrimido[5,4-b]indol-9-ol
4-Ethoxy-2-(piperazin-1-yl)-7-(pyridin-4-yl)-5*H*-pyrimido[5,4-b]indol-9-ol
4-Ethoxy-7-(3,4-dimethoxyphenyl)-2-(piperazin-1-yl)-5*H*-pyrimido[5,4-b]indol-9-ol
7-(3,4-Dimethoxyphenyl)-2-(piperazin-1-yl)-5*H*-pyrimido[5,4-b]indol-4,9-diol
4-Ethoxy-8-(3,4,5-trimethoxyphenyl)-2-(piperazin-1-yl)-5*H*-pyrimido[5,4-b]indol
1-(7-(3,4-Dimethoxyphenyl)-2-(piperazin-1-yl)-5*H*-pyrimido[5,4-b]indol-4-yl)piperidin-4-ol
7-(3,4-Dimethoxyphenyl)-2-(piperazin-1-yl)-5*H*-pyrimido[5,4-b]indol-4-ol
4-((Pyridin-2-yl)methoxy))-7-(3,4-dimethoxyphenyl)-2-(piperazin-1-yl)-5*H-*pyrimido[5,4-b]indol
4-((Pyridin-4-yl)methoxy))-7-(3,4-dimethoxyphenyl)-2-(piperazin-1-yl)-5*H-*pyrimido[5,4-b]indol
4-((Pyridin-3-yl)methoxy))-7-(3,4-dimethoxyphenyl)-2-(piperazin-1-yl)-5*H-*pyrimido[5,4-b]indol
4-Methylthio-2-(piperazin-1-yl)-7-(pyridin-4-yl)-5*H*-pyrimido[5,4-b]indol
2-(7-(3,4-Dimethoxyphenyl)-2-(piperazin-1-yl)-5*H*-pyrimido[5,4-b]indol-4-ylamino)ethanol
7-Brom-2-(piperazin-1-yl)-5*H*-pyrimido[5,4-b]indol-4-ol
4-(2-Morpholinoethoxy))-7-(3,4-dimethoxyphenyl)-2-(piperazin-1-yl)-5*H*-pyrimido[5,4-b]indol
7-(3,4-Dimethoxyphenyl)-4-morpholino-2-(piperazin-1-yl)-5*H*-pyrimido[5,4-b]indol
7-(3,4-Dimethoxyphenyl)-2-(piperazin-1-yl)-4-thiomorpholino-5*H*-pyrimido[5,4-b]indol
4-Morpholino-2-(piperazin-1-yl)-7-(pyrindin-4-yl)-5*H*-pyrimido[5,4-b]indol
7-(3,4-Dimethoxyphenyl)-*N*-(2-morpholinoethyl)-2-(piperazin-1-yl)-5*H*-pyrimido[5,4-b]indol-4-amin
7-(3,4-Dimethoxyphenyl)-2-(piperazin-1-yl)-4-piperidino-5*H*-pyrimido[5,4-b]indol
4-Cyclopropylmethoxy-7-(3,4-dimethoxyphenyl)-2-(piperazin-1-yl)-5*H*-pyrimido[5,4-b]indol
4-(1*H*-Imidazol-1-yl)-7-(3,4-dimethoxyphenyl)-2-(piperazin-1-yl)-5*H*-pyrimido[5,4-b]indol
7-(3,4-Dimethoxyphenyl)-2-(piperazin-1-yl)-4-(piperidin-4-yloxy)-5*H*-pyrimido[5,4-b]indol
4-Cyclopropylmethoxy-2-(piperazin-1-yl)-7-(pyridine-4-yl)-5*H*-pyrimido[5,4-b]indol
4-Ethoxy-2-morpholino-7-(pyridine-4-yl)-5*H*-pyrimido[5,4-b]indol
4-(4-Ethoxy-2-(piperazin-1-yl)-5*H*-pyrimido[5,4-b]indol-7-yl)benzoesäureethylester
4-(4-Ethoxy-2-(piperazin-1-yl)-5*H*-pyrimido[5,4-b]indol-7-yl)benzoesäure-hydrochlorid
4-(4-Ethoxy-2-(piperazin-1-yl)-5*H*-pyrimido[5,4-b]indol-7-yl)phenol-hydrochlorid
3-(4-Ethoxy-2-(piperazin-1-yl)-5*H*-pyrimido[5,4-b]indol-7-yl)benzoesäuremethylester
3-(4-Ethoxy-2-(piperazin-1-yl)-5*H*-pyrimido[5,4-b]indol-7-yl)benzoesäure-hydrochlorid
4-Ethoxy-7-(furan-2-yl)-2-(piperazin-1-yl)-5*H*-pyrimido[5,4-b]indol
*tert*-Butyl-2-(4-ethoxy-2-(piperazin-1-yl)-5*H*-pyrimido[5,4-b]indol-7-yl)-1*H*-pyrrol-1-carboxylat
7-(Benzo[*d*][1,3]dioxol-5-yl)-4-ethoxy-2-(piperazin-1-yl)-5*H*-pyrimido[5,4-b]indol
7-(3,4-Dimethoxyphenyl)-2-(piperazin-1-yl)-*N*-(thiazol-2-yl)-5*H*-pyrimido[5,4-b]indol-4-amin
7-(3,4-Dimethoxyphenyl)-2-(piperazin-1-yl)-4-(1*H*-pyrrol-1-yl)-5*H*-pyrimido[5,4-b]indol
7-(3,4-Dimethoxyphenyl)-2-(piperazin-1-yl)-4-(1*H*-pyrazol-1-yl)-5*H*-pyrimido[5,4-b]indol
7-(3,4-Dimethoxyphenyl)-2-(piperazin-1-yl)-4-(1*H*-1,2,3-triazol-1-yl)-5*H*-pyrimido[5,4-b]indol
7-(3,4-Dimethoxyphenyl)-2-(piperazin-1-yl)-4-(4*H*-1,2,4-triazol-4-yl)-5*H*-pyrimido[5,4-b]indol
7-(3,4-Dimethoxyphenyl)-2-(piperazin-1-yl)-4-(pyrrolidin-1-yl)-5*H*-pyrimido[5,4-b]indol (4-(4-Ethoxy-2-(piperazin-1-yl)-5*H*-pyrimido[5,4-b]indol-7-yl)piperazin-1-yl)(phenyl)methanon
4-Ethoxy-2-(piperazin-1-yl)-7-(4-(pyrimidin-2-yl)piperazin-1-yl)-5*H*-pyrimido[5,4-b]indol
4-Ethoxy-2-(piperidin-4-yl)-7-(pyridin-4-yl)-5*H*-pyrimido[5,4-b]indol
4-Ethoxy-7-(3,4-dimethoxyphenyl)-2-(piperidin-4-yl)-5*H*-pyrimido[5,4-b]indol
4-Ethoxy-7-(3,4-dimethoxyphenyl)-2-(piperidin-3-yl)-5*H*-pyrimido[5,4-b]indol
2-(4-Ethoxy-9-methoxy-2-(piperazin-1-yl)-5*H*-pyrimido[5,4-b]indol-7-yl)benzenamin
2-{2-(Piperazin-1-yl)-7-(pyridin-4-yl)-5*H*-pyrimido[5,4-b]indol-4-yloxy}ethanol
4-Ethoxy-2-(4-methylpiperazin-1-yl)-7-(pyridin-4-yl)-5*H*-pyrimido[5,4-b]indol
4-Ethoxy-2-(piperazin-1-yl)-6-(pyridin-4-yl)-5*H*-pyrimido[5,4-b]indol
4-Ethoxy-2-(piperazin-1-yl)-7-(2-aminopyridin-5-yl)-5*H*-pyrimido[5,4-b]indol
4-Ethoxy-2-(piperazin-1-yl)-7-(3,4-dimethoxy-phenyl)-5*H*-pyrimido[5,4-b]indol
4-Ethoxy-2-(piperazin-1-yl)-7-(pyridin-3-yl)-5*H*-pyrimido[5,4-b]indol
4-Ethoxy-2-(piperazin-1-yl)-7-(pyridin-4-yl)-5*H*-pyrimido[5,4-b]indol
4-Ethoxy-2-(piperazin-1-yl)-8-(pyridin-4-yl)-5*H*-pyrimido[5,4-b]indol
4-Ethoxy-7-morpholino-2-(piperazin-1-yl)-5*H*-pyrimido[5,4-b]indol
4-Ethoxy-9-methoxy-2-(piperazin-1-yl)-7-(pyridin-4-yl)-5*H*-pyrimido[5,4-b]indol

Besonders bevorzugt sind die folgenden Verbindungen:
2-(4-Ethoxy-9-methoxy-2-(piperazin-1-yl)-5*H*-pyrimido[5,4-b]indol-7-yl)benzenamin
2-{2-(Piperazin-1-yl)-7-(pyridin-4-yl)-5*H*-pyrimido[5,4-b]indol-4-yloxy}ethanol
4-Ethoxy-2-(4-methylpiperazin-1-yl)-7-(pyridin-4-yl)-5*H*-pyrimido[5,4-b]indol
4-Ethoxy-2-(piperazin-1-yl)-6-(pyridin-4-yl)-5*H*-pyrimido[5,4-b]indol
4-Ethoxy-2-(piperazin-1-yl)-7-(2-aminopyridin-5-yl)-5*H*-pyrimido[5,4-b]indol
4-Ethoxy-2-(piperazin-1-yl)-7-(3,4-dimethoxy-phenyl)-5*H*-pyrimido[5,4-b]indol
4-Ethoxy-2-(piperazin-1-yl)-7-(pyridin-3-yl)-5*H*-pyrimido[5,4-b]indol
4-Ethoxy-2-(piperazin-1-yl)-7-(pyridin-4-yl)-5*H*-pyrimido[5,4-b]indol
4-Ethoxy-2-(piperazin-1-yl)-8-(pyridin-4-yl)-5*H*-pyrimido[5,4-b]indol
4-Ethoxy-7-morpholino-2-(piperazin-1-yl)-5*H*-pyrimido[5,4-b]indol
4-Ethoxy-9-methoxy-2-(piperazin-1-yl)-7-(pyridin-4-yl)-5*H*-pyrimido[5,4-b]indol
4-(4-Ethoxy-2-(piperazin-1-yl)-5*H* pyrimido[5,4-b]indol-7-yl)benzoesäureethylester
4-(4-Ethoxy-2-(piperazin-1-yl)-5*H*-pyrimido[5,4-b]indol-7-yl)benzoesäure-hydrochlorid
4-(4-Ethoxy-2-(piperazin-1-yl)-5*H*-pyrimido[5,4-b]indol-7-yl)phenol-hydrochlorid
3-(4-Ethoxy-2-(piperazin-1-yl)-5*H*-pyrimido[5,4-b]indol-7-yl)benzoesäuremethylester
3-(4-Ethoxy-2-(piperazin-1-yl)-5*H*-pyrimido[5,4-b]indol-7-yl)benzoesäure-hydrochlorid

Ganz besonders bevorzugt ist die Verbindung 4-Ethoxy-2-(piperazin-1-yl)-7-(pyridin-4-yl)-5*H-*pyrimido[5,4-b]indol.

Die Erfindung betrifft auch Verfahren zur Herstellung pharmazeutischer Zubereitungen, die dadurch gekennzeichnet sind, dass die erfindungsgemäße Verbindung mit einem pharmazeutisch verträglichen Träger vermischt wird.

Die erfindungsgemäßen Verbindungen eignen sich auch im Rahmen von Kombinationstherapien mit schon bekannten Wirkstoffen zur Behandlung der oben genannten Erkrankungen. Dabei sollen überraschende Synergieeffekte zur Steigerung der therapeutischen Wirksamkeit der erfindungsgemäßen Substanzen genutzt werden. Die Kombination kann zum einen darin bestehen, eine einzige pharmazeutische Zusammensetzung anzubieten, die mindestens eine der erfindungsgemäßen Verbindungen in Kombination mit einem oder mehrere der nachfolgend genannten Wirkstoffen enthält oder dem Patienten werden gleichzeitig oder zeitlich versetzt zur erfindungsgemäßen pharmazeutischen Zusammensetzung mehrere Mittel, die einen oder mehreren der nachfolgenden Wirkstoffe enthalten, verabreicht.

Es ist bevorzugt eine oder mehrere der erfindungsgemäßen Verbindungen mit einem oder mehreren der folgenden Wirkstoffe zu kombinieren:
- Nukleosidanaloga (z.B. Fludarabin, Cladribin)
- Alkylantien (z.B. Chlorambucil, Cyclophosphamid)
- ß₂-Adrenoceptor Agonisten (z.B. Terbutalin, Salbutanol, Salmetanol, Fenoterol, Formoterol)
- Korticosteroide
- Leukotrien-Antagonisten (entweder Enzyminhibitoren [wie 5-Lipoxygenaseinhibitoren oder Arachidonsäure-Enzyminhibitoren] oder Rezeptorantagonisten), z.B. Pramkulast, Montelukast, Zafirlukast, Zileuton
- Antihistaminika (bevorzugt solche mit Mastzellen-stabilisierenden Eigenschaften oder Leukotrien-antagonisierenden Aspekten, wie z.B. Loratadin, Astemizol, Mizolastin, Olopatadin
- Theophyllin
- Breitbandinhibitoren der Phosphodiesterasen
- Inhibitoren der Phosphodiesterasen 3, 4 und 7
- (monoklonale) Antikörper gegen TNF-alpha oder andere Wirkstoffe, die die Bildung bzw. Freisetzung von TNF-alpha oder die Aktivität von TNF-alpha hemmen (z.B. rekombinante lösliche Rezeptorkonstrukte)
- (monoklonale) Antikörper (z.B. Rituximab, TACI-Ig)

Die Kombination mit Nukleosidanaloga, Alkylantien, monoklonalen Antikörpern, Korticosteroiden, PDE-Inhibitoren, Leukotrien-Antagonisten, Antihistaminika, Theophylin, und/oder TNF-alpha-Hemmem dient besonders dazu, den akuten zu behandelnden Krankheitszustand zu bremsen, da die erfindungsgemäßen Verbindungen und die anderen Wirkstoffe komplementäre Aspekte der der Erkrankung zugrundeliegenden pathophysiologischen Mechanismen positiv beeinflussen. Erfindungsgemäß sollte insbesondere die Kombination der erfindungsgemäßen Verbindungen mit Nukleosidanaloga oder Alkylantien, PDE-Inhibitoren und Glucocortikoiden zu synergistischen Effekten in der Auslösung einer Apoptose der leukämischen B-Zellen führen. Eine solcher synergistischer Effekt konnte beispielsweise in einer Kombination mit Fludarabin beobachtet werden (s. Beispiele). In der Kombination mit Glucocorticoiden ergibt sich ein positiver Effekt daraus, dass weniger Glucocorticoide angewendet werden müssen und damit ein Spareffekt zu erreichen ist und die von Glucocorticoiden bekannten Nebenwirkungen vermindert werden bzw. ganz ausbleiben.

Abhängig von der Krankheitsausprägung und den zugrunde liegenden Symptomen können die erfindungsgemäßen Verbindungen zu den anderen Wirkstoffen in der Kombination im Verhältnis von 1:10.000 bis 10.000:1, bevorzugt 1:1000 bis 1000:1, ganz bevorzugt 1:10 bis 10:1, vorliegen.

Für die erfindungsgemäßen Substanzen wurden Dosis-Wirkungskurven mit dem Programm *Sigma Plot* erstellt und anhand dieser Verlaufskurven die EC₅₀/IC₅₀-Werte für jede Substanz berechnet. Die IC₅₀-Werte für die erfindungsgemäßen Substanzen liegen zwischen 0,1 - 5 µM.

Von der Anmelderin wurde herausgefunden, dass die Apoptose-induzierende Wirkung der erfindingsgemäßen Substanzen auf gereinigte B-Lymphozyten von Patienten mit Leukämie einer individuelle Schwankungsbreite unterlag. Es gab bei den Patienten Gruppen, die stark, mittel oder eher schwach auf die erfindungsgemäßen Wirkstoffe reagierten. Eine Zytotoxitzität auf leukämische Zellen war aber in jeden Fall festzustellen. Von der Anmelderin wurde daraufhin in Untersuchungen herausgefunden, dass die leukämischen B-Lymphozyten von B-CLL Patienten mit einer 11q Deletion besonders empfindlich auf die Apoptose-induzierende Wirkung der erfindungsgemäßen Substanzen reagierten. Diese stellen daher ein besonders bevorzugtes Patientenkollektiv dar und hier sind auch die besten Behandlungsergebnisse gegeben.

Von der Anmelderin wurde auch untersucht, ob die erfindungsgemäßen Substanzen in gesunden B-Lymphozyten Apoptose auslösen, was sich auf deren Nebenwirkungsprofil negativ auswirken würde. Es wurde gefunden, dass durch die erfindungsgemäßen Substanzen B-CLL Zellen mit einem EC₅₀ von 1,83 ± 0,95 µM ca. sechzehnfach stärker beeinflusst werden als gesunde PBMC Zellen mit einem EC₅₀ von 29,49 ± 13,4 µM. Hiermit konnte zweifelsfrei gezeigt werden, dass die erfindungsgemäßen Substanzen einen sehr guten therapeutischen Effekt auf Leukämiezellen zeigen, ohne die anderen gesunden Blutzellen anzugreifen. Auch der unmittelbare Wirkvergleich von Fludarabin als momentaner "golden standard" mit den erfindungsgemäßen Substanzen im Blut von B-CLL Patienten, zeigt einen EC₅₀ Wert von 2 µM bis 200 µM für Fludarabin (Literatur- und eigene Werte), während die erfindungsgemäßen Substanzen EC₅₀ Werte von 10 nM bis 5 µM aufwiesen.

Eine weitere vorteilhafte Eigenschaft der erfindungsgemäßen Substanzen ist, dass im Gegensatz zur Positivkontrolle Saponin humanen Erythrozyten nicht hämolysieren.

Um die spezifische Induktion des programmierten Zelltods (Apoptose) festzustellen wurde ein Nachweis desselben anhand der Caspase-3/7 Aktivität durchgeführt; die Caspase-3/7 Aktivität wird in der Literatur als ein sicherer Nachweis der Apoptose beschrieben. Es wurde gefunden, dass mit den erfindungsgemäßen Substanzen in einer Konzentrationsabhängigkeit die für die Apoptose entscheidenden Triggerenzyme, die Caspasen 3, 7 und 9 aktiviert werden. Diese erhobenen Daten belegen eindeutig, dass die durch die erfindungsgemäßen Substanzen verursachte Zellzytotoxizität kein toxischer Effekt ist, der zur Nekrose der Zellen führt, sondern dass es sich hierbei um eine Induktion der Apoptose handelt.

Die Erfindung betrifft weiterhin Verfahren zur Herstellung der erfindungsgemäßen Verbindungen.

Die erfindungsgemäßen Verfahren zur Herstellung der Verbindungen der allgemeinen Formel 1 mit den zuvor aufgeführten Bedeutungen von R₀, R₁, R₂, R₃, R₄, R₅ und R₆ sind gekennzeichnet durch folgende Verfahrensweisen:
Allgemeine Darstellung nach Schema 1: 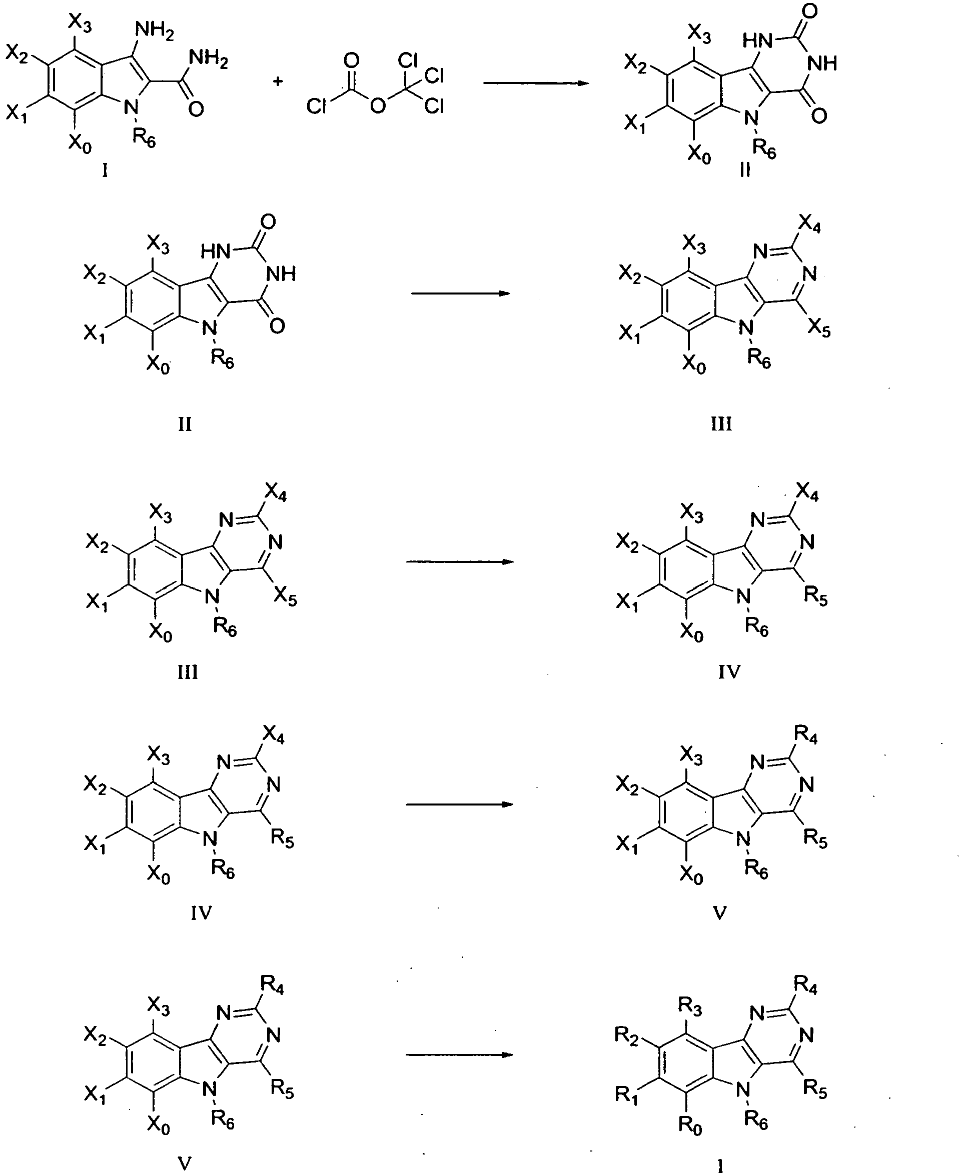 X₀-X₅ = unabhängig gleich oder ungleich F, Cl, Br, I, H oder R wobei X₄, X₅ ungleich H
   - Umsetzung der Verbindungen der allgemeinen Formel I mit Phosgenderivaten, vorzugsweise Diphosgen, in einem geeignetem Lösungsmittel, vorzugsweise Dioxan oder Toluol, zu den Verbindungen der allgemeinen Formel II.
   - Umsetzung der Verbindungen der allgemeinen Formel II mit einem Halogenierungsmittel, vorzugsweise Dichlorphenylphosphinoxid, Phosphor-trichlorid, Phosphorpentachlorid, Phosphoroxychlorid bzw. deren Mischungen, jeweils in der Hitze, zu den Verbindungen der allgemeinen Formel III.
   - Umsetzung der Verbindungen der allgemeinen Formel III mit O-, N-, S- oder C-Nucleophilen, vorzugsweise Alkoholate, Amine und Thiolate, in Alkanolen oder gegebenenfalls aprotischen, dipolaren Lösungsmitteln unter Erwärmen, in Ausnahmefällen auch bei Raumtemperatur, zu den tricyclischen Verbindungen der allgemeinen Formel IV.
   - Umsetzung der Verbindungen der allgemeinen Formel IV mit O-, N-, S- oder C-Nucleophilen, vorzugsweise Alkoholate, Amine und Thiolate, in einem geeigneten Lösungsmittel vorzugsweise Toluol, Mesitylen oder Dioxan unter Erwärmen, in Ausnahmefällen auch bei Raumtemperatur, zu den Verbindungen der allgemeinen Formel V.
   - Umsetzung der Verbindungen der allgemeinen Formel V mit O-, N-, S- oder C-Nucleophilen in einem geeigneten Lösungsmittel, vozugsweise Toluol, Dioxan oder THF, unter Erwärmen und unter Verwendung geeigneter Katalysatoren, in Ausnahmefällen unter Verwendung einer Mikrowelle, zu den Verbindungen der allgemeinen Formel 1.
   - Die Einführung der Reste R₀, R₁. R_{2,} R₄, R₅ und R₆ kann jeweils auf verschiedenen Synthesestufen, mittels geeigneter Reaktionen, insbesondere metallkatalysierter C-C-Kreuzkupplungsreaktionen, und gegebenenfalls unter Verwendung geeigneter Schutzgruppen, erfolgen oder sind bereits in den entsprechenden Edukten enthalten.

Die Erfindung wird weiter anhand der nachfolgenden Beispiele beschrieben.

### Beispiel 1

### Die Synthese von 2-(4-Ethoxy-9-methoxy-2-(piperazin-1-yl)-5H-pyrimido[5,4-b]indol-7-yl)benzenamin

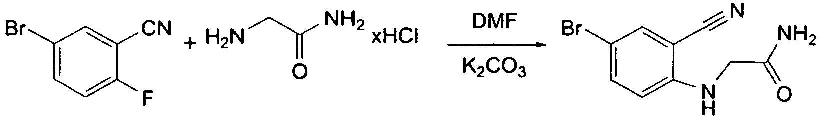

12.6 g (58.3 mmol) 4-Brom-2,6-difluorbenzonitril, 6.8 g (61.5 mmol) Glycinamidhyrdrochlorid und 16.1 g (116.5 mmol) K₂CO₃ wurden in 50 ml DMF suspendiert und 18 h bei 70 °C erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 13.3 g (84 %) 2-(5-Brom-2-cyano-3-fluorophenylamino)acetamid. ESI-MS [m/z]: 272, 274 [M+H]⁺

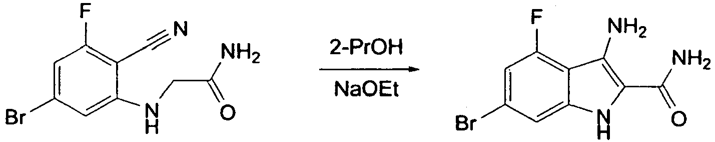

17.3 g (63.6 mmol) 2-(5-Brom-2-cyano-3-fluorophenylamino)acetamid wurden in 150 ml 2-Propanol suspendiert, nach Zugabe von 10 ml NaOEt-Lösung (21 % in EtOH) wurde 14 h unter Rückfluß erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 15.4 g (89 %) 3-Amino-6-brom-4-fluor-1*H*-indol-2-carbonsäureamid. ESI-MS [m/z]: 272, 274 [M+H]⁺

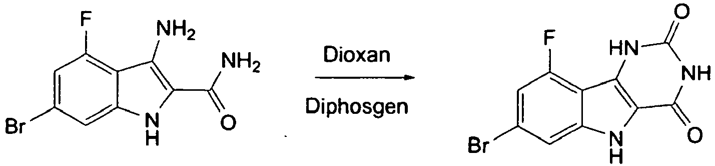

15.4 g (56.6 mmol) 3-Amino-6-brom-4-fluor-1H-indol-2-carbonsäureamid wurden in 300 ml Dioxan suspendiert, nach Zugabe von 7.5 ml (61.8 mmol) Diphosgen wurde 2 h unter Rückfluß erhitzt. Nach dem Abkühlen auf Raumtemperatur wurden vorsichtig 20 ml Wasser zugegeben und im Anschluss der Niederschlag abgesaugt. Man erhält 12.5 g (74 %) 7-Brom-9-fluor-1*H*-pyrimido[5,4-b]indol-2,4(3*H*,5*H*)-dion. ESI-MS [m/z]: 296, 298 [M-H]⁻

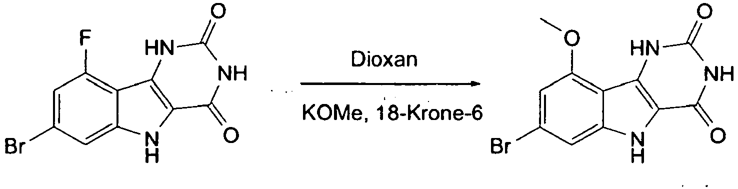

1.7 g (5.7 mmol) 7-Brom-9-fluor-1*H*-pyrimido[5,4-b]indol-2,4(3*H*,5*H*)-dion, 4.5 g (17.0 mmol) 18-Krone-6 und 12 ml (162.5 mmol) 30 % KOMe-Lösung (MeOH) wurden in 600 ml Dioxan 20 h unter Rückfluß erhitzt. Nach dem Abkühlen auf Raumtemperatur wurde das Lösungsmittel eingeengt, Wasser zugegeben und mit 1 N HCl angesäuert. Der entstandene Niederschlag wurde abgesaugt und mit Wasser gewaschen. Man erhält 650 mg (37 %) 7-Brom-9-methoxy-1*H*-pyrimido[5,4-b]indol-2,4(3*H*,5*H*)-dion. ESI-MS [m/z]: 308, 310 [M-H]⁻

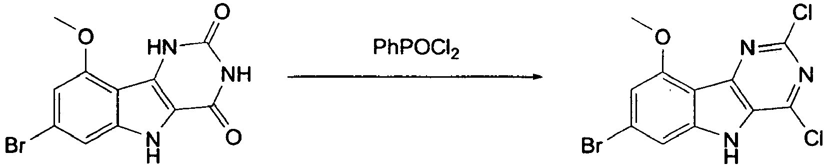

650 mg (2.1 mmol) 7-Brom-9-methoxy-1*H*-pyrimido[5,4-b]indol-2,4(3H,5H)-dion und 1.8 ml (12.6 mmol) Dichlorphenylphosphinoxid wurden 6 h auf 185 °C erhitzt. Nach Abkühlung auf RT wurde auf 40 g Eiswasser gegossen und mit gesättigter Natriumhydrogencarbonat-Lösung neutralisiert. Der entstandene Niederschlag wurde abgesaugt und mit Wasser gewaschen. Man erhält 600 mg (83 %) 7-Brom-2,4-dichlor-9-methoxy-5*H*-pyrimido[5,4-b]indol. ESI-MS [m/z]: 344, 346, 348 [M-H]⁻

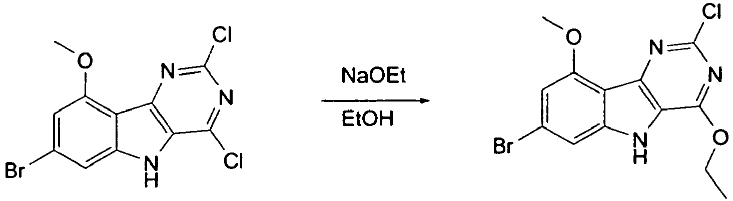

600 mg (1.7 mmol) 7-Brom-2,4-dichlor-9-methoxy-5*H*-pyrimido[5,4-b]indol wurden in 20 ml Ethanol suspendiert und mit 174 mg (2.6 mmol) Natriumethylat versetzt. Das Gemisch wurde 3 h unter Rückfluss erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 450 mg (73 %) 7-Brom-2-chlor-4-ethoxy-9-methoxy-5*H*-pyrimido[5,4-b]indol. ESI-MS [m/z]: 354, 356 [M-H]⁻

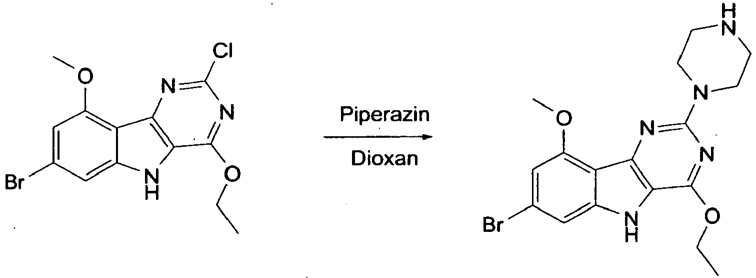

150 mg (0.42 mmol)7-Brom-2-chlor-4-ethoxy-9-methoxy-5*H*-pyrimido[5,4-b]indol und 181 mg (2.1 mmol) Piperazin wurden in 10 ml Dioxan 60 h unter Rückfluss erhitzt. Anschließend wird das Lösungsmittel entfernt und der Rückstand in Wasser suspendiert und abgesaugt. Man erhält 130 mg (76 %) 7-Brom-4-ethoxy-9-methoxy-2-(piperazin-1-yl)-5*H*-pyrimido[5,4-b]indol. ESI-MS [m/z]: 406, 408 [M+H]⁺

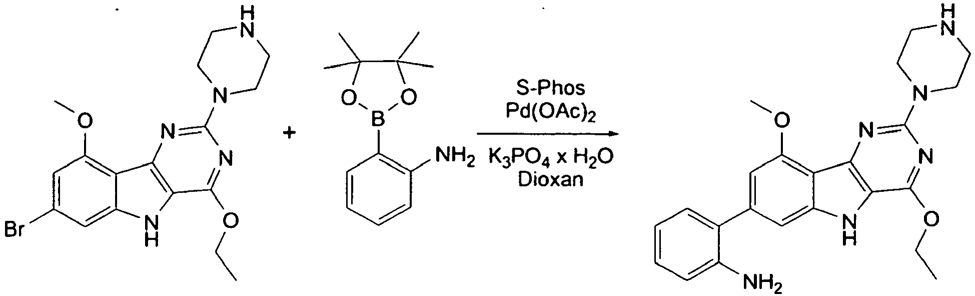

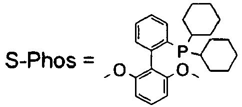

70 mg (0.17 mmol) 7-Brom-4-ethoxy-9-methoxy-2-(piperazin-1-yl)-5*H*-pyrimido[5,4-b]indol, 56 mg (0.26 mmol) 2-(4,4,5,5-Tetramethyl-1,3,2-dioxaborolan-2-yl)anilin, 119 mg (0.51 mmol) K₃PO₄ x H₂O, 2.8 mg (6,8 µmol) S-Phos und 0.8 mg (3.4 µmol) Pd(OAc)₂ in 6 ml Dioxan wurden in einer Mikrowelle 1 h auf 120 °C erhitzt. Anschließend wurde das Lösungsmittel entfernt und nach FC (CH₂Cl₂:MeOH, 0 - 20 %) erhielt man 20 mg (24 %) der Titelsubstanz. ESI-MS [m/z]: 419 [M+H]⁺

### Beispiel 2

### Die Synthese von 4-Ethoxy-9-methoxy-2-(piperazin-1-yl)-7-(pyridin-4-yl)-5H-pyrimido[5,4-b]indol

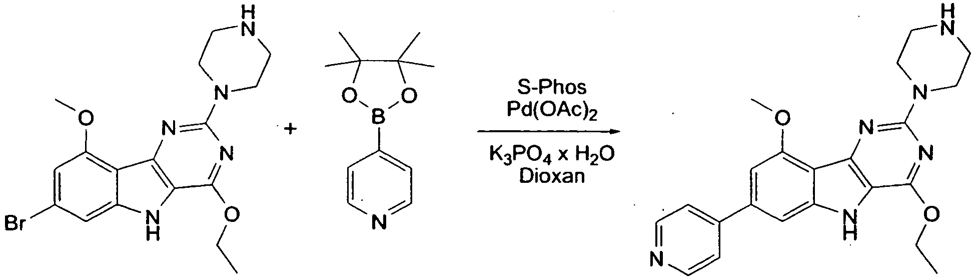

60 mg (0.15 mmol) 7-Brom-4-ethoxy-9-methoxy-2-(piperazin-1-yl)-5*H*-pyrimido[5,4-b]indol (Beispiel 1), 47 mg (0.23 mmol) 4-(4,4,5,5-Tetramethyl-1,3,2-dioxaborolan-2-yl)pyridin, 61 mg (0.45 mmol) K₂CO₃, 2.4 mg (6,0 µmol) S-Phos und 0.7 mg (3.0 µmol) Pd(OAc)₂ in 6 ml Dioxan wurden in einer Mikrowelle 1 h auf 120 °C erhitzt. Anschließend wurde das Lösungsmittel entfernt und nach FC (CH₂Cl₂:MeOH, 0 - 30 %) erhielt man 27 mg (45 %) der Titelsubstanz. ESI-MS [m/z]: 405 [M+H]⁺

Folgende Verbindungen wurden analog den Beispielen 1 und 2 erhalten (Tabelle 1)

**Tabelle 1**

| **Struktur** | **M [g/mol]** | **ESI MS [m/z]** |
|---|---|---|
| 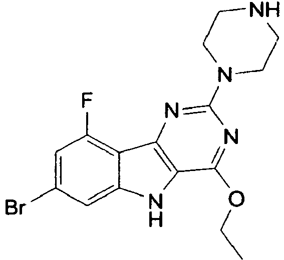 | 394.24 | 394,396 [M+H]⁺ |
| 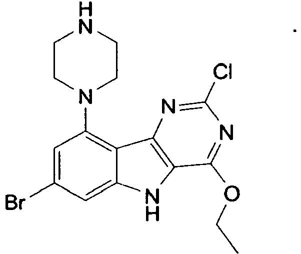 | 410.70 | 410,412 [M+H]⁺ |
| 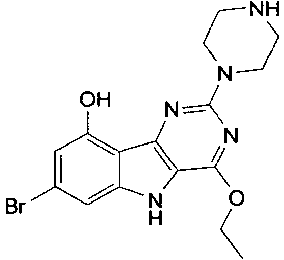 | 392.25 | 392,394 [M+H]⁺ |
| 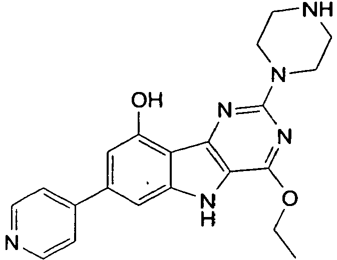 | 390.44 | 391 [M+H]⁺ |
| 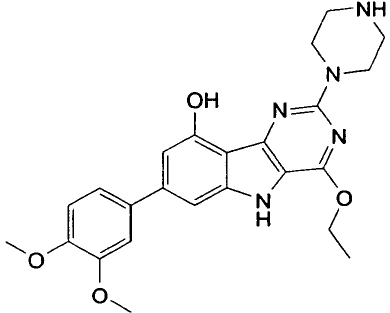 | 449.50 | 450 [M+H]⁺ |
| 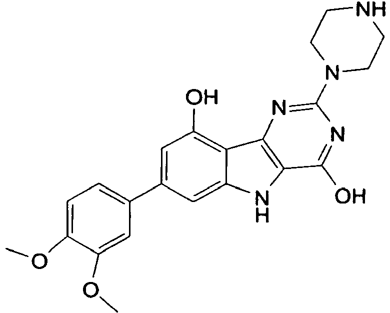 | 421.45 | 422 [M+H]⁺ |

### Beispiel 3

### Die Synthese von 4-Ethoxy-2-(piperazin-1-yl)-8-(pyridin-4-yl)-5H-pyrimido[5,4-b]indol

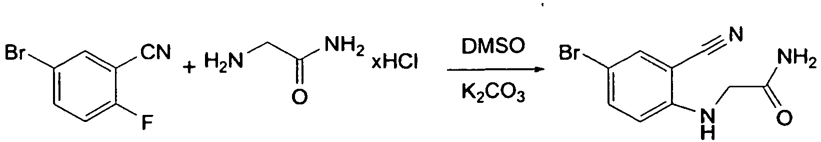

16.3 g (81.5 mmol) 5-Brom-2-fluorbenzonitril, 9.2 g (83.1 mmol) Glycinamidhyrdrochlorid und 23.6 g (170.8 mmol) K₂CO₃ wurden in 40 ml DMSO suspendiert und 3 h bei 100 °C erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 16.0 g (77 %) 2-(4-Brom-2-cyanophenylamino)acetamid. ESI-MS [m/z]: 254, 256 [M+H]⁺

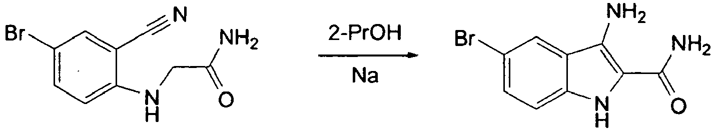

1.4 g (63.0 mmol) Natrium wurden in 200 ml 2-Propanol aufgelöst, anschließend wurden 16.0 g (63.0 mmol) 2-(4-Brom-2-cyano-phenylamino)acetamid zugegeben und die Lösung 14 h bei Raumtemperatur gerührt. Anschließend wurde das Lösungsmittel eingeengt und Wasser zugegeben. Der entstandene Niederschlag wurde abgesaugt und mit Wasser gewaschen. Man erhält 1.4 g (8 %) 3-Amino-5-brom-1*H-*indol-2-carbonsäureamid. ESI-MS [m/z]: 254, 256 [M+H]⁺

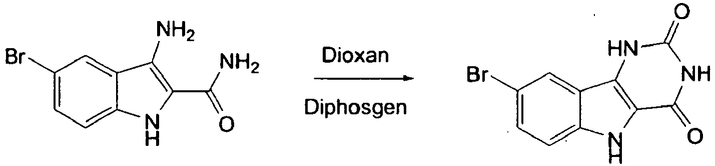

1.3 g (5.1 mmol) 3-Amino-5-brom-1*H*-indol-2-carbonsäureamid wurden in 50 ml Dioxan suspendiert, nach Zugabe von 670 µl (5.5 mmol) Diphosgen wurde 2 h unter Rückfluß erhitzt. Nach dem Abkühlen auf Raumtemperatur wurden vorsichtig 2 ml Wasser zugegeben und im Anschluss der Niederschlag abgesaugt. Man erhält 1.4 g (94 %) 8-Brom-1H-pyrimido[5,4-b]indol-2,4(3*H*,5*H*)-dion. ESI-MS [m/z]: 278, 280 [M-H]-

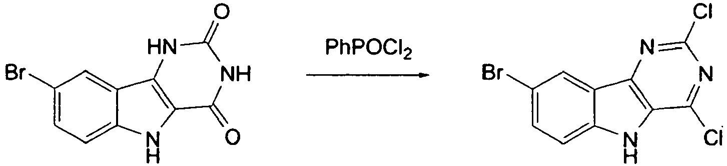

1.35 g (4.8 mmol) 8-Brom-1*H*-pyrimido[5,4-b]indol-2,4(3*H*,5*H*)-dion und 8 ml (57.4 mmol) Dichlorphenylphosphinoxid wurden 6 h auf 180 °C erhitzt. Nach Abkühlung auf RT wurde auf 40 g Eiswasser gegossen und mit gesättigter Natriumhydrogencarbonat-Lösung neutralisiert. Der entstandene Niederschlag wurde abgesaugt und mit Wasser gewaschen. Man erhält 930 mg (61 %) 8-Brom-2,4-dichlor-5*H-*pyrimido[5,4-b]indol. ESI-MS [m/z]: 314, 316, 318 [M-H]⁻

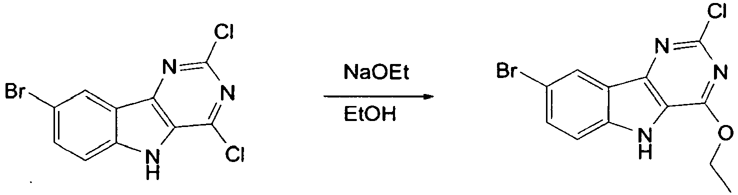

930 mg (2.9 mmol) 8-Brom-2,4-dichlor-5*H*-pyrimido[5,4-b]indol wurden in 20 ml Ethanol suspendiert und mit 592 mg (8.7 mmol) Natriumethylat versetzt. Das Gemisch wurde 8 h unter Rückfluss erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 920 mg (97 %) 8-Brom-2-chlor-4-ethoxy-5*H*-pyrimido[5,4-b]indol. ESI-MS [m/z]: 324, 326 [M-H]⁻

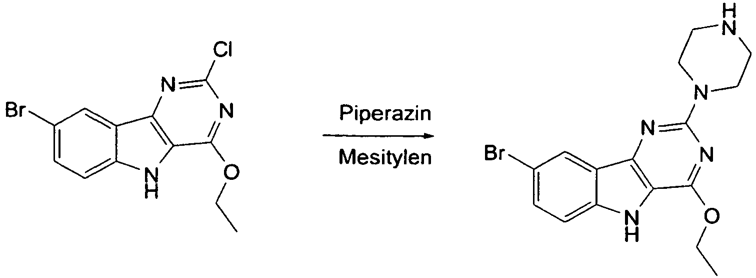

920 mg (2.9 mmol) 8-Brom-2-chlor-4-ethoxy-5*H*-pyrimido[5,4-b]indol und 813 mg (9.5 mmol) Piperazin wurden in 10 ml Mesitylen 18 h auf 150 °C erhitzt. Anschließend wird das Lösungsmittel entfernt und der Rückstand in Wasser suspendiert und abgesaugt. Man erhält 650 mg (60 %) 8-Brom-4-ethoxy-2-(piperazin-1-yl)-5*H-*pyrimido[5,4-b]indol. ESI-MS [m/z]: 376, 378 [M+H]⁺

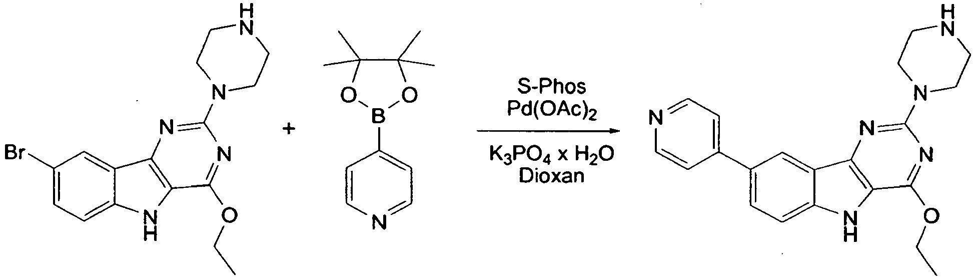

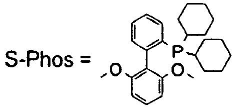

100 mg (0.27 mmol) 8-Brom-4-ethoxy-2-(piperazin-1-yl)-5*H*-pyrimido[5,4-b]indol, 83 mg (0.41 mmol) 4-(4,4,5,5-Tetramethyl-1,3,2-dioxaborolan-2-yl)pyridin, 344 mg (1.62 mmol) K₃PO₄ x H₂O, 2.8 mg (6,8 µmol) S-Phos und 0.8 mg (3.4 µmol) Pd(OAc)₂ in 6 ml THF wurden in einer Mikrowelle 2 h auf 120 °C erhitzt. Anschließend wurde das Lösungsmittel entfernt und nach Reinigung mittels präparativer HPLC (RP18) erhielt man 52 mg (32 %) der Titelsubstanz als 2-faches TFA-Salz. ESI-MS [m/z]: 375 [M+H]⁺

Folgende Verbindungen wurden analog Beispiel 3 erhalten (Tabelle 2)

**Tabelle 2**

| **Struktur** | **M [g/mol]** | **ESI MS [m/z]** |
|---|---|---|
| 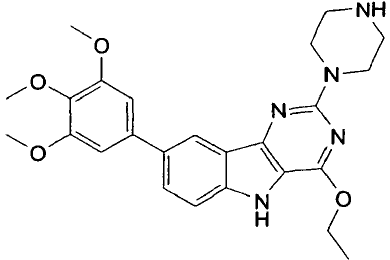 | 463.53 | 464 [M+H]⁺ |

### Beispiel 4

### Die Synthese von 7-Bromo-4-ethoxy-2-(piperazin-1-yl)-5H-pyrimido[5,4-b]indol

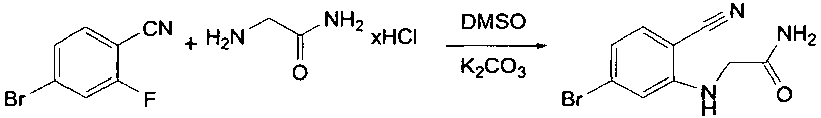

9.1 g (45.5 mmol) 4-Brom-2-fluorbenzonitril, 10.0 g (91.0 mmol) Glycinamidhyrdrochlorid und 15.7 g (114 mmol) K₂CO₃ wurden in 80 ml DMSO suspendiert und 5.5 h bei 120 °C erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 11.6 g (100 %) 2-(5-Brom-2-cyano-phenylamino)acetamid. ESI-MS [m/z]: 254, 256 [M+H]⁺

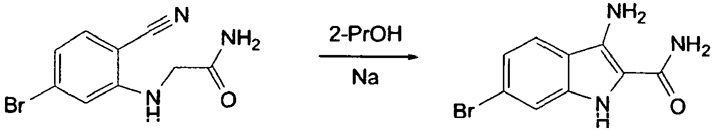

1.55 g (67.3 mmol) Natrium wurden in 200 ml 2-Propanol aufgelöst, anschließend wurden 14.3 g (56.1 mmol) 2-(5-Brom-2-cyano-phenylamino)acetamid zugegeben und die Lösung 30 min unter Rückfluss erhitzt. Anschließend wurde die Reaktionsmischung auf 0 °C abgekühlt und gesätt. NH₄Cl-Lösung zugegeben. Das Lösungsmittel wurde eingeengt, der entstandene Niederschlag abgesaugt und mit Wasser gewaschen. Man erhält 12.2 g (86 %) 3-Amino-6-brom-1*H*-indol-2-carbonsäureamid. ESI-MS [m/z]: 254, 256 [M+H]⁺

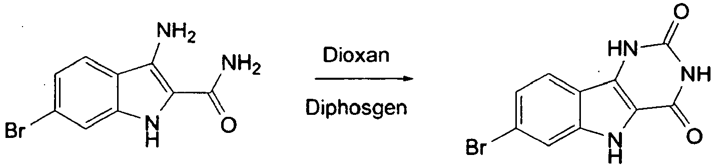

12.2 g (58 mmol) 3-Amino-6-brom-1*H*-indol-2-carbonsäureamid wurden in 50 ml Dioxan suspendiert, nach Zugabe von 11.7 ml (96 mmol) Diphosgen wurde 2 h unter Rückfluß erhitzt. Nach dem Abkühlen auf Raumtemperatur wurden vorsichtig 20 ml Wasser zugegeben und im Anschluss der Niederschlag abgesaugt. Man erhält 13.4 g (99 %) 7-Brom-1*H*-pyrimido[5,4-b]indol-2,4(3*H*,5*H*)-dion. ESI-MS [m/z]: 278, 280 [M-H]⁻

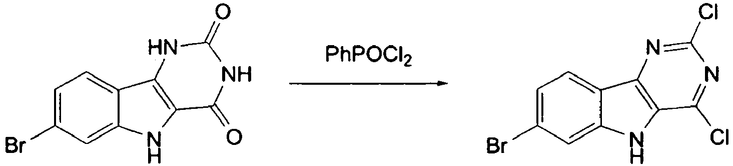

13.4 g (47 mmol) 7-Brom-1*H*-pyrimido[5,4-b]indol-2,4(3*H*,5*H*)-dion und 40 ml (287 mmol) Dichlorphenylphosphinoxid wurden 6 h auf 180 °C erhitzt. Nach Abkühlung auf RT wurde auf 40 g Eiswasser gegossen und mit gesättigter Natriumhydrogencarbonat-Lösung neutralisiert. Der entstandene Niederschlag wurde abgesaugt und mit Wasser gewaschen. Man erhält 11.6 g (71 %) 7-Brom-2,4-dichlor-5*H-*pyrimido[5,4-b]indol. ESI-MS [m/z]: 314, 316, 318 [M-H]⁻

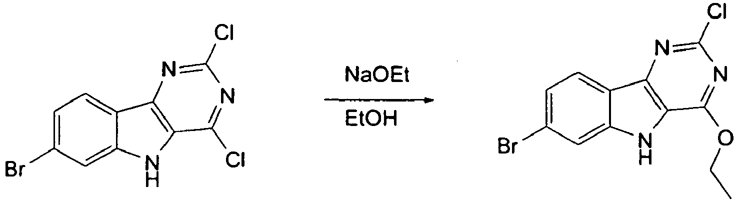

5 g (15.8 mmol) 7-Brom-2,4-dichlor-5*H*-pyrimido[5,4-b]indol wurden in 20 ml Ethanol suspendiert und mit 15.8 ml (31.5 mmol) 2M Natriumethylat-Lösung (EtOH) versetzt. Das Gemisch wurde 9.5 h unter Rückfluss erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 5.1 1 g (99 %) 7-Brom-2-chlor-4-ethoxy-5*H*-pyrimido[5,4-b]indol. ESI-MS [m/z]: 324, 326 [M-H]⁻

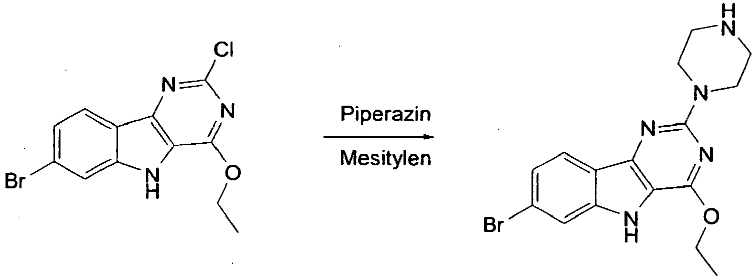

5.1 g (15.6 mmol) 7-Brom-2-chlor-4-ethoxy-5*H*-pyrimido[5,4-b]indol und 13.4 g (155.8 mmol) Piperazin wurden in 80 ml Mesitylen 7.5 h auf 150 °C erhitzt. Anschließend wird das Lösungsmittel entfernt und der Rückstand in Wasser suspendiert und abgesaugt. Man erhält 5.8 g (99 %) der Titelsubstanz. ESI-MS [m/z]: 376, 378 [M+H]⁺

### Beispiel 5

### Die Synthese von 7-Brom-2-ethoxy-4-(piperazin-1-yl)-5H-pyrimido[5,4-b]indol

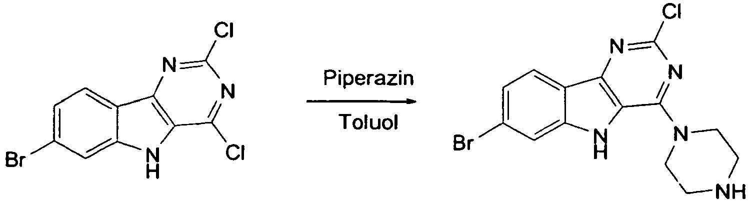

1.3 g (4.10 mmol) 7-Brom-2,4-dichlor-5*H*-pyrimido[5,4-b]indol (Beispiel 4) und 1.3 g (14.8 mmol) Piperazin wurden in 50 ml Toluol 3h bei 65°C erhitzt. Anschließend wurde das Lösungsmittel entfernt und der Rückstand in Wasser suspendiert und abgesaugt. Man erhält 1.3 g (87 %) 7-Brom-2-chlor-4-(piperazin-1-yl)-5*H*-pyrimido[5,4-b]indol. ESI-MS [m/z]: 366, 368 [M+H]⁺

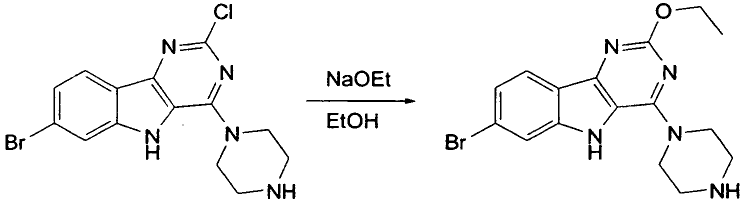

200 mg (0.55 mmol) 7-Brom-2-chlor-4-(piperazin-1-yl)-5*H*-pyrimido[5,4-b]indol und 1.64 ml (1.64 mmol) 1 M NaOEt-Lösung (EtOH) in 6 ml EtOH wurden in einer Mikrowelle 3 h auf 130 °C erhitzt. Anschließend wurde das Lösungsmittel entfernt und der Rückstand in Wasser suspendiert und abgesaugt. Man erhält 200 mg (97 %) der Titelsubstanz. ESI-MS [m/z]: 376, 378 [M+H]⁺

### Beispiel 6

### Die Synthese von 2-{7-bromo-2-(piperazin-1-yl)-5H-pyrimido[5,4-b]indol-4-yloxy}ethanol

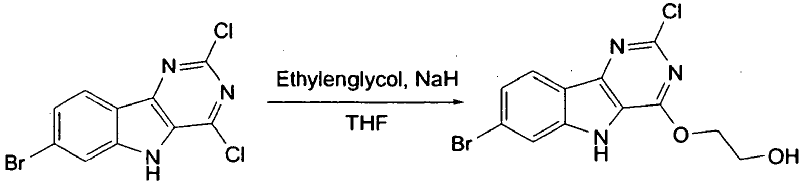

Zu 115 mg (4.8 mmol) NaH in 20 ml THF wurden 397 mg (6.4 mmol) Ethylenglycol gegeben und 30 min bei RT gerührt. Anschließend wurden 500 mg (1.6 mmol) 7-Brom-2,4-dichlor-5*H*-pyrimido[5,4-b]indol (Beispiel 4) hinzugegeben und 5h unter Rückfluss erhitzt. Anschließend wurde das Lösungsmittel entfernt und der Rückstand in Essigester aufgenommen und mit gesätt. NaCl-Lösung gewaschen, über Na₂SO₄ getrocknet und filtriert. Nach Entfernung des Lösungsmittels erhält man 450 mg (88 %) 2-(7-Brom-2-chlor-5*H*-pyrimido[5,4-b]indol-4-yloxy)ethanol. ESI-MS [m/z]: 342, 344 [M+H]⁺

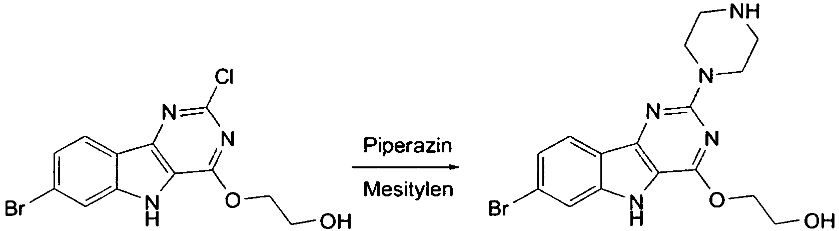

400 mg (1.17 mmol) 2-(7-Brom-2-chlor-5*H*-pyrimido[5,4-b]indol-4-yloxy)ethanol und 1.0 g (11.7 mmol) Piperazin in 50 ml Mesitylen wurden 5 h auf 150 °C erhitzt. Anschließend wurde das Lösungsmittel entfernt und der Rückstand in EtOH aufgenommen und filtriert. Nach Entfernung des Lösungsmittels erhält man 250 mg (55 %) der Titelsubstanz. ESI-MS [m/z]: 392, 394 [M+H]⁺

### Beispiel 7

### Die Synthese von 4-Ethoxy-2-(piperazin-1-yl)-7-(pyridin-4-yl)-5H-pyrimido[5,4-b]indol

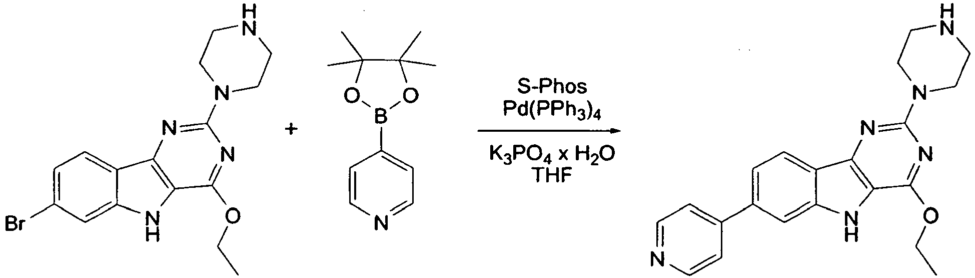

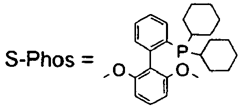

150 mg (0.4 mmol) 7-Brom-4-ethoxy-2-(piperazin-1-yl)-5*H*-pyrimido[5,4-b]indol (Beispiel 4), 123 mg (0.6 mmol) 4-(4,4,5,5-Tetramethyl-1,3,2-dioxaborolan-2-yl)pyridin, 276 mg (1.2 mmol) K₃PO₄ x H₂O, 6.6 mg (16 µmol) S-Phos und 9.2 mg (8 µmol) Pd(PPh₃)₄ in 6 ml THF wurden in einer Mikrowelle 2 h auf 120 °C erhitzt. Anschließend wurde das Lösungsmittel entfernt und nach FC(CH₂Cl₂/MeOH, 0-20 %) erhielt man 93 mg (62 %) der Titelsubstanz ESI-MS [m/z]: 375 [M+H]⁺

Folgende Verbindungen wurden analog den Beispielen 4-7 erhalten (Tabelle 3)

**Tabelle 3**

| **Struktur** | **M [g/mol]** | **ESI MS [m/z]** |
|---|---|---|
| 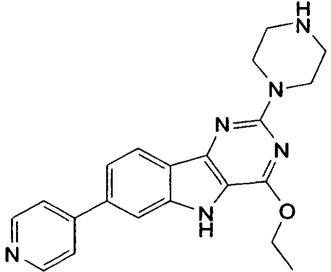 | 374.44 | 375 [M+H]⁺ |
| 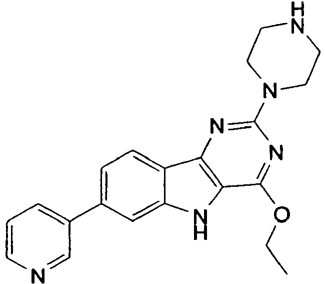 | 374.44 | 375 [M+H]⁺ |
| 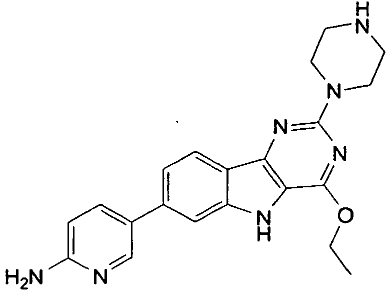 | 389.45 | 390 [M+H]⁺ |
| 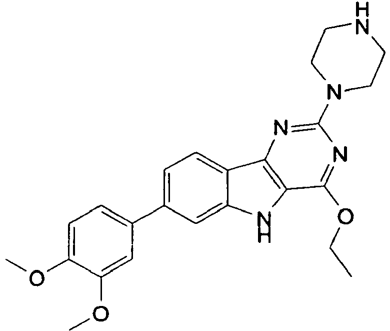 | 433.50 | 434 [M+H]⁺ |
| 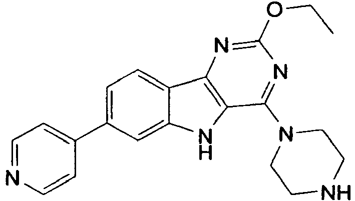 | 374.44 | 375 [M+H]⁺ |
| 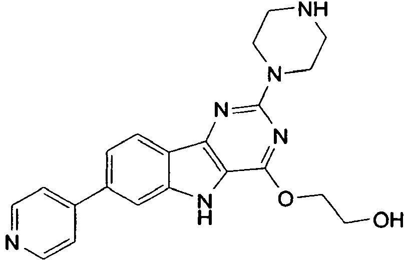 | 390.44 | 391 [M+H]⁺ |
| 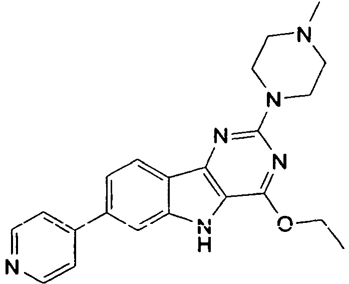 | 388.47 | 389 [M+H]⁺ |
| 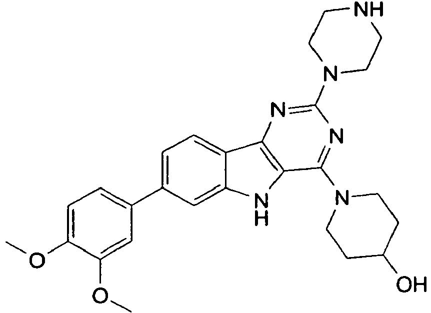 | 488.58 | 489 [M+H]⁺ |
| 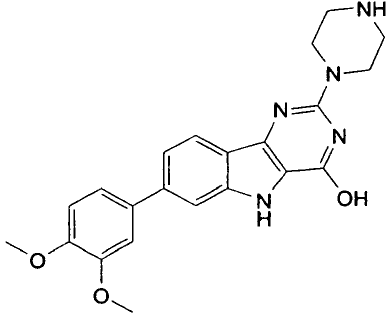 | 405.45 | 406 [M+H]⁺ |
| 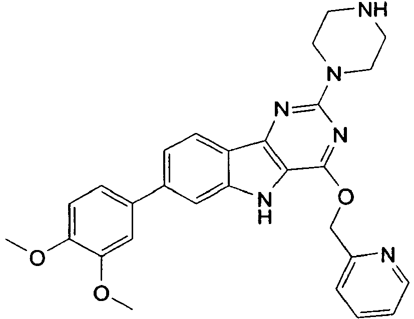 | 496.56 | 497 [M+H]⁺ |
| 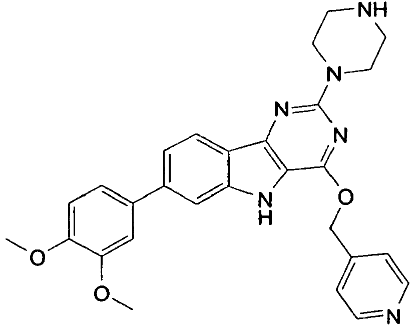 | 496.56 | 497 [M+H]⁺ |
| 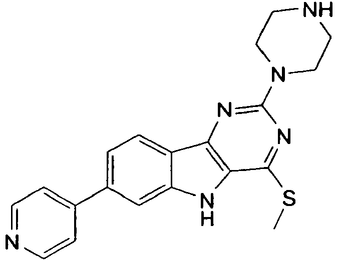 | 376.89 | 377 [M+H]⁺ |
| 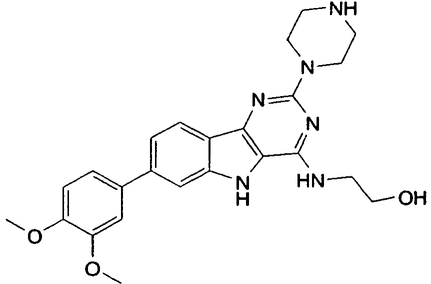 | 448.52 | 449 [M+H]⁺ |
| 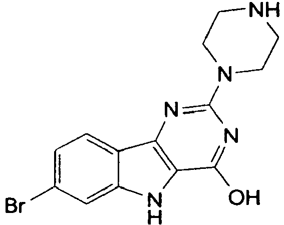 | 348.20 | 348,350 [M+H]⁺ |
| 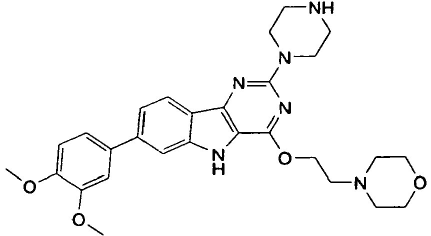 | 518.61 | 519 [M+H]⁺ |
| 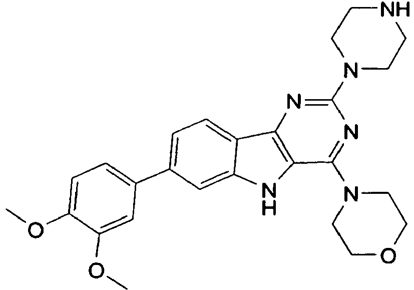 | 474.55 | 475 [M+H]⁺ |
| 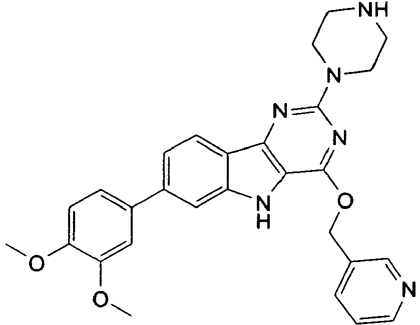 | 496.56 | 497 [M+H]⁺ |
| 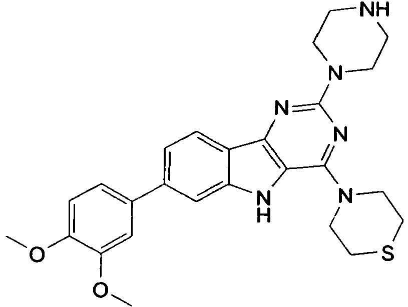 | 490.62 | 491 [M+H]⁺ |
| 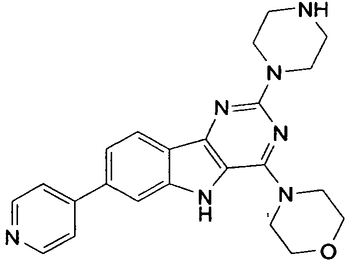 | 415.49 | 416 [M+H]⁺ |
| 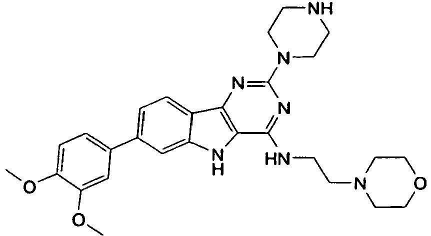 | 517.62 | 518 [M+H]⁺ |
| 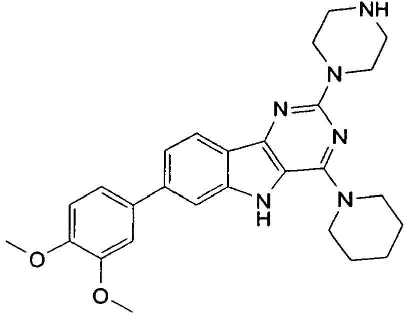 | 472.58 | 473 [M+H]⁺ |
| 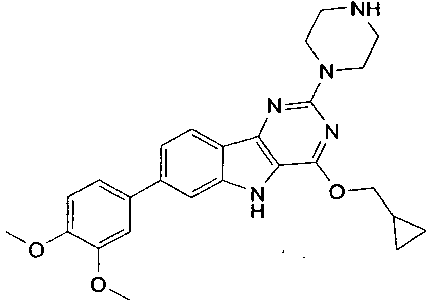 | 459.54 | 460 [M+H]⁺ |
| 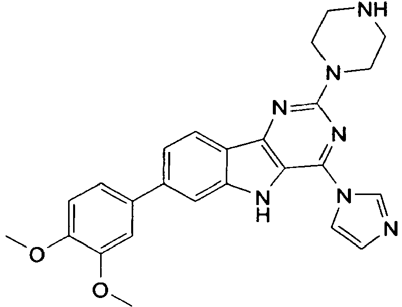 | 455.51 | 456 [M+H]⁺ |
| 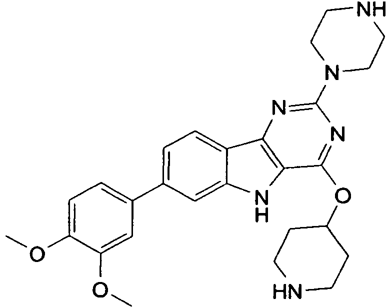 | 488.58 | 489 [M+H]⁺ |
| 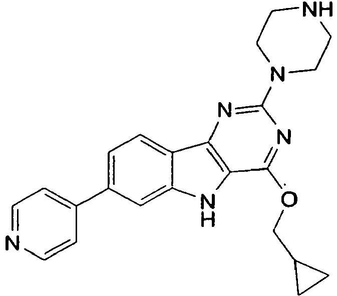 | 400.48 | 401 [M+H]⁺ |
| 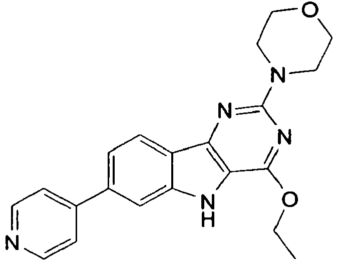 | 375.42 | 376 [M+H]⁺ |
| 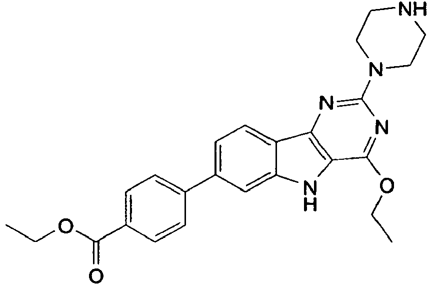 | 445.51 | 446 [M+H]⁺ |
| 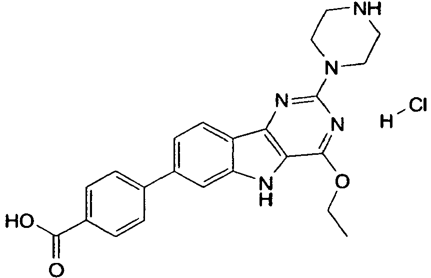 | 453.92 | 418 [M+H]⁺ |
| 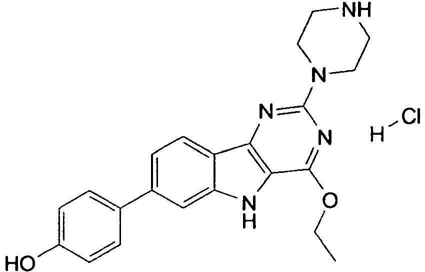 | 425.91 | 390 [M+H]⁺ |
| 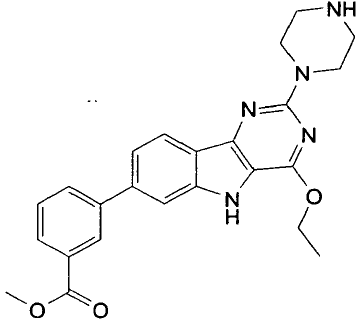 | 431.49 | 432 [M+H]⁺ |
| 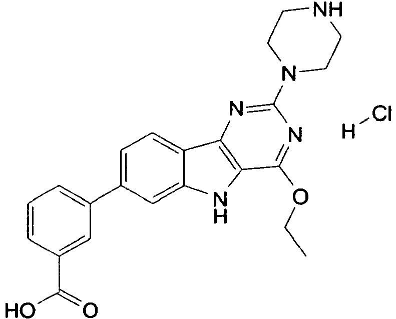 | 453.92 | 418 [M+H]⁺ |
| 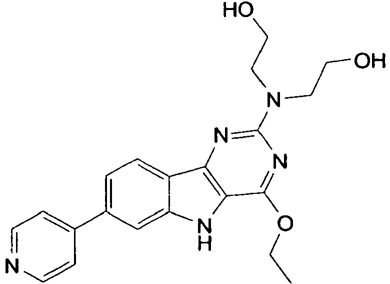 | 393.44 | 394 [M+H]⁺ |
| 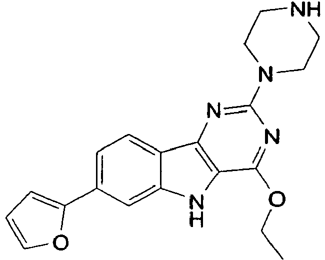 | 463.41 | 464 [M+H]⁺ |
| 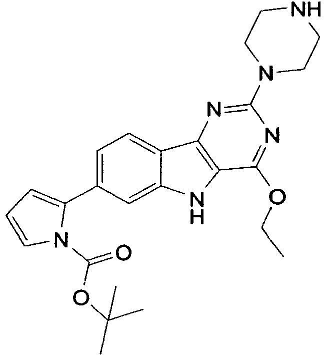 | 462.54 | 463 [M+H]⁺ |
| 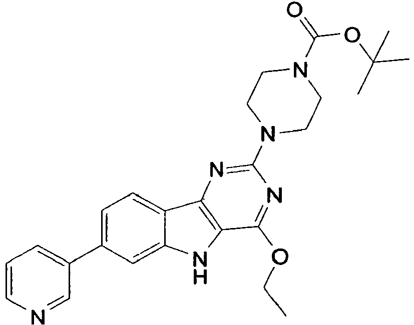 | 474.55 | 475 [M+H]⁺ |
| 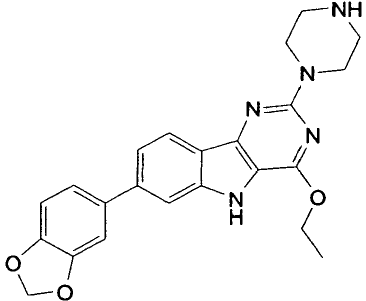 | 417.46 | 418 [M+H]⁺ |
| 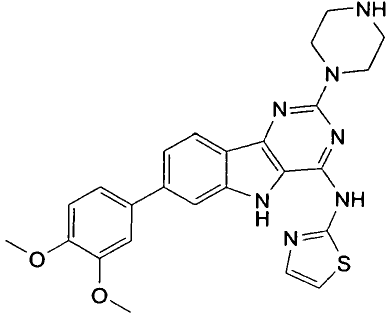 | 487.58 | 488 [M+H]⁺ |
| 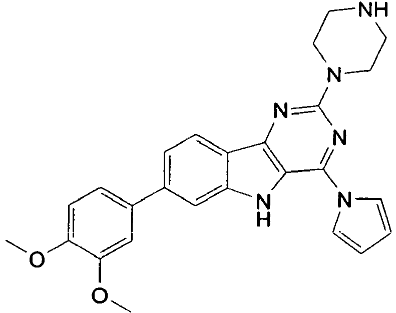 | 454.52 | 455 [M+H]⁺ |
| 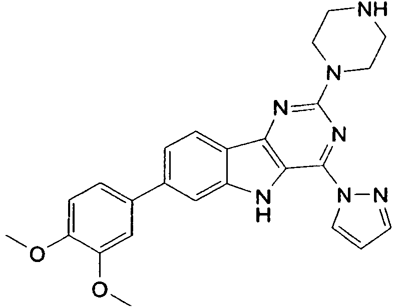 | 455.51 | 456 [M+H]⁺ |
| 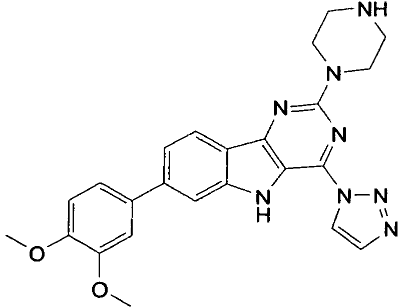 | 456.50 | 457 [M+H]⁺ |
| 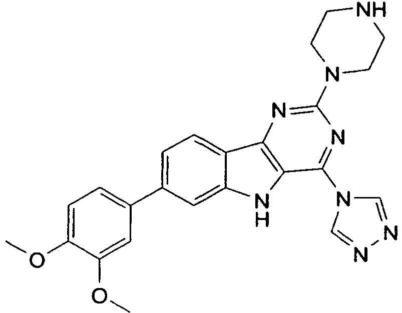 | 456.50 | 457 [M+H]⁺ |
| 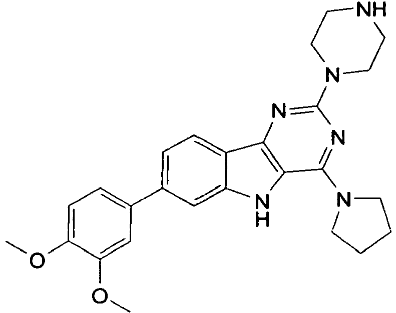 | 458.56 | 459 [M+H]⁺ |

### Beispiel 8

### Die Synthese von 4-Ethoxy-2-(piperazin-1-yl)-6-(pyridin-4-yl)-5H-pyrimido[5,4-b]indol

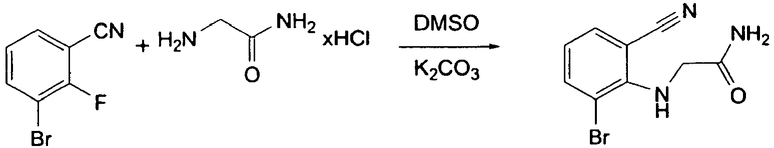

10.0 g (50.0 mmol) 3-Brom-2-fluorbenzonitril, 11.1 g (100.0 mmol) Glycinamidhydrochlorid und 17.3 g (125 mmol) K₂CO₃ wurden in 90 ml DMSO suspendiert und 4 h bei 100 °C erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 10.8 g (85 %) 2-(2-Brom-6-cyano-phenylamino)acetamid. ESI-MS [m/z]: 254, 256 [M+H]⁺

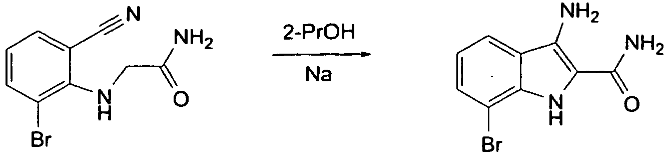

1.17 g (50.9 mmol) Natrium wurden in 150 ml 2-Propanol aufgelöst, anschließend wurden 10.82 g (42.6 mmol) 2-(2-Brom-6-cyano-phenylamino)acetamid zugegeben und die Lösung 15 min unter Rückfluss erhitzt. Anschließend wurde die Reaktionsmischung auf 0 °C abgekühlt und gesätt. NH₄Cl-Lösung zugegeben. Das Lösungsmittel wurde eingeengt, der entstandene Niederschlag abgesaugt und mit Wasser gewaschen. Man erhält 8.6 g (79 %) 3-Amino-7-brom-1*H*-indol-2-carbonsäureamid. ESI-MS [m/z]: 254, 256 [M+H]⁺

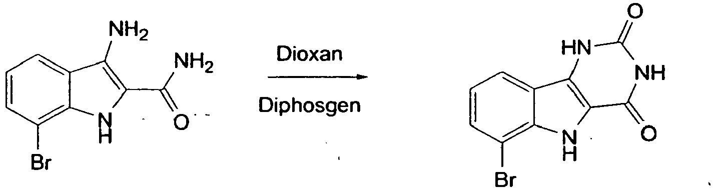

8.0 g (32 mmol) 3-Amino-7-brom-1*H*-indol-2-carbonsäureamid wurden in 50 ml Dioxan suspendiert, nach Zugabe von 3.8 ml (32 mmol) Diphosgen wurde 3 h unter Rückfluß erhitzt. Nach dem Abkühlen auf Raumtemperatur wurden vorsichtig 20 ml Wasser zugegeben und im Anschluss der Niederschlag abgesaugt. Man erhält 9.0 g (32 %) 6-Brom-1*H*-pyrimido[5,4-b]indol-2,4(3*H*,5*H*)-dion. ESI-MS [m/z]: 278, 280 [M-H]⁻

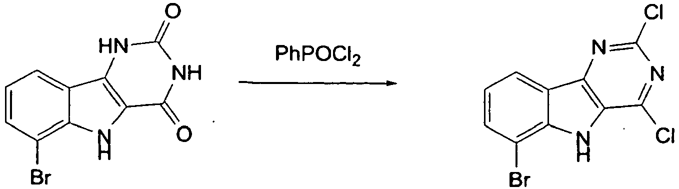

9.0 g (32 mmol) 6-Brom-1*H*-pyrimido[5,4-b]indol-2,4(3*H*,5*H*)-dion und 30 ml (215 mmol) Dichlorphenylphosphinoxid wurden 5 h auf 180 °C erhitzt. Nach Abkühlung auf RT wurde auf 40 g Eiswasser gegossen und mit gesättigter Natriumhydrogencarbonat-Lösung neutralisiert. Der entstandene Niederschlag wurde abgesaugt und mit Wasser gewaschen. Man erhält 8.5 g (84 %) 6-Brom-2,4-dichlor-5*H-*pyrimido[5,4-b]indol. ESI-MS [m/z]: 314, 316, 318 [M-H]⁻

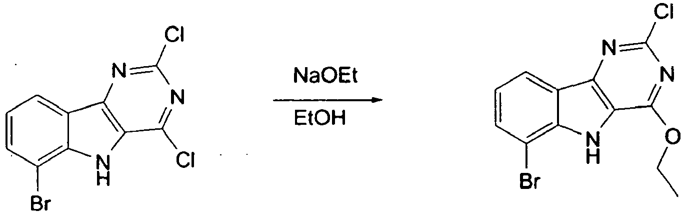

1.0 g (3.2 mmol) 6-Brom-2,4-dichlor-5*H*-pyrimido[5,4-b]indol wurden in 5 ml abs. Ethanol suspendiert und mit 6 ml (6.4 mmol) 1 M Natriumethylat-Lösung (EtOH) versetzt. Das Gemisch wurde 15 h unter Rückfluss erhitzt. Nach dem Abkühlen auf Raumtemperatur wurde das Lösungsmittel eingeengt und Wasser zugegeben. Der entstandene Niederschlag wurde abgesaugt und mit Wasser gewaschen. Man erhält 0.9 g (86 %) 6-Brom-2-chlor-4-ethoxy-5*H*-pyrimido[5,4-b]indol. ESI-MS [m/z]: 324, 326 [M-H]⁻

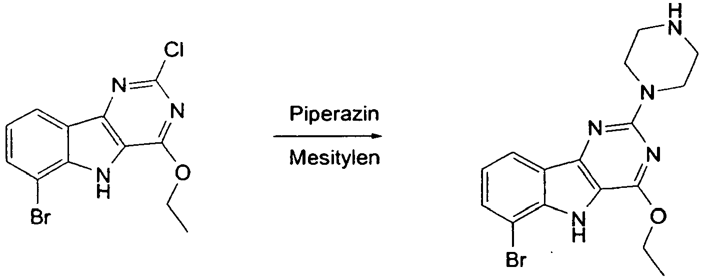

0.8 g (2.4 mmol) 6-Brom-2-chlor-4-ethoxy-5*H*-pyrimido[5,4-b]indol und 0.8 g (9.3 mmol) Piperazin wurden in 10 ml Mesitylen 2.5 h unter Rückfluß erhitzt. Anschließend wurde das Lösungsmittel entfernt und der Rückstand in Wasser suspendiert und abgesaugt. Man erhält 0.8 g (85 %) 6-Brom-4-ethoxy-2-(piperazin-1-yl)-5*H-*pyrimido[5,4-b]indol. ESI-MS [m/z]: 376, 378 [M+H]⁺

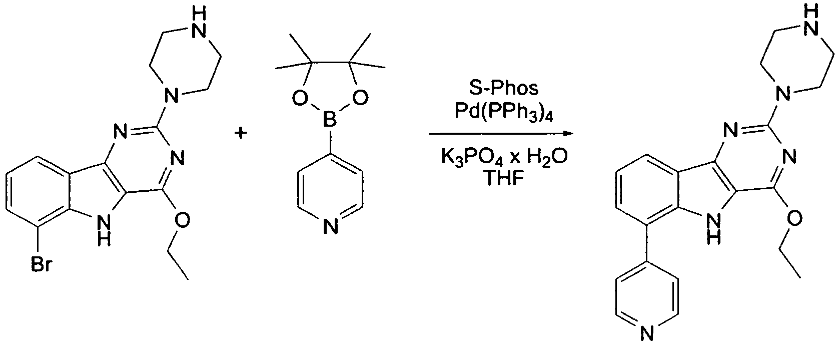

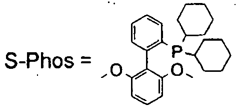

150 mg (0.4 mmol) 6-Brom-4-ethoxy-2-(piperazin-1-yl)-5*H*-pyrimido[5,4-b]indol (Beispiel 4), 183 mg (0.9 mmol) 4-(4,4,5,5-Tetramethyl-1,3,2-dioxaborolan-2-yl)pyridin, 276 mg (1.2 mmol) K₃PO₄ × H₂O, 6.6 mg (16 µmol) S-Phos und 9.2 mg (8 µmol) Pd(PPh₃)₄ in 6 ml THF wurden in einer Mikrowelle 5 h auf 120 °C erhitzt. Anschließend wurde das Lösungsmittel entfernt und nach FC(CH₂Cl₂/MeOH, 0-20 %) erhält man 112 mg (75 %) der Titelsubstanz ESI-MS [m/z]: 375 [M+H]⁺

### Beispiel 9

### Die Synthese von 4-Ethoxy-7-morpholino-2-(piperazin-1-yl)-5H-pyrimido[5,4-b]indol

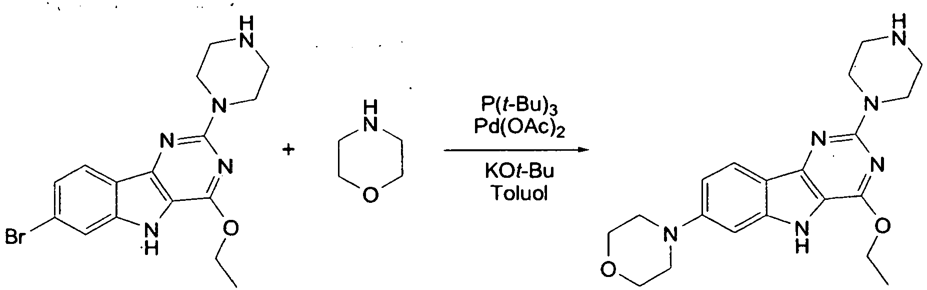

100 mg (0.27 mmol) 7-Bromo-4-ethoxy-2-(piperazin-1-yl)-5*H*-pyrimido[5,4-b]indol (Beispiel 4), 164 µl (1.86 mmol) Morpholin, 179 mg (1.59 mmol) KO*t*-Bu, 1.2 mg (5.4 µmol) Pd(OAc)₂ und 4.4 mg (21.6 µmol) P(t-Bu)₃ wurden in 6 ml Toluol 3 h in einer Mikrowelle auf 140 °C erhitzt. Anschließend wurde das Lösungsmittel entfernt und der Rückstand durch präparative HPLC (RP18) gereinigt. Man erhält 8 mg (8 %) der Titelsubstanz. ESI-MS [m/z]: 383 [M+H]⁺

Folgende Verbindungen wurden analog Beispiel 9 erhalten (Tabelle 4)

**Tabelle 4**

| **Struktur** | **M [g/mol]** | **ESI MS [m/z]** |
|---|---|---|
| 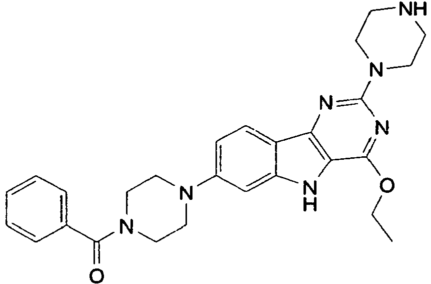 | 485.58 | 486 [M+H]⁺ |
| 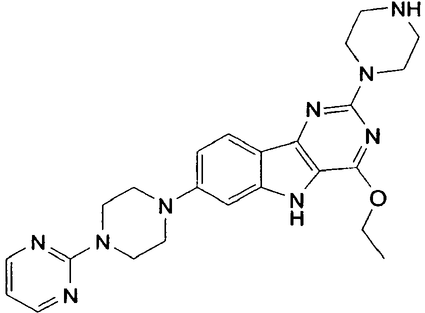 | 459.55 | 460 [M+H]⁺ |

### Beispiel 10

### Die Synthese von 7-Brom-4-ethoxy-2-(4-piperidyl)-5H-pyrimido[5,4-b]indol

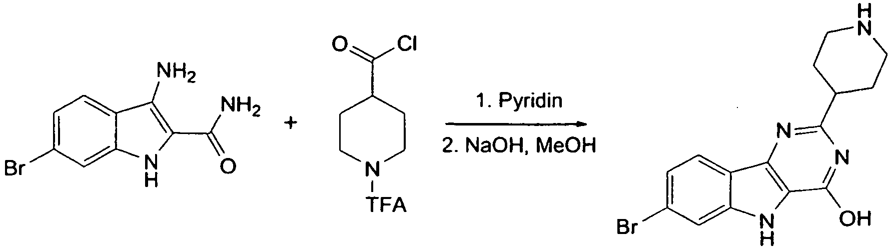

Zu 526 mg (2.10 mmol) 3-Amino-6-brom-1*H*-indol-2-carbonsäureamid (Beispiel 4) in Pyridin wurden bei 0 °C 767 mg (3.15 mmol) 1-(2,2,2-Trifluoroacetyl)piperidin-4-carbonsäurechlorid gelöst in CH₂Cl₂ langsam zugetropft. Anschließend wurde 40 min bei Raumtemperatur gerührt. Nach Zugabe von Wasser wurde das Lösungsmittel im Vakuum entfernt und der Rückstand in Methanol aufgenommen. Nach Zugabe von 2 M NaOH (Überschuss) wurde die Reaktion 45 min am Rückfluss gehalten. Anschließend wurde das Methanol im Vakuum entfernt, der Niederschlag abgesaugt und mit Wasser gewaschen. Man erhält 652 mg (89 %) 7-Brom-2-(4-piperidyl)-5*H-*pyrimido[5,4-b]indol-4-ol. ESI-MS [m/z]: 347, 349 [M+H]⁺

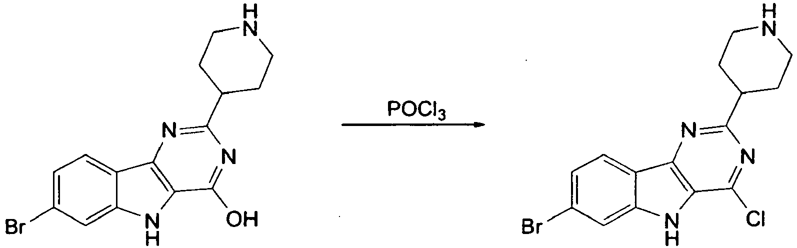

650 mg (1.90 mmol) 7-Brom-2-(4-piperidyl)-5*H*-pyrimido[5,4-b]indol-4-ol wurden 9 h in POCl₃ am Rückfluss gehalten. Anschließend wurde das POCl₃ im Vakuum entfernt, der Rückstand mit Wasser versetzt und mit NaHCO₃ neutralisiert. Der gebildete Niederschlag wurde abgesaugt und mit Wasser gewaschen. Man erhält 500 mg (72 %) 7-Brom-4-chlor-2-(4-piperidyl)-5*H*-pyrimido[5,4-b]indol. ESI-MS [m/z]: 365, 367 [M+H]⁺

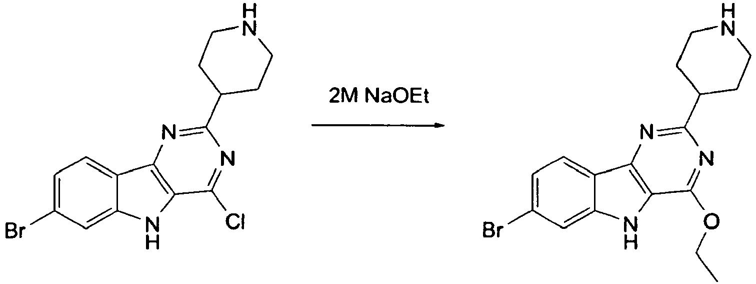

300 mg (0.82 mmol) 7-Brom-4-chlor-2-(4-piperidyl)-5*H*-pyrimido[5,4-b]indol wurden mit einem Überschuss 2 M NaOEt versetzt und 2 h am Rückfluss gehalten. Anschließend wurde Wasser zugegeben und das Lösungsmittel im Vakuum entfernt. Nach FC(CH₂Cl₂/MeOH, 0-50 %) erhielt man 160 mg (52 %) der Titelsubstanz ESI-MS [m/z]: 375, 377 [M+H]⁺

Folgende Verbindungen wurden analog den Beispielen 7 und 10 erhalten (Tabelle 5)

**Tabelle 5**

| **Struktur** | **M [g/mol]** | **ESI MS [m/z]** |
|---|---|---|
| 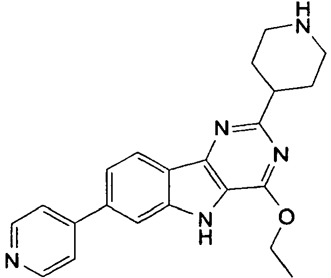 | 373.45 | 374 [M+H]⁺ |
| 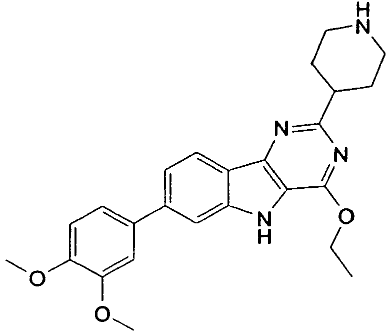 | 432.51 | 433 [M+H]⁺ |
| 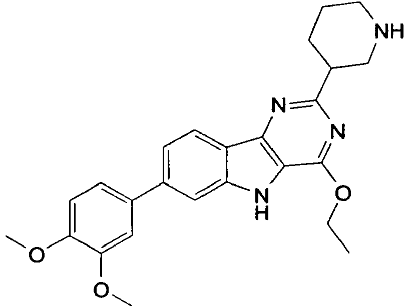 | 432.51 | 433 [M+H]⁺ |
| 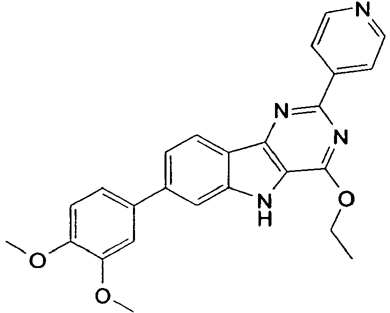 | 426.47 | 427 [M+H]⁺ |
| 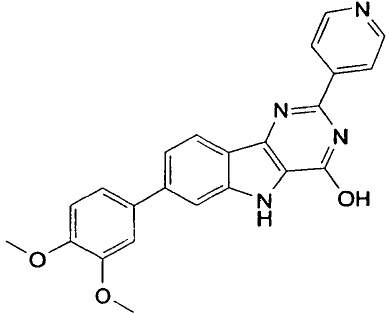 | 398.41 | 399 [M+H]⁺ |

### Beispiel 11

### Induktion von Apoptose bei chronisch lymphatischer Leukämie (B-CLL)

Es wurden erfindungsgemäße Verbindungen als Induktoren einer zytotoxischen Reaktion, d.h. Auslösung von Apoptose, bei chronisch lymphatischer Leukämie (B-CLL) getestet.

Als Positivkontrolle wurde Fludarabin, das bei der B-CLL als Standard-Chemotherapeutikum Anwendung findet, mitgeführt. Mit Fludarabin konnte eine Zytotoxizität bis zu maximal 60% unter den gleichen Versuchsbedingungen erreicht werden.

Die Zytotoxizität der B-CLL Zellen wurde mittels eines kommerziell erhältlichen Formazan Reduktions-Assays (MTT Test) nach 24 h Inkubation des Patientenblutes mit den Wirkstoffen bestimmt.

Die folgende Auflistung zeigt eine Auswahl der erfindungsgemäßen Substanzen, die *ex vivo* im Blut von B-CLL Patienten bei bis zu 100 % der Leukämiezellen eine Zytotoxizität (Apoptose) auslösen und in den nachfolgenden Experimenten auf ihre Fähigkeiten untersucht wurden:
2-(4-Ethoxy-9-methoxy-2-(piperazin-1-yl)-5*H*-pyrimido[5,4-b]indol-7-yl)benzenamin
2-{2-(Piperazin-1-yl)-7-(pyridin-4-yl)-5*H*-pyrimido[5,4-b]indol-4-yloxy}ethanol
4-Ethoxy-2-(4-methylpiperazin-1-yl)-7-(pyridin-4-yl)-5*H*-pyrimido[5,4-b]indol
4-Ethoxy-2-(piperazin-1-yl)-6-(pyridin-4-yl)-5*H*-pyrimido[5,4-b]indol
4-Ethoxy-2-(piperazin-1-yl)-7-(2-aminopyridin-5-yl)-5*H*-pyrimido[5,4-b]indol
4-Ethoxy-2-(piperazin-1-yl)-7-(3,4-dimethoxy-phenyl)-5*H*-pyrimido[5,4-b]indol
4-Ethoxy-2-(piperazin-1-yl)-7-(pyridin-3-yl)-5*H*-pyrimido[5,4-b]indol
4-Ethoxy-2-(piperazin-1-yl)-7-(pyridin-4-yl)-5*H*-pyrimido[5,4-b]indol
4-Ethoxy-2-(piperazin-1-yl)-8-(pyridin-4-yl)-5*H*-pyrimido[5,4-b]indol
4-Ethoxy-7-morpholino-2-(piperazin-1-yl)-5*H*-pyrimido[5,4-b]indol
4-Ethoxy-9-methoxy-2-(piperazin-1-yl)-7-(pyridin-4-yl)-5*H*-pyrimido[5,4-b]indol
4-(4-Ethoxy-2-(piperazin-1-yl)-5*H*-pyrimido[5,4-b]indol-7-yl)benzoesäureethylester
4-(4-Ethoxy-2-(piperazin-1-yl)-5*H*-pyrimido[5,4-b]indol-7-yl)benzoesäure-hydrochlorid
4-(4-Ethoxy-2-(piperazin-1-yl)-5*H*-pyrimido[5,4-b]indol-7-yl)phenol-hydrochlorid
3-(4-Ethoxy-2-(piperazin-1-yl)-5*H*-pyrimido[5,4-b]indol-7-yl)benzoesäuremethylester
3-(4-Ethoxy-2-(piperazin-1-yl)-5*H*-pyrimido[5,4-b]indol-7-yl)benzoesäure-hydrochlorid

### Nachweis der Apoptose-induzierenden Wirkung der erfindungsgemäßen Substanzen auf humane Blutzellen

Um die selektive Wirkung verschiedener Substanzen auf die Vitalität von humanen Zellen zu überprüfen, wurde neben isolierten B-Zellen von Patienten mit chronischer B-Zell Leukämie (B-CLL) auch aufgereinigte weiße Blutkörperchen (PBMC) und Erythrozyten von gesunden Spendern untersucht. Als Modelsystem für die chronisch lymphatische B-Zellleukämie wurde die permanente humane EHEB B-CLL Zelllinie ausgewählt. Es ist literaturbekannt, dass die EHEB Zellen im Vergleich zu primären B-CLL Zellen wesentlich insensitiver auf proapoptotische Signale reagieren. Die Zellen wurden mit Konzentrationsreihen der Testsubstanzen für 3 Tage inkubiert und die Vitalität der Zellen nach diesem Zeitraum mit Hilfe des CellTiter-Glo™ ATP Assays (Promega, Katalognr. G7571) nach Herstellerangaben bestimmt.

### Isolierung und Aufreinigung primärer humaner B-CLL Zellen und gesunder humaner PBMC's

Primäre Tumorzellen werden mittels Dichtegradientenzentrifugation aus Vollblut isoliert. Hierzu wird das Vollblut des Patienten mit 50,0 µl Human B Cell Enrichment Cocktail (RosetteSep; Stem Cell Technology, Cat # 15064) pro Milliliter Vollblut für 20 Minuten bei Raumtemperatur (RT) inkubiert. Anschließend wird das Vollblut im Verhältnis 1:1 mit Phosphat-gepufferter Salzlösung (PBS; PAA, Katalognr. H15-002) mit 2 % fötalem Kälberserum (FCS; PAA, Katalognr. A15-151) verdünnt. Nach Vorlage des benötigten Volumens einer Ficoll Paque Plus Lösung (GE Healthcare, Katalognr. 17-1440-02; siehe Herstellerangaben) in ein Zentrifugationsröhrchen (TPP, Katalognr. 91015, oder 91016, oder 91050) wird diese Lösung mit einem entsprechenden Volumen verdünnten Vollbluts (siehe Herstellerangaben) vorsichtig überschichtet und für 20 Minuten bei 2500 U/min RT zentrifugiert. Nach dieser Zentrifugation wird die zellreiche Interphase / Mittelschicht vorsichtig abgenommen und in ein neues Zentrifugationsröhrchen überführt. Zum Waschen der Zellen wird ein gleiches Volumen PBS / 2 % FCS zugegeben und die Suspension für 5 Minuten bei 1200 U/min und RT zentrifugiert. Nach vorsichtiger Abnahme der Waschlösung wird das Zellpellet in 10 ml komplettem Zellkulturmedium (RPMI 1640, PAA Katalognr. E15-840, mit 10 % FCS, 1 % Penicillin / Streptomycin, PAA, Katalognr. P11-010) resuspendiert. Nach Bestimmung der Zellzahl mittels Trypan-Blau Färbung (Trypan-Blau Lösung von Sigma-Aldrich, Katalognr. T-8154) kann die gewünschte Anzahl an Testplatten vorbereitet werden.

### Vorbereitung isolierter primärer humanere B-CLL Zellen für die Testung

Nach Bestimmung der Zellzahl wird das benötigte Volumen einer Zellsuspension mit je 2,86 x 10⁶ Zellen pro Milliliter in komplettem Zellkulturmedium hergestellt. Die Zellen werden in einer Dichte von 2,86 x 10⁵ Zellen pro Kavität in 70 µl komplettem Zellkulturmedium in eine 96-Kavitäten Messplatte (Costar, Katalognr. 3610) ausgesät und über Nacht bei 37 °C, 5 % CO₂ kultiviert. Die Zugabe der Testsubstanz(en) erfolgt am folgenden Tag. Nach Zugabe der Testsubstanz(en) liegen die Zellen in einer Dichte von 2,00 x 10⁵ in 100 µl komplettem Zellkulturmedium pro Kavität vor.

### Behandlung der Zellen mit Testsubstanz(en)

Entsprechend ihrer Löslichkeit werden die Testsubstanzen in Dimethylsulfoxid (DMSO, Sigma-Aldrich, Katalognr. 276855) in einer Konzentration von 1,00 x 10⁻³ bis 1,00 x 10⁻¹ molar gelöst. Aus diesen Stammlösungen werden Verdünnungsreihen in dem Lösungsmittel DMSO hergestellt; die Anzahl der Verdünnungen ist abhängig von der Anzahl der zu vermessenden Testsubstanzen und der Anzahl der Testplatten. Wenn möglich werden acht Verdünnungen hergestellt, die einen Konzentrationsbereich von ca. 1,00 x 10⁻³ bis 1,00 x 10⁻⁹ molar abdecken. Die Zugabe der Testsubstanz(en) erfolgt in 30 µl komplettem Zellkuturmedium, wobei die Endkonzentration des Lösungsmittels DMSO maximal 1 % beträgt. Als Kontrolle dienen Zellen, welche nur mit dem Lösungsmittel DMSO in einer Endkonzentration von 1% behandelt werden. Nach Zugabe der Testsubstanz-Verdünnung(en) liegen die Zellen in einer Dichte von 2,0 x 10⁵ in 100 µl komplettem Zellkulturmedium pro Kavität vor. Die so behandelten Zellen werden nun für 72 h bei 37 °C, 5 % CO₂ inkubiert.

### Bestimmung der Zytotoxizität

Die toxische Wirkung der Testsubstanz(en) wird mit Hilfe des CellTiter-Glo™ ATP Assays (Promega, Katalognr. G7571) nach Herstellerangaben bestimmt. Bei dieser Methode werden die noch vitalen Zellen anhand des vorhandenen ATPs detektiert, welches von lebenden Zellen für die Aufrechterhaltung des Stoffwechsels benötigt wird. Dieses ATP setzt ein zugegebenes Lumineszenzsubstrat um und generiert damit ein Lichtsignal. Die Lumineszenz wird mit Hilfe des FLUOstar Optima Reader (BMG Labtechnologies) aufgezeichnet und in Zahlenwerte umgewandelt. Die Erstellung einer Dosis-Wirkungskurve erfolgt anschließend mit Hilfe der Sigma-Plot Auswertungssoftware (SYSTAT, Version 6 für Windows), ebenso wie die Bestimmung der effektiven Konzentration, welche 50 % der Zellen tötet (EC₅₀-Konzentration).

### Vitalitätstest

Zum Nachweis der Zellvitalität wurde nach dem Inkubationszeitraum der Fluoreszenzindikator Resazurin in einem Anteil von 10 % (v/v) zupipettiert und die Zellen für weitere 4 h im Brutschrank inkubiert. Dabei wird das nicht-fluoreszente Resazurin durch vitale Zellen in deren Mitochondrien zu einem fluoreszenten Farbstoff umgesetzt, dessen Intensität proportional zur Anzahl der vitalen Zellen ist. Die Fluoreszenzintensität wurde nach dem Inkubationszeitraum bei einer Anregungswellenlänge von 530 nm und einer Emissionswellenlänge von 590 nm am FluostarOPTIMA (BMG Labtechnologies) Mikroplattenreader gemessen.

### Auswertung der Ergebnisse

Anhand der relativen Fluoreszenzwerte wurde der prozentuale Anteil toter Zellen, im Vergleich zur Lösungsmittelkontrolle (DMSO) berechnet und diese Werte gegen die Substanzkonzentrationen aufgetragen. Es wurden Dosis-Wirkungskurven mit dem Programm *Sigma Plot* erstellt und anhand dieser Verlaufskurven die EC₅₀/IC₅₀-Werte für jede Substanz berechnet. Die IC₅₀-Werte für die erfindungsgemäßen Substanzen liegen zwischen 0,1 - 5 µM.

### Einfluss der erfindungsgemäßen Substanzen auf B-CLL Zellen

Die Apoptose-induzierende Wirkung der im Beispiel 11 aufgeführten Substanzen auf gereinigte B-Lymphozyten von Patienten mit chronischer B-CLL zeigte eine starke interindividuelle Schwankungsbreite, aus der ersichtlich wurde, dass es bei den Patienten Gruppen gab, die stark, mittel und schwach auf die erfindungsgemäßen Wirkstoffe reagierten. In Tab.6 sind die Ergebnisse der Apoptose-induzierenden Wirkung der Substanzen dargestellt.

**Tab. 6 Einfluss der erfindungsgemäßen Substanzen auf die Vitalität von B-CLL Zellen**

| **Responder** | **Anzahl Patienten** | **Zytotoxizität (EC₅₀ µM)** |
|---|---|---|
| Gesamt | n = 30 | 1,83 ± 0,95 |
| stark | n = 9 | 0,679 ± 0,45 |
| mittel | n = 19 | 2,081 ± 1,32 |
| schwach | n = 2 | 7,24 ± 3,61 |

stark (EC₅₀ 10nM -1µM)
mittel (EC₅₀ 1,1µM - 5µM)
schwach (EC₅₀ > 5µM)

Tabelle 6 gibt Mittelwerte der in Beispiel 11 genannten Verbindungen wieder,

Wie aus der Tabelle 6 ersichtlich ist, wirken die erfindungsgemäßen Substanzen auf die leukämischen B-CLL Zellen zytotoxisch. Auffällig war, dass bei dem untersuchten Patientenblut eine unterschiedliche Ansprechbarkeit auf die erfindungsgemäßen Substanzen zu beobachten war.

### Einfluss der erfindungsgemäßen Substanzen auf die Vitalität von leukämischen B-Zellen bei B-CLL Patienten mit einer 11q Deletion

Wie nachfolgend exemplarisch dargestellt, reagieren die leukämischen B-Lymphozyten von B-CLL Patienten mit einer 11q Deletion besonders empfindlich auf die Apoptose-induzierende Wirkung der erfindungsgemäßen Substanzen. Das nachfolgende Bild stellt beispielhaft die Wirkung der erfindungsgemäßen Substanz 4-Ethoxy-2-(piperazin-1-yl)-7-(pyridin-4-yl)-5H-pyrimido[5,4-b]indol bei solch einem Patienten dar.

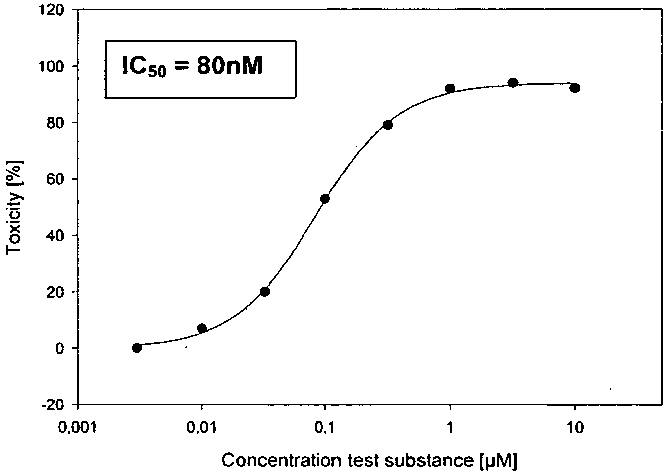

Wie dargestellt, liegt der IC₅₀ mit 80nM verglichen mit dem Durchschnittswert aller B-CLL Patienten(IC₅₀ 1,83 ± 0,95µM) deutlich besser, was belegt, dass Patienten mit dieser chromosomalen Deletion wesentlich empfindlicher auf die Behandlung mit den erfindungsgemäßen Substanzen ansprechen.

Im Weiteren interessierte, ob die erfindungsgemäßen Substanzen auch in gesunden Blutzellen eine Apoptose auslösen können.

**Tabelle 7: Einfluss der erfindungsgemäßen Substanzen auf die Vitalität von gesunden PBMC's**

| **PBMC Zellen (EC₅₀ µM)** | **B-CLL Zellen (EC₅₀ µM)** |
|---|---|
| 29,49 ± 13,4 µM | 1,83 ± 0,95 |
| n =23 | n=30 |

Wie aus der Tabelle 7 ersichtlich, werden beispielhaft durch die erfindungsgemäßen Substanzen B-CLL Zellen mit einem EC₅₀ von 1,83 ± 0,95 µM ca. sechzehnfach stärker beeinflusst, als gesunde PBMC Zellen mit einem EC₅₀ von 29,49 ± 13,4 µM. Hiermit konnte zweifelsfrei gezeigt werden, dass die erfindungsgemäßen Substanzen einen sehr guten therapeutischen Effekt auf Leukämiezellen zeigen, ohne die anderen gesunden Blutzellen anzugreifen. Auch der unmittelbare Wirkvergleich von Fludarabin als momentaner "golden standard" mit den erfindungsgemäßen Substanzen im Blut von B-CLL Patienten, zeigt einen EC₅₀ Wert von 2 µM bis 200 µM für Fludarabin (Literatur- und eigene Werte), während die erfindungsgemäßen Substanzen EC₅₀ Werte von 10 nM bis 5 µM aufwiesen.

### Apoptose-Nachweis

Um die spezifische Induktion des programmierten Zelltods (Apoptose) festzustellen wurde ein Nachweis desselben anhand der Caspase-3/7 Aktivität durchgeführt; die Caspase-3/7 Aktivität wird in der Literatur als ein sicherer Nachweis der Apoptose beschrieben.

Die EHEB Zellen wurden wie oben beschrieben präpariert und mit drei Konzentrationsverdünnungen, jeweils in Dreifachbestimmung, für 4.5 und 24 Stunden inkubiert. Dabei wurden Substanz-Endkonzentrationen von 50,0, 10,0 und 2,00 µM für die 4 Stunden Ansätze, sowie 10,0, 1,00 und 0,50 µM für die 24 Stunden Ansätze ausgewählt. Als Positiv-Kontrolle fungierte dabei Staurosporin in einer Endkonzentration von 2,00 µM (4 Stunden Ansätze) und 0,50 µM (24 Stunden Ansätze). Zum Nachweis der Caspase-3/7 Aktivität wurde das Caspase-Glo 3/7 und Caspase-Glo 9 Assay der Firma Promega verwendet; dabei wurde die Enzymaktivität anhand eines Lumineszenzsignals festgestellt, welches mit dem FluostarOPTIMA Messgerät (BMG Labtechnologies) aufgezeichnet wurde.

Die Auswertung der Ergebnisse erfolgte durch den Vergleich der relativen Lumineszenzintensität der Testsubstanzen im Vergleich zur Positiv-Kontrolle (Staurosporin = 100%) mit Hilfe des Excel Programms. Die relativen Intensitäten für die erfindungsgemäßen Substanzen liegen bis zu 100 % bei einer Inhibitorkonzentration zwischen 1 - 50 µM.

Als Negativkontrolle in diesem Versuchsansatz diente DMSO in der gleichen Endkonzentration von 1%, in der auch die Wirkstoffe gelöst waren.

Das nachfolgende Bild stellt beispielhaft die Wirkung der erfindungsgemäßen Substanz 4-Ethoxy-2-(piperazin-1-yl)-7-(pyridin-4-yl)-5H-pyrimido[5,4-b]indol dar.

### Aktivierung der Caspasen 3, 7 und 9 durch eine beispielhafte erfindungsgemäße Substanz

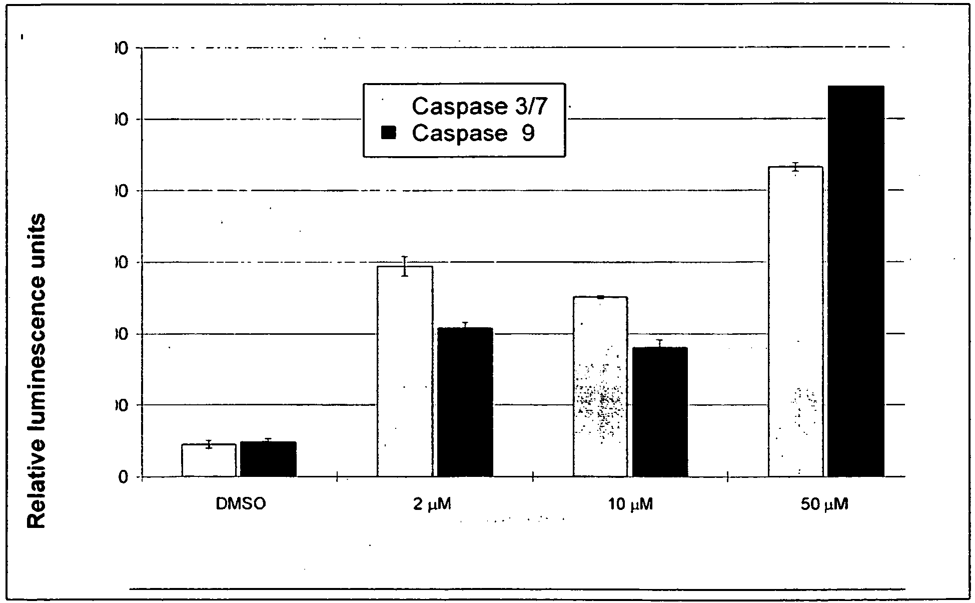

Wie ersichtlich ist, werden in der permanenten B-CLL Zelllinie EHEB nach Inkubation mit den erfindungsgemäßen Substanzen in einer Konzentrationsabhängigkeit die für die Apoptose entscheidenden Triggerenzyme, die Caspasen 3, 7 und 9 aktiviert. Diese Befunde iassen sich auch in den ieukämischen B-CLL Zellen bestätigen (Ergebnisse hier nicht dargestellt). Diese Daten belegen eindeutig, dass die durch die erfindungsgemäßen Substanzen verursachte Zellzytotoxizität kein toxischer Effekt ist, der zur Nekrose der Zellen führt, sondern dass es sich hierbei um eine Induktion der Apoptose handelt.

### Beispiel 12

### Hämolyseassay

Aus dem verbliebenen Erythrozytenrückstand nach Gewinnung der PBMC, wurde ein entsprechendes Volumen an Erythrozyten entnommen und für den Test eingesetzt. Hierbei wurden die Erythrozyten 1:100 (v/v) in RPMI-1640 Medium (Phenolrot-frei) mit 2% Ultroser HY verdünnt. Je 100 µl dieser Suspension wurden in die Wells einer 96 well Rundbodenplatte pipettiert. Anschließend wurden 100 µl einer 2-fach konzentrierten Testsubstanzverdünnungsreihe und je einer 2-fach konzentrierten Saponin-Verdünnungsreihe zu den Erythrozyten zugegeben. Die Bestimmung erfolgte in Dreifachansätzen. Als Positivkontrolle diente Saponin. Die Ansätze wurden für 2 Std. im Dunkeln bei Raumtemperatur auf einem Schüttler inkubiert. Danach wurde die Platte bei 300 RPM für 10 min. zentrifugiert und je 100 µl der Überstände in eine neue 96-well Flachbodenplatte transferiert. Die Bestimmung der Lyse erfolgte durch Messung der Hämoglobinabsorption bei 414 nm gegen eine Referenzwellenlänge von 620 nm.

Die nachfolgende Bild stellt beispielhaft die Wirkung der erfindungsgemäßen Substanz 4-Ethoxy-2-(piperazin-1-yl)-7-(pyridin-4-yl)-5H-pyrimido[5,4-b]indol dar.

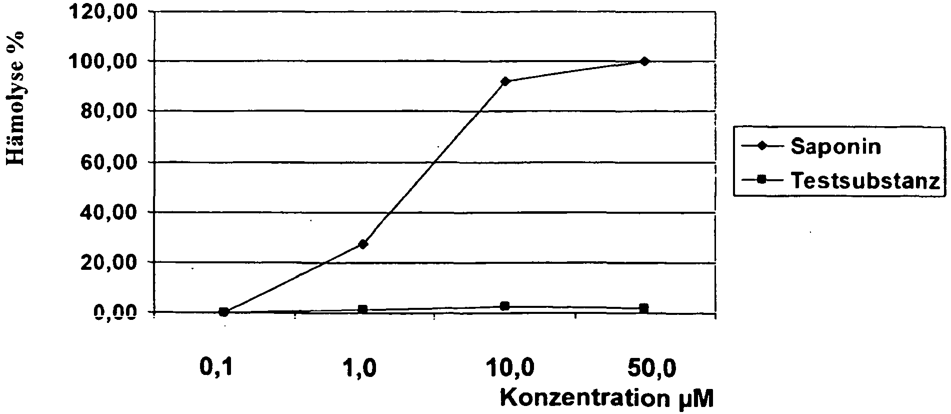

Die erfindungsgemäßen Substanzen hämolysieren im Gegensatz zur Positivkontrolle Saponin, die humanen Erythrozyten nicht.

### Beispiel 13

### Synergistische Wirkung der erfindungsgemäßen Substanzen mit Fludarabin

Werden gereinigte B-CLL Zellen mit den erfindungsgemäßen Substanzen zusammen mit dem Nukleosid Analogon Fludarabin inkubiert, dann ergibt ich eine synergistische Wirkung der beide Substanzen auf die Induktion der Apoptose.

Das nachfolgende Bild stellt beispielhaft die Wirkung der erfindungsgemäßen Substanz 4-Ethoxy-2-(piperazin-1-yl)-7-(pyridin-4-yl)-5H-pyrimido[5,4-b]indol dar.

### Synergistische Wirkung einer beispielhaften erfindungsgemäßen Substanz mit Fludarabin auf die Induktion der Apoptose in B-CLL Zellen

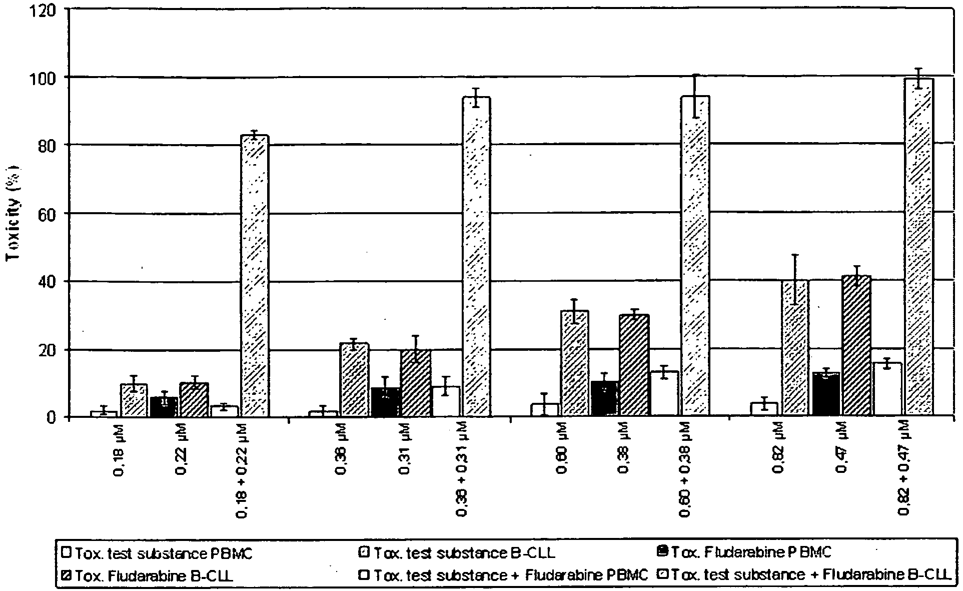

Wie dargestellt, erreicht man in der Kombination einer beispielhaften erfindungsgemäßen Substanz mit Fludarabin schon bei einer 10%igen toxischen Konzentration jedes Partners eine Gesamztoxizität der B-CLL Zellen von über 80%. Bei einem additiven Effekt der beiden Substanzen wären nur ca. 20% Toxizität zu erwarten gewesen. Wird die Konzentration der beiden Substanzen soweit erhöht, dass jede Substanz in einem gesonderten Versuch 20% der Zellen abtötet, dann erzielt man in diesem Synergieexperiment eine Toxizität von nahezu 100%. Diese Daten belegen, dass bei einer kombinierten klinischen Behandlung von Fludarabin mit einer der erfindungsgemäßen Substanzen die Dosierung wesentlich verringert werden könnte und dadurch mögliche unerwünschte Nebenwirkungen minimiert bzw. ausgeschlossen werden könnten.

## Patentansprüche

1. Verbindungen der allgemeinen Formel 1 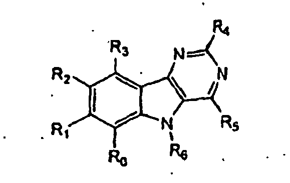 in welcher die Substituenten die nachstehende Bedeutung haben:
R₀, R₁ bzw. R₂
- L_{A}-A-L_{B}-B, worin bedeuten:
L_{A}:
- Einfachbindung,
- NR^{#}, O, S, S(O), S(O)₂, S(O)₂-O, O-S(O)₂, -CHR^{§}-, -CH₂-O-, -CH₂-CH₂-O-, -O-CH₂, -O-CH₂-CH₂-, C=O,
A:
- Wasserstoff,
- C₁₋₆Alkyl (ggf. mit R^{§} substituiert),
- C₂₋₆Alkenyl (ggf. mit R^{§} substituiert),
- C₂₋₆Alklnyl (ggf. mit R^{§} substituiert),
- Chlor, Brom, lod,
- Azido, Hydrazino,
- Phenyl, ggf. ein-, zwei- oder dreifach mit unabhängig voneinander ausgewählten Resten R^{§} substituiert
- ein mono- oder bicyclischer gesättigter oder ein- oder mehrfach ungesättigter Heterocyclus mit 4 - 14 Ringatomen darunter 1 - 5 Heteroatome (vorzugsweise N, O und S) der ein oder mehrere Sauerstoff-Atome an C, N und/oder S tragen kann und ggf. mit R^{§} substituiert ist
und für den Fall, dass A nicht weiter substituierbar ist, L_{B} und B gegenstandslos sind,
L_{A}-A sowie L_{A}-A-L_{B} jeweils gemeinsam auch eine Einfachbindung bedeuten können
L_{B}:
- Einfachbindung,
- NR^{#}, O, S, S(O), S(O)₂, S(O)₂-O, O-S(O)₂, -CHR^{§}-, -CH₂-O-, -CH₂-CH₂-O-, -O-CH₂, -O-CH₂-CH₂-, C=O,
- folgende funktionelle Gruppen: 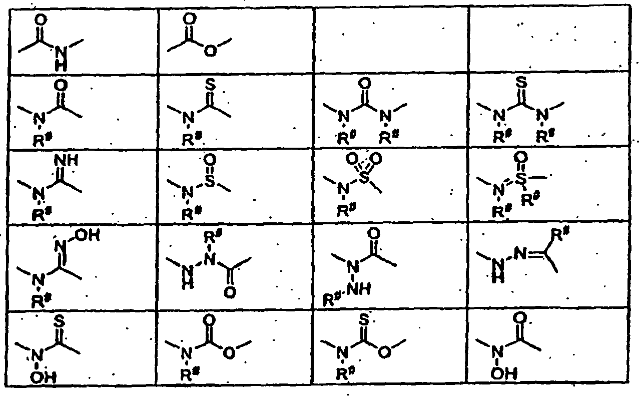
B:
- Wasserstoff
- Alkyl, ggf. mit R^{§} substituiert
- C₂₋₆Alkenyl (ggf. mit R^{§} substitulert),
- C₂₋₆Alkinyl (ggf, mit R^{§} substituiert).
- Aryalkyl- mit C₆₋₁₂Aryl und C₁₋₅ Alkyl, (Alkyl und/oder Arylggf. mit R^{§} substituiert),
- Alkyl, ein- oder mehrfach substituiert mit mono- oder bicyclischen gesättigten oder ein- oder mehrfach ungesättigten Heterocyclen mit 4 - 14 Ringatomen darunter 1 - 5 Hetero-atome (vorzugsweise N, O, S), die ggf. ein- zwei- oder dreifach mit unabhängig voneinander ausgewählten Resten R^{§} und/ oder ein oder mehreren Sauerstoffatomen substituiert sein können.
- Aryl, insb. Phenyl, ggf. mit R^{§} substituiert
- ein mono- oder bicyclischer gesättigter oder ein- oder mehrfach ungesättigter Heterocyclus mit 4 - 14 Ringatomen, darunter 1 - 5 Heteroatome (vorzugsweise N, O, S), der ggf. ein- zwei- oder dreifach mit unabhängig voneinander ausgewählten Resten R^{§} und/ oder ein oder mehreren Sauerstoffatomen substituiert sein kann.
R^{#} - Wasserstoff, Alkyl (ggf. mit R^{§} substituiert)
R₃
- Wasserstoff,
- C₁₋₆Alkyl, geradkettig, verzweigt oder C₃₋₆Alkyl auch cyclisch sowie ggf. ein-, zwei- oder dreifach mit unabhängig voneinander ausgewählten Resten R^{§} substituiert,
R^{§}
- C₁₋₆ Alkoxy, geradkettig, verzweigt oder C₃₋₆Alkoxy auch cyclisch sowie ggf. ein-, zwei- oder dreifach mit unabhängig voneinander ausgewählten Resten R^{§} substituiert.
- ein monocyclischer gesättigter oder ein- oder mehrfach ungesättigter Heterocyclus mit 4 - 8 Ringatomen darunter 1 - 3 Heteroatomen, vorzugsweise N, O und S
R₄
- NR₇R₈, worin dieser Substituent insgesamt bedeutet:
- Morpholino, Thiomorpholino, Thiomorpholino-S,S,-dioxid, Pyrrolidino, Piperidino, Piperazin-1-yl, 1-Homopiperazinyl, 4-C₁₋₆-Alkyl-Piperazin-1-yl, 4-Aryl-1-Piperazin-1-yl, 4-Bn-Piperazin-1-yl (ggf. mit R^{§} am heterocycloaliphatischen Ring substituiert),
- weitere Amino-Reste sekundärer, mono- oder polycyclischer Amine mit insgesamt 4-14 Ringatomen, einschließlich der am C-Skelett mit R^{§} substituierten Vertreter
R₅
- mono-, bi- oder trocyclischer gesättigter oder ein- oder mehrfach ungesättigter heterocyclischer Rest mit insgesamt 4-14 Ring-Atomen, davon 1-5 Heteroatomen, die vorzugsweise N, O und S sind, ggf. mit R^{§} substituiert,
- C₁₋₆ Alkyl, C₁₋₆ Alkoxy, C₁₋₆ Alkylthio, jeweils geradkettig, verzweigt oder cyclisch und ggf. mit R^{§} substituiert
- ein über ein exocyclisches Atom aus der Gruppe O, N, S gebundener gesättigter oder ein- oder mehrfach ungesättigter heterocyclischer Rest mit insgesamt 4-10 Ring-Atomen, davon 1-5 Heteroatomen, die vorzugsweise N, O und S sind, ggf. mit R^{§} substituiert,
- ein über ein am Tricyclus gebundenes Atom aus der Gruppe O, N oder S und eine nachfolgend angeordnete C₁₋₆ Alkylen-Gruppe gebundener gesättigter oder ein- oder mehrfach ungesättigterheterocyclischer Rest mit insgesamt 4-10 Ring-Atomen, davon 1-5 Heteroatomen (vorzugsweise N, O und S), ggf. mit R^{§} substituiert,
- Hydroxy, Halogen (Cl, Br, I)
R₆
- Wasserstoff,
- C₁₋₆Alkyl, geradkettig, verzweigt oder C₃₋₆Alkyl auch cyclisch sowie ggf. ein-, zwei- oder dreifach mit unabhängig voneinander ausgewählten Resten R^{§} substituiert
- C₂₋₆Alkenyl (ggf. mit R^{§} substituiert)
- Alkyl, einfach oder mehrfach, mit mono- oder bicyclischen gesättigten oder ein- oder mehrfach ungesättigten Heterocyclen mit 4 - 14 Ringatomen darunter 1 - 5 Heteroatome, die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sein können, substituiert sowie ggf, mit R^{§} substituiert
- Carbonyl oder Sulfonyl, jeweils substituiert mit
- Wasserstoff, C₁₋₆Alkyl (ggf. mit R^{§} substituiert),
- C₂₋₆Alkenyl (ggf. mit R^{§} substituiert),
- C₂₋₆Alkinyl (ggf. mit R^{§} substituiert),
- Aryl, insb. Phenyl (ggf. mit R^{§} substituiert),
- mono- oder bicyclischer gesättigter oder ein- oder mehrfach ungesättigter Heterocyclus mit 4 - 14 Ringatomen darunter 1 - 5 Heteroatome; die vorzugsweise N, O und S sind, die ein oder mehrere Sauerstoff-Atome an C, N und/oder S tragen können und ggf. ein-, zwei- oder dreifach mit unabhängig voneinander ausgewählten Resten R^{§} substituiert sein können,
R^{§}
-OH, -SH, -O-C₁₋₆Alkyl, -O-C₆₋₁₄Aryl, -S-C₁₋₄ Alkyl, -S-C₆₋₁₄Aryl,
-SO-C₁₋₄Alkyl, -SO-C₆₋₁₄Aryl, -SO₂-C₁₋₄Alkyl, -SO₂-C₆₋₁₄Aryl, -SO₃H,
-OSO₂C₁₋₆Alkyl, -OSO₂O₆₋₁₄Aryl, -COOH, -COOC₁₋₆Alkyl,
-(CO)C₁₋₆Alkyl,
-COOH, -CONH₂, -CONHC₁₋₆Alkyl, -CON(C₁₋₆Alkyl)₂,
-NH₂, -NHC₁₋₆Alkyl, -N(C₁₋₆Alkyl)₂, -NHC₆₋₁₄Aryl, -NH-Hetaryl,
-N(C₆₋₁₄Aryl)₂, -N(C₁₋₆Alkyl)(C₆₋₁₄Aryl),
-C₁₋₆Alkyl, -C₂₋₁₂ Alkenyl, -C₂₋₁₂Alkinyl, jeweils geradkettig, verzweigt oder cyclisch sowie ggf. ein-, zwei- oder dreifach unabhängig voneinander mit Halogen substituiert,
-Halogen (-F, -Cl, -Br, -I),
-CH₂CH₂OH, -CH₂CH₂SH, -CH₂CH₂SCH₃,
-Sulfamoyl, Alkylsulfamoyl, Dialkylsulfamoyl mit Alkyl C₁₋₅ ggf. substituiert mit Methoxy,
- Amidino, Hydroxyamidino
- Sulfo, Phosphono,
- CN, -NO₂, und -SCN
sowie pharmazeutisch verträgliche Salze, Solvate, Täutomere und Prodrugs dieser Verbindungen,
wobei Verbindungen mit:
- R₀=R₁=R₂=R₃=H
vom Schutz ausgenommen sind.

2. Verbindungen nach Anspruch 1, wobei die Substituenten die nachstehende Bedeutung haben:
R₀, R₁ bzw. R₂ werden unabhängig voneinander ausgewählt aus :
- Chlor, Brom, lod, Azido, Hydrazino
- L_{A}-A-L_{B}-B, worin bedeuten:
L_{A}:
- Einfachbindung,
- NR^{#}, O, S, S(O)₂, C=O,
A:
- Wasserstoff,
- C₁₋₆Alkyl (ggf. mit R^{§a} substituiert),
- C₂₋₆Alkenyl (ggf. mit R^{§a} substituiert),
- C₂₋₆Alkinyl (ggf. mit R^{§a} substituiert),
- Phenyl, ggf. ein-, zwei- oder dreifach mit unabhängig voneinander ausgewählten Resten R^{§b} substituiert
- ein mono- oder bicyclischer gesättigter oder ein- oder mehrfach ungesättigter Heterocyclus mit 4 - 10 Ringatomen darunter 1 - 5 Heteroatome (vorzugsweise N, O und S), ggf. durch ein oder mehrere Sauerstoff-Atome und/ oder einen Rest R^{§b} substituiert,
und für den Fall, dass A nicht weiter subtituierbar ist, sind L_{B} und B gegenstandslos,
L_{A}-A sowie L_{A}-A-L_{B} können jeweils gemeinsam auch eine Einfachbindung bedeuten
L_{B}:
- Einfachbindung,
- NR^{#}, O, S, S(O)₂, C=O
- folgende funktionelle Gruppen: 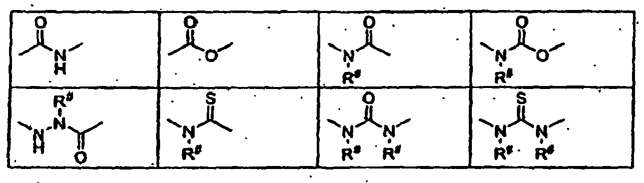
B:
- Wasserstoff
- Alkyl, ggf. mit R^{§a} substituiert
- C₂₋₆Alkenyl (ggf. mit R^{§a} substituiert),
- C₂₋₆Alkinyl (ggf. mit R^{§a} substituiert),
- Aryl, insb. Phenyl, ggf. mit R^{§b} substituiert
- ein mono- oder bicyclischer gesättigter oder ein- oder mehrfach ungesättigter Heterocyclus mit 4 - 10 Ringatomen, darunter 1 - 5 Heteroatome (vorzugsweise N, O, S), der ggf. ein- zwei- oder dreifach mit unabhängig voneinander ausgewählten Resten R^{§b} und/ oder ein oder mehreren Sauerstoffatomen substituiert sein kann
R^{#} - Wasserstoff, Alkyl (ggf. mit R^{§} substituiert)
R₃
- Wasserstoff,
- C₁₋₆Alkyl, geradkettig, verzweigt sowie ggf. ein-, zwei- oder dreifach mit unabhängig voneinander ausgewählten Resten R^{§a} substituiert,
- R^{§b}
- C₁₋₆ Alkoxy, geradkettig, verzweigt oder C₃₋₆ Alkoxy auch cyclisch sowie ggf. ein-, zwei- oder dreifach mit unabhängig voneinander ausgewählten Resten R^{§} substituiert.
- ein monocyclischer gesättigter oder ein- oder mehrfach ungesättigter Heterocyclus mit 4 - 8 Ringatomen darunter 1 - 3 Heteroatomen, vorzugsweise N, O und S
R₄
- NR₇R₈, worin dieser Substituent insgesamt bedeutet:
- Morpholino, Thiomorpholino, Thiomorpholino-S,S,-dioxid, Pyrrolidino, Piperidino, Piperazin-1-yl, 1-Homopiperazinyl, 4-C₁₋₆-Alkyl-Piperazin-1-yl, 4-Aryl-1-Piperazin-1-yl, 4-Bn-Piperazin-1-yl (ggf. mit R^{§a} am heterocycloaliphatischen Ring substituiert),
- weitere Amino-Reste sekundärer, mono- oder polycyclischer Amine mit insgesamt 4-14 Ringatomen, einschließlich der am C-Skelett mit R^{§} substituierten Vertreter
R₅
- ein gesättigter oder ein- oder mehrfach ungesättigter heterocyclischer Rest mit insgesamt 4-8 Ring-Atomen, davon 1-3 Heteroatomen, die vorzugsweise N, O und S sind, ggf. mit R^{§b} substituiert,
- C₁₋₆ Alkoxy, C₁₋₆ Alkylthio, jeweils geradkettig, verzweigt oder cyclisch sowie ggf. mit R^{§a} substituiert
- ein über ein exocyclisches Atom aus der Gruppe O, N, S gebundener gesättigter oder ein- oder mehrfach ungesättigter heterocyclischer Rest mit insgesamt 4-10 Ring-Atomen, davon 1-5 Heleroatomen (vorzugsweise N, O und S), ggf. mit R^{§b} substituiert,
- ein über ein am Tricyclus gebundenes Atom aus der Gruppe O, N oder S und eine nachfolgend angeordnete C₁₋₆ Alkylen-Gruppe gebundener gesättigter oder ein- oder mehrfach ungesättigter heterocyclischer Rest mit insgesamt 4-10 Ring-Atomen, davon 1-5 Heteroatomen (vorzugsweise N, O und S), ggf. mit R^{§b} substituiert.
- Hydroxy, Halogen (Cl, Br, I)
R₆
- Wasserstoff,
- C₁₋₆Alkyl, geradkettig oder verzweigt sowie ggf. ein-, zwei- oder dreifach mit unabhängig voneinander ausgewählten Resten R^{§a} substituiert
- C₂₋₆Alkenyl (ggf. mit R^{§a} substituiert)
- Carbonyl oder Sulfonyl, jeweils substituiert mit C₁₋₈Alkyl (ggf. mit R^{§a} substituiert),
R^{§a}
-OH, -O-C₁₋₈Alkyl, -S-C₁₋₄ Alkyl, -SO₂-C₁₋₄Alkyl, -COOH, -COOC₁₋₈Alkyl,
-CONH₂, -CONHC₁₋₆Alkyl, -CON(C₁₋₆Alkyl)₂, -NH₂, -NHC₁₋₆Alkyl,
-N(C₁₋₆Alkyl)₂,
-Halogen (-F, -Cl, -Br, I),
-CN, -SCN
R^{§b}
-OH, -O-C₁₋₆Alkyl
-SO₂-C₁₋₄Alkyl, -SO₃H, -OSO₂C₁₋₈-Alkyl,
-COOH, -COOC₁₋₈Alkyl,
-CONH₂, -CONHC₁₋₆Alkyl, -CON(C₁₋₆Nkyl)₂,
-NH₂, -NHC₁₋₆Alkyl, -N(C₁₋₆Alkyl)₂,
-C₁₋₆Alkyl, -C₂₋₆ Alkenyl, -C₂₋₆Alkinyl, jeweils geradkettig, verzweigt oder cyclisch sowie ggf. ein-, zwei- oder dreifach unabhängig voneinander mit Halogen substituiert,
-Halogen (-F, -Cl, -Br, -I),
-CH₂CH₂OH, -CH₂CH₂SCH₃,
-sulfamoyl, Alkylsulfamoyl, Dialkylsulfamoyl mit Alkyl C₁₋₅ ggf. substitulert mit Methoxy,
-Amidino, Hydroxyamidino
-Sulfo, Phosphono,
-CN, -NO₂, und-SCN
sowie pharmazeutisch verträgliche Salze, Solvate, Tautomere und Prodrugs dieser Verbindungen,
wobei Verbindungen mit:
- R₀=R₁=R₂=R₃=H
vom Schutz ausgenommen sind.

3. Verbindungen nach Anspruch 1, wobei die Substituenten die nachstehende Bedeutung haben:
R₀, R₁ bzw. R₂ werden unabhängig voneinander ausgewählt aus:
- Chlor, Brom, Iod, Azido, Hydrazino
- L_{A}-A-L_{B}-B, worin bedeuten:
L_{A}:
- Einfachbindung, - NR^{#}, O
A:
- Wasserstoff,
- C₁₋₆Alkyl (ggf. mit R^{§a} substituiert),
- C₂₋₆Alkenyl (ggf, mit R^{§a} substituiert),
- C₂₋₆Alkinyl (ggf, mit R^{§a} substituiert),
- Phenyl, ggf. ein-, zwei- oder dreifach mit unabhängig voneinander ausgewählten Resten R^{§b} substituiert
- ein mono- oder bicyclischer gesättigter oder ein- oder mehrfach ungesättigter. Heterocyclus mit 4-10 Ringatomen darunter 1 - 5 Heteroatome(vorzugsweise N, O, S), der ggf. mit einem Rest R^{§b} und/ oder ein oder mehreren Sauerstoffatomen substituiert sein kann.
und für den Fall, dass A nicht weiter substituierbar ist, sind L_{B} und B gegenstandslos,
L_{A}-A sowie L_{A}-A-L_{B} können jeweils gemeinsam auch eine Einfachbindung bedeuten
L_{B}:
- Einfachbindung,
- NR^{#}, O, S, S(O)₂, C=O
- folgende funktionelle Gruppen: 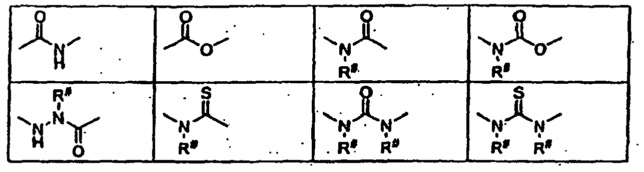
B:
- Wasserstoff,
- Alkyl, ggf. mit R^{§a} substituiert
- C₂₋₆Alkenyl (ggf. mit R^{§a} substituiert),
- C₂₋₆Alkinyl (ggf. mit R^{§a} substituiert),
- Aryl, insb. Phenyl, ggf. mit R^{§b} substituiert
ein mono- oder bicyclischer gesättigter oder ein- oder mehrfach ungesättigter Heterocyclus mit 4 - 14 Ringatomen, darunter 1 - 5 Heteroatome (vorzugsweise N, O, S), der
ggf. ein- zwei- oder dreifach mit unabhängig voneinander ausgewählten Resten R^{§b} und/ oder ein oder mehreren Sauerstoffatomen substituiert sein-kann,
R^{#} - Wasserstoff, Alkyl (ggf. mit R^{§} substituiert).
R₃
- Wasserstoff,
- C₁₋₄Alkyl, geradkettig oder verzweigt sowie ggf. ein-, zwei- oder dreifach mit Halogen (F, Cl) substituiert,
- C₁₋₆ Alkoxy, geradkettig oder verzweigt oder cyclisch sowie ggf. mit -OH, - OCH₃, -NH₂, -N(CH₃)₂, -COOH oder ein-, zwei- oder dreifach mit Halogen (F, Cl) substituiert,
- ein monocyclischer gesättigter oder ein- oder mehrfach ungesättigter Heterocyclus mit 4 - 8 Ringatomen darunter 1 - 3 Heteroatomen, vorzugsweise N, O und S
R₄
- NR₇R₈, worin dieser Substituent insgesamt bedeutet:
- Morpholino, Thiomorpholino, Thiomorpholino-S,S,-dioxid, Pyrrolidino, Piperidino, piperazin-1yl, 1-Homopiperazinyl, 4-C₁₋₆-Alkyl-Piperazin-1-yl, 4-Aryl-1-Piperazin-1-yl, 4-Bn-Piperazin-1-yl (ggf. mit R^{§a} am heterocycloaliphatischen Ring substituiert),
- weitere Amino-Reste sekundärer, mono- oder polycyclischer Amine mit insgesamt 4-14 Ringatomen, einschließlich der am C-Skelett mit R^{§} substituierten Vertreter
Rₛ
- ein gesättigter oder ein- oder mehrfach ungesättigter heterocyclischer Rest mit insgesamt 4-8 Ring-Atomen, davon 1-3 Heteroatomen (vorzugsweise N, O und S), ggf. mit R^{§b} substituiert,
- C₁₋₆ Alkoxy, C₁₋₆ Alkylthio, jeweils geradkettig, verzweigt oder cyclisch sowie ggf. mit R^{§a} substituiert
- ein Ober ein exocyclisches Atom aus der Gruppe O, N, S gebundener gesättigter oder ein- oder mehrfach ungesättigter heterocyclischer Rest mit insgesamt 4-10 Ring-Atomen, davon 1-5 Heteroatome, (vorzugsweise N, O und S), ggf. mit R^{§b} substituiert,
- ein über ein am Tricyclus gebundenes Atom aus der Gruppe O, N oder S und eine nachfolgend angeordnete C₁₋₆ Alkylen-Gruppe gebundener gesättigter oder ein- oder mehrfach ungesättigter heterocyclischer Rest mit insgesamt 4-10 Ring-Atomen, davon 1-5 Heteroatome (vorzugsweise N, O und S), ggf. mit R^{§b} substituiert,
- Hydroxy, Halogen (Cl, Br, I)
R₆
- Wasserstoff,
- C₁₋₆Alkyl, geradkettig oder verzweigt sowie ggf. ein-, zwei- oder dreifach mit unabhängig voneinander ausgewählten Resten R^{§a} substituiert
- C₂₋₆Alkenyl
- Carbonyl oder Sulfonyl, jeweils substituiert mit C₁₋₆Alkyl und ggf. mit R^{§a} substituiert,
R^{§a}
- OH, -O-C₁₋₈Alkyl, -S-C₁₋₄Alkyl, -SO₂-C₁₋₄Alkyl, -COOH, -C-OOC₁₋₈Alkyl
- CONH₂, -CONHC₁₋₆Alkyl, -CON(C₁₋₆Alkyl)₂, -NH₂, -NHC₁₋₆Alkyl,
- N(C₁₋₆Alkyl)₂,
- Halogen (-F, -Cl, -Br, -I),
- CN, -SCN
R^{§b}
- OH, -O-C₁₋₆Alkyl
- SO₂-C₁₋₄Alkyl, -SO₃H, -OSO₂C₁₋₈Alkyl;
- COOH, -COOC₁₋₆Alkyl,
- CONH₂, -CONHC₁₋₆Alkyl, -CON(C₁₋₆Alkyl)₂,
- NH₂, -NHC₁₋₆Alkyl, -N(C₁₋₆Alkyl)₂,
- C₁₋₆Alkyl, -C₂₋₆ Alkenyl, -C₂₋₆Alkinyl, jeweils geradkettig, verzweigt oder cyclisch sowie ggf. ein-, zwei- oder dreifach unabhängig voneinander mit Halogen substituiert.
- Halogen (-F, -Cl, -Br, -I),
- Sulfamoyl, Alkylsulfamoyl, Dialkylsulfamoyl mit Alkyl C₁₋₅ ggf. substituiert mit Methoxy,
- CN, -NO₂, und -SCN
sowie pharmazeutisch verträgliche Salze, Solvate, Tautomere und Prodrugs dieser Verbindungen,
wobei Verbindungen mit:
- R₀=R₁=R₂=R₃=H
vom Schutz ausgenommen sind.

4. Verbindungen nach Anspruch 1 ausgewählt aus:
7-Brom-4-ethoxy-9-fluor-2-(piperazin-1-yl)-5H-pyrimido[5,4-*b*]indol
7-Brom-4-ethoxy-2-(piperazin-1-yl)-5*H*-pyrimido[5,4-*b*]indol-9-ol
4-Ethoxy-2-(piperazin-1-yl)-7-(pyridin-4-yl)-5*H*-pyrimido[5,4-b*]*indol-9-ol
4-Ethoxy-7-(3,4-dimethoxyphenyl)-2-(piperazin-1-yl)-5*H*-pyrimido[5,4-b]indol-9-ol
7-(3,4-Dimethoxyphenyl)-2-(piperazin-1-yl)-5*H*-pyrimido[5,4-b]indol-4,9-diol
4-Ethoxy-8-(3,4,5-trimethoxyphenyl)-2-(piperazin-1-yl)-5*H*-pyrimido[5,4-b]indol
1-(7-(3,4-Dimethoxyphenyl)-2-(piperazin-1-yl)-5*H*-pyrimido[5,4-*b*]indol-4-yl)piperidin-4-ol
7-(3,4-Dimethoxyphenyl)-2-(piperazin-1-yl)-5*H*-pyrimido[5,4-b]indol-4-ol
4-((Pyridin-2-yl)methoxy))-7-(3,4-dimethoxyphenyl)-2-(piperazin-1-yl)-5*H-*pyrimido[5,4-b]indol
4-((Pyridin-4-yl)methoxy))-7-(3,4-dimethoxyphenyl)-2-(piperazin-1-yl)-5*H-*pyrimido[5,4-b]indol
4-((Pyridin-3-yl]methoxy))-7-(3,4-dimethoxyphenyl)-2-(piperazin-1-yl)-5*H-*Pyrimido[5,4-b]indol
4-Methylthio-2-(piperazin-1-yl)-7-(pyridin-4-yl)-5*H*-pyrimido(5,4-b]indol
2-(7-(3,4-Dimethoxyphenyl)-2-(piperazin-1-yl)-5*H*-pyrimido[5,4-b]indol-4-ylamino)ethanol
7-Brom-2-(piperazin-1-yl)-5*H*-pyrimido[5,4-b]indol-4-ol
4-(2-Morpholinoethoxy))-7-(3,4-dimethoxyphenyl)-2-(piperazin-1-yl)-5*H*-pyrimido[5,4-b]indol
7-(3,4-Dimethoxyphenyl)-4-morpholino-2-(piperazin-1-yl)-5*H*-pyrimido[5,4-b]indol
7-(3,4-Dimethoxyphenyl)-2-(piperazin-1-yl)-4-thiomorpholino-5*H*-pyrimido[5,4-b]indol
4-Morpholino-2-(piperazin-1-yl)-7-(pyrindin-4-yl)-5-pyrimido[5,4-b]indol
7-(3,4-Dimethoxyphenyl)-N-(2-morpholinoethyl)-2-(piperazin-1-yl)-5*H*-pyrimido[5,4-b]indol-4-amin
7-(3,4-Dimethoxyphenyl)- 2-(piperazin-1-yl)-4-piperidino-5*H*-pyrimido[5,4-b]indol
4-Cyclopropylmethoxy-7-(3,4-dimethoxyphenyl)-2-(piperazin-1-yl)-5*H*-pyrimido[5,4-b]indol
4-(1*H*-imidazol-1-yl)-7-(3,4-dimethoxyphenyl)-2-(piperazin-1-yl)-5*H*-pyrimido[5,4-b]indol
7-(3,4-Dimethoxyphenyl)-2-(piperazin-1-yl)-4-(piperidin-4-yloxy)-5*H*-pyrimido[5,4-b]indol
4-Cyclopropylmethoxy-2-(piperazin-1-yl)-7-(pyridine-4-yl)-5*H*-pyrimido[5,4-b]indol
4-Ethoxy-2-morpholino-7-(pyridine-4-yl)-5*H*-pyrimido[5,4-b]indol
4-(4-Ethoxy-2-(piperazin-1-yl)-5*H*-pyrimido[5,4-b]indol-7-yl)benzoesäureethylester
4-(4-Ethoxy-2-)piperazin-1-yl)-5*H*-pyrimido[5,4-b]indol-7-yl)benzoesäure-hydrochlorid
4-(4-Ethoxy-2-(piperazin-1-yl)-5*H*-pyrimido[5,4-b]indol-7-yl)phenol-hydrochlorid
3-(4-Ethoxy-2-(piperazin-1-yl)-5*H*-pyrimido[5,4-b]indol-7-yl)benzoesäuremethylester
3-(4-Ethoxy-2-(piperazin-1-yl)-5*H*-pyrimido[5,4-b]indol-7-yl)benzoesäure-hyrdrochlorid
4-Ethoxy-7-(furan-2-yl)-2-(piperazin-1-yl)-5*H*-pyrimido[5,4-b]indol
tert-Butyl-2-(4-ethoxy-2-(piperazin-1-yl)-5*H*-pyrimido[5,4-b]indol-7-yl)-1*H*-pyrrol-1-carboxylat
7-(Benzo[d][1,3]dioxol-5-yl)-4-ethoxy-2-(piperazin-1-yl)-5*H*-pyrimido[5,4]indol
7-(3,4-Dimethoxyphenyl)-2-(piperazin-1-yl)-N-(thiazol-2-yl)-5*H*-pyrimido[5,4-b]indol-amin
7-(3,4-Dimethoxyphenyl)-2-(piperazin-1-yl)-4-(1*H*-pyrrol-1-yl)-5*H*-pyrimido[5,4-b]indol
7-(3,4-Dimethoxyphenyl)-2-(piperazin-1-yl)-4-(1*H*-pyrazol-1-yl)-5*H*-pyrimido[5,4-b]indol
7-(3,4-Dimethoxyphenyl)-2-(piperazin-1-yl)-4-(1*H*-1,2,3-triazol-1-yl)-5*H*-pyrimido[5,4-b]indol
7-(3,4-Dimethoxyphenyl)-2-(piperazin-1-yi)-4-(4*H*-1,2,4-triazol-4-yi)-5*H*-pyrimido[5,4-b]indol
7-(3,4-Dimethoxyphenyl)-2-(piperazin-1-yl)-4-(pyrrolidin-1-yl)-5*H*-pyrimido[5,4-b]indol
(4-(4-Ethoxy-2-(piperazin-1-yl)-5*H*-pyrimido[5,4-b]indol-7-yl)piperazin-1-yl)(phenyl)methanon
4-Ethoxy-2-(piperazin-1-yl)-7-(4-(pyrimidin-2-yl)piperazin-1-yl)-5*H*-pyrimido[5,4-b]indol
4-Ethoxy-2-(piperidin-4-yl)-7-(pyridin-4-yl)-5*H*-pyrimido[5,4-b]indol
4-Ethoxy-7-(3,4-dimethoxyphenyl)-2-(piperidin-4-yl)-5*H*-pyrimido[5,4-b]indol
4-Ethoxy-7-(3,4-dimethoxyphenyl)-2-(piperidin-3-yl)-5*H*-pyrimido[5,4-b]indol
2-(4-Ethoxy-9-methoxy-2-(piperazin-1-yl)-5*H*-pyrimido[5,4-b]indol-7-yl)benzenamin
2-{2-(Piperazin1-yl)-1-(pyridin-4-yl)-5*H*-pyrimido[5,4-b]indol-4-yloxy}ethanol
4-Ethoxy-2-(4-methylpiperazin-1-yl)-7-(pyridin-4-yl)-5-*H*-pyrimido[5,4-b]indol
4-Ethoxy-2-(piperazin-1-yl)-6-(pyridin-4-yl)-5*H*-pyrimido[5,4-b]indol
4-Ethoxy-2-(piperazin-1-yl)-7-(2-aminopyiridin-5-yl)-5*H*-pyrimido[5,4-b]jndol
4-Ethoxy-2-(piperazin-1-yl)-7-(3,4-dimethoxy-phenyl)-5*H*-pyrimido[5,4-b]indol
4-Ethoxy-2-(piperazin-1-yl)-7-(pyridin-3-yl)-5*H*-pyrimido[5,4-b]indol
4-Ethoxy-2-(piperazin-1-yl)-7-(pyridin-4-yl)-5*H*-pyrimido[5,4-b]indol
4-Ethoxy-2-(piperazin-1-yl)-8-(pyridin-4-yl)-5*H*-pyrimido[5,4-b]indol
4-Ethoxy-7-morpholino-2-(piperazin-1-yl)-5*H*-pyrimido[5,4-b]indol
4-Ethoxy-9-methoxy-2-(piperazin-1-yl)-7-(pyridin-4-yl)-5*H*-pyrimido[5,4-b]indol

5. Pharmazeutische Zusammensetzung enthaltend eine oder mehrere Verbindungen gemäß einem der Ansprüche 1-4 sowie übliche Hilfs- und Trägerstoffe.

6. Pharmazeutische Zusammensetzung nach Anspruch 5, aufweisend weiter eine oder mehrere der folgenden Wirksstoffe:
- Nukleosidanaloga (z.B. Fludarabin, Cladribin)
- Alkylantien (z.B. Chlorambucil, Cyclophosphamid)
- ß₂-Adrenoceptor Agonisten (z.B. Terbutalin, Salbutanol, Salmelanol, Fenoterol, Formoterol)
- Korticosteroide
- Leukotrien-Antagonisten (entweder Enzyminhibitoren [wie 5-Lipoxygenase-Inhibitoren oder Arachidonsäure-Enzyminhibitoren] oder Rezeptorantagonisten), z.B. Pramkulast, Montelukast, Zafirlukast, Zileuton
- Antihistaminika (bevorzugt solche mit Mastzellen-stabilisierenden Eigenschaften oder Leukotrien-antagonisierenden Aspekten, wie z.B. Loratadin, Astemizol, Mizolastin, Olopatadin
- Theophyllin
- PDE-Inhibitoren
- (monoklonale) Antikörper gegen TNF-alpha oder andere Wirkstoffe, die die Bildung bzw. Freisetzung von TNF-alpha oder die Aktivität von TNF-alpha hemmen (z.B. rekombinante lösliche Rezeptorkonstrukte)
- (monoklonale) Antikörper (z.B. Rituximab, TACl-Ig)

7. Eine oder mehrere Verbindungen gemäß einem der Ansprüche 1 bis 4 zur Verwendung bei der Behandlung von nicht-soliden malignen Tumoren des blutbildenden Systems.

8. Verbindung nach Anspruch 7 zur Verwendung bei der Behandlung von Leukämien und Lymphomen.

9. Verbindung nach Anspruch 8 zur Verwendung bei der Behandlung von chronisch lymphatischer Leukämie vom B-Zelltyp.

10. Verbindung nach Anspruch 9 zur Verwendung bei der Behandlung von chronisch lymphatischer Leukämie, die mit chromosomalen Deletionen vergesenschaftet ist.

11. Verbindung nach Anspruch 10, wobei die chromosomale Deletion eine 11q Deletion ist.

12. Verfahren zur Herstellung von Verbindungen nach einem der Ansprüche 1 bis 4, aufweisend die folgenden Verfahrensschritte: 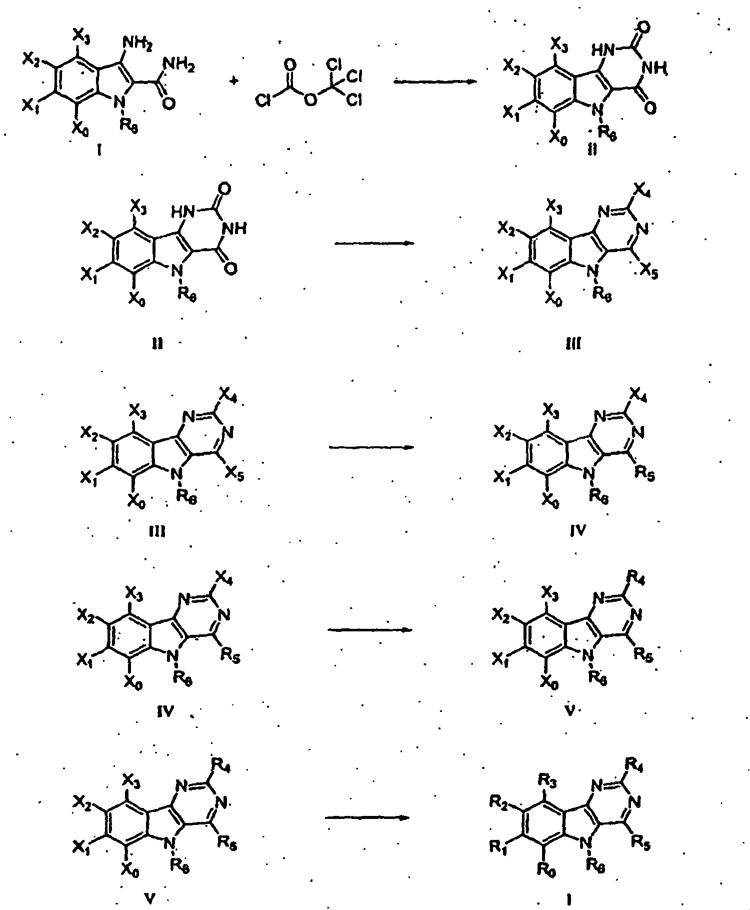 X₀-X₅ = unabhängig gleich oder ungleich F, Cl, Br, I, H wobei X₄, X₅ ungleich H
• Umsetzung der Verbindungen der allgemeinen Formel I mit Phosgenderivaten, vorzugsweise Diphosgen, in einem geeignetem Lösungsmittel, vorzugsweise Dioxan oder Toluol, zu den Verbindungen der allgemeinen Formen II
• Umsetzung der Verbindungen der allgemeinen Formel II mit einem Halogenierungsmittel, vorzugsweise Dichlorphenylphosphinoxid, Phosphor-trichlorid, Phosphorpentachlorid, Phosphoroxychlorid bzw. deren Mischungen, jeweils in der Hitze, zu den Verbindungen der allgemeinen Formel III
• Umsetzung der Verbindungen der allgemeinen Formel III mit O-, N-, S- oder C-Nucleophilen, vorzugsweise Alkoholate, Amine und Thiolate, in Alkanolen oder gegebenenfalls aprotischen, dipolaren Lösungsmitteln unter Erwärmen, in Ausnahmefällen auch bei Raumtemperatur, zu den tricyclischen Verbindungen der allgemeinen Formel IV
• Umsetzung der Verbindungen der allgemeinen Formel IV mit O-, N-, S- oder C-Nucleophilen, vorzugsweise Alkoholate, Amine und Thiolate, in einem geeigneten Lösungsmittel vorzugsweise Toluol, Mesitylen oder Dioxan unter Erwärmen, in Ausnahmefällen auch bei Raumtemperatur, zu den Verbindungen der allgemeinen Formel V
• Umsetzung der Verbindungen der allgemeinen Formel V mit O-, N-, S- oder C-Nucleophilen in einem geeigneten Lösungsmittel, vozugsweise Toluol, Dioxan oder THF, unter Erwärmen und unter Verwendung geeigneter Katalysatoren, in Ausnahmefällen unter Verwendung einer Mikrowelle, zu den Verbindungen der allgemeinen Formel 1
• Die Einführung der Reste R₀, R₁, R₂, R₄, R₅ und R₆ kann jeweils auf verschiedenen Synthesestufen, mittels geeigneter Reaktionen, Insbesondere metallkartalysierter C-C-Kreuzkupplungsreaktionen, und gegebenenfalls unter Verwendung geeigneter Schutzgruppen, erfolgen.

## Claims

1. Compounds of general formula 1 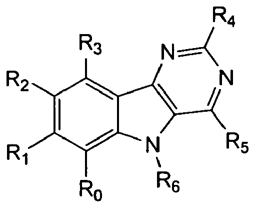 in which the substituents have the following meanings:
R₀, R₁ and R₂ are
- L_{A}-A-L_{B}-B in which:
L_{A} is:
- single bond,
- NR^{#}, O, S, S(O), S(O)₂, S(O)₂-O, O-S(O)₂,
- CHR^{§}, -CH₂-O-, -CH₂-CH₂-O-, -O-CH₂, -O-CH₂-CH₂-, C=O
A is:
- hydrogen
- C₁₋₆ alkyl (substituted, where appropriate, with R^{§}),
- C₂₋₆ alkenyl (substituted, where appropriate, with R^{§}),
- C₂₋₆ alkynyl (substituted, where appropriate, with R^{§}),
- chlorine, bromine, iodine,
- azido, hydrazino,
- phenyl, substituted, where appropriate, once, twice or thrice with residues R^{§} independently selected from one another,
- a monocyclic or bicyclic, saturated or monounsaturated or polyunsaturated heterocycle having 4 - 14 ring atoms, among them 1 - 5 heteroatoms (preferably N, O and S) which may carry one or more oxygen atoms on C, N and/or S and is substituted, where appropriate, with R^{§},
and, in case A cannot be substituted any further, L_{B} and B are irrelevant,
L_{A}-A and L_{A}-A-L_{B} may jointly also be a single bond in each case,
L_{B} is:
- single bond
- NR^{#}, O, S, S(O), S(O)₂, S(O)₂-O, O-S(O)₂,
- CHR^{§}, -CH₂-O-, -CH₂-CH₂-O, -O-CH₂, -O-CH₂-CH₂-, C=O,
- the following functional groups 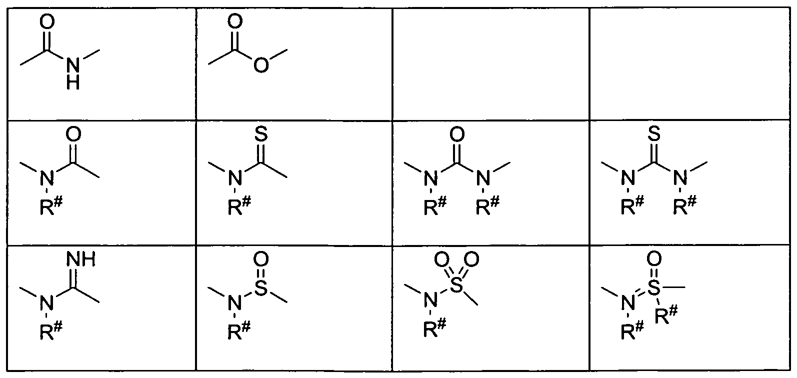 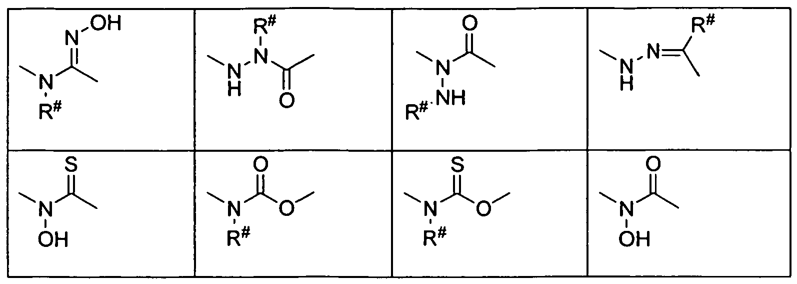
B is:
- hydrogen
- alkyl, substituted, where appropriate, with R^{§},
- C₂₋₆ alkenyl (substituted, where appropriate, with R^{§}),
- C₂₋₆ alkynyl (substituted, where appropriate, with R^{§}),
- arylalkyl- with C₆₋₁₂ aryl and C₁₋₅ alkyl (alkyl and/or a aryl substituted, where appropriate, with R^{§}).
- alkyl, monosubstituted or polysubstituted with monocyclic or bicyclic, saturated or monounsaturated or polyunsaturated heterocycles having 4 - 14 ring atoms among them 1 - 5 heteroatoms (preferably N, O, S), which may be substituted, where appropriate, once, twice or thrice with residues R^{§} independently selected from one another and/or one or more oxygen atoms,
- aryl, in particular phenyl substituted, where appropriate, with R^{§}
- a monocyclic or bicyclic, saturated or monounsaturated or polyunsaturated heterocycle having 4 - 14 ring atoms among them 1 - 5 heteroatoms (preferably N, O, S) which, where appropriate, may be substituted once, twice or thrice with residues R^{§} independently selected from one another and/or one or more oxygen atoms,
R^{#} - hydrogen, alkyl (substituted, where appropriate, with R^{§})
R₃ is
- hydrogen,
- C₁₋₆ alkyl, straight-chain, branched or C₃₋₆ alkyl also cyclic as well as substituted, where appropriate, once, twice or thrice with residues R^{§} independently selected from one another,
- R^{§}
- C₁₋₆ alkoxy, straight-chain, branched or C₃₋₆ alkoxy also cyclic as well as substituted, where appropriate, once, twice or thrice with residues R^{§} independently selected from one another,
- a monocyclic, saturated or monounsaturated or polyunsaturated heterocycle having 4 - 8 ring atoms among them 1 - 3 heteroatoms, preferably N, O and S,
R₄ is
- NR₇R₈, wherein this substituent is on the whole:
- morpholino, thiomorpholino, thiomorpholino-S,S-dioxide, pyrrolidino, piperidino, piperazin-1-yl, 1-homopiperazinyl, 4-C₁₋₆ alkyl piperazin-1-yl, 4-aryl-1-piperzin-1-yl, 4-Bn-piperazin-1-yl (substituted, where appropriate, with R^{§} at the heterocycloaliphatic ring),
- further amino residues of secondary, monocyclic or polycyclic amines having a total of 4 - 14 ring atoms, including the representatives substituted on the C skeleton with R^{§}
R₅ is
- monocyclic, bicyclic or tricyclic, saturated or monounsaturated or polyunsaturated heterocyclic residue having a total of 4 - 14 ring atoms, among them 1 - 5 heteroatoms, which are preferably N, O and S, substituted, where appropriate, with R^{§},
- C₁₋₆ alkyl, C₁₋₆ alkoxy, C₁₋₆ alkylthio, each straight-chain, branched or cyclic and substituted, where appropriate, with R^{§}
- a saturated or monounsaturated or polyunsaturated heterocyclic residue bound via an exocyclic atom selected from group O, N, S and having a total of 4 - 10 ring atoms among them 1 - 5 heteroatoms which are preferably N, O and S, substituted, where appropriate, with R^{§},
- a saturated or monounsaturated or polyunsaturated heterocyclic residue which is bound via an atom bound at the tricycle and selected from group O, N or S and a downstream C₁₋₆ alkylene group and which has a total of 4 - 10 ring atoms among them 1 - 5 heteroatoms (preferably N, O and S), substituted, where appropriate, with R^{§},
- hydroxy, halogen (Cl, Br, I)
R₆ is
- hydrogen
- C₁₋₆ alkyl, straight-chain, branched or C₃₋₆ alkyl also cyclic as well as substituted, where appropriate, once, twice or thrice with residues R^{§} selected independently from one another
- C₂₋₆ alkenyl (substituted, where appropriate, with R^{§})
- alkyl substituted once or several times with monocyclic or bicyclic, saturated or monounsaturated or polyunsaturated heterocycles having 4 - 14 ring atoms among them 1 -5 heteroatoms which are preferably N, O and S that may carry one or several oxygen atoms at C, N and/or S and substituted, where appropriate, once, twice or thrice with residues R^{§} independently selected from one another, as well as substituted, where appropriate, with R^{§},
- carbonyl or sulfonyl, each substituted with
- hydrogen, C₁₋₆ alkyl (substituted, where appropriate, with R^{§})
- C₂₋₆ alkenyl (substituted, where appropriate, with R^{§})
- C₂₋₆ alkynyl (substituted, where appropriate, with R^{§})
- aryl, in particular phenyl (substituted, where appropriate, with R^{§})
- a monocyclic or bicyclic, saturated or monounsaturated or polyunsaturated heterocycle having 4 - 14 ring atoms among them 1 - 5 heteroatoms which are preferably N, O and S, which may carry one or several oxygen atoms on C, N and/or S and may be substituted, where appropriate, once, twice or thrice with residues R^{§} independently selected from one another,
R^{§} is
- OH, -SH, -O-C₁₋₈ alkyl, -O-C₆₋₁₄ aryl, -S-C₁₋₄ alkyl, -S-C₆₋₁₄ aryl, -SO-C₁₋₄ alkyl, -SO-C₆₋₁₄ aryl, -SO₂-C₁₋₄ alkyl, -SO₂-C₆₋₁₄ aryl, -SO₃H,
- OSO₂C₁₋₈ alkyl, -OSO₂C₆₋₁₄ aryl, -COOH, -COOC₁₋₈ alkyl,
- (CO)C₁₋₈ alkyl,
- COOH, -CONH₂, -CONHC₁₋₆ alkyl, -CON(C₁₋₆ alkyl)₂,
- NH₂, -NHC₁₋₆ alkyl, -N(C₁₋₆ alkyl)₂, -NHC₆₋₁₄ aryl, -NH-hetaryl,
- N(C₆₋₁₄ aryl)₂, -N(C₁₋₆ alkyl)(C₆₋₁₄ aryl),
- C₁₋₆ alkyl, -C₂₋₁₂ alkenyl, -C₂₋₁₂ alkynyl, each straight-chain, branched or cyclic as well as substituted, where appropriate, once, twice or thrice with halogen independently of one another,
- halogen (-F, -Cl, -Br, -I)
- CH₂CH₂OH, -CH₂CH₂SH, -CH₂CH₂SCH₃,
- sulfamoyl, alkyl sulfamoyl, dialkyl sulfamoyl with alkyl C₁₋₅ substituted, where appropriate, with methoxy,
- amidino, hydroxyamidino
- sulfo, phosphono,
- -CN, -NO₂ and -SCN
as well as pharmaceutically compatible salts, solvates, tautomers and prodrugs of these compounds,
with compounds wherein
-R₀=R₁=R₂=R₃=H
being excluded from protection.

2. Compounds according to claim 1, wherein the substituents have the following meanings:
R₀, R₁ and R₂ are independently selected from:
- chlorine, bromine, iodine, azido, hydrazino,
- L_{A}-A-L_{B}-B in which:
L_{A} is: - single bond,
- NR^{#}, O, S, S(O)₂, C=O
A is: - hydrogen
- C₁₋₆ alkyl (substituted, where appropriate, with R^{§a}),
- C₂₋₆ alkenyl (substituted, where appropriate, with R^{§a}),
- C₂₋₆ alkynyl (substituted, where appropriate, with R^{§a}),
- phenyl, substituted, where appropriate, once, twice or thrice with residues R^{§b} independently selected from one another,
- a monocyclic or bicyclic, saturated or monounsaturated or polyunsaturated heterocycle having 4 - 10 ring atoms among them 1 - 5 heteroatoms (preferably N, O and S) substituted, where appropriate, by one or more oxygen atoms and/or a residue R^{§b},
and, in case A cannot be substituted any further, L_{B} and B are irrelevant,
L_{A}-A and L_{A}-A-L_{B} may jointly also be a single bond in each case,
L_{B} is: - single bond
- NR^{#}, O, S, S(O)₂, C=O,
- the following functional groups: 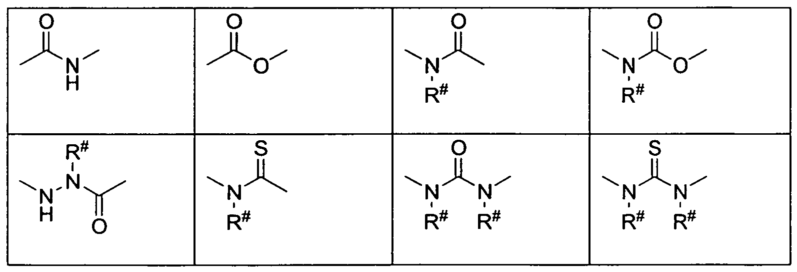
B is: - hydrogen
- alkyl, substituted, where appropriate, with R^{§a},
- C₂₋₆ alkenyl (substituted, where appropriate, with R^{§a}),
- C₂₋₆ alkynyl (substituted, where appropriate, with R^{§a}),
- aryl, in particular phenyl, substituted, where appropriate, with R^{§b}
- a monocyclic or bicyclic, saturated or monounsaturated or polyunsaturated heterocycle having 4 - 10 ring atoms among them 1 - 5 heteroatoms (preferably N, O, S) which, where appropriate, may be substituted once, twice or thrice with residues R^{§b} independently selected from one another and/or one or more oxygen atoms,
R^{#} - hydrogen, alkyl (substituted, where appropriate, with R^{§})
R₃ is
- hydrogen,
- C₁₋₆ alkyl, straight-chain, branched and substituted, where appropriate, once, twice or thrice with residues R^{§a} independently selected from one another,
- R^{§b}
- C₁₋₆ alkoxy, straight-chain, branched or C₃₋₆ alkoxy also cyclic as well as substituted, where appropriate, once, twice or thrice with residues R^{§} independently selected from one another,
- a monocyclic, saturated or monounsaturated or polyunsaturated heterocycle having 4 - 8 ring atoms among them 1 - 3 heteroatoms, preferably N, O and S,
R₄ is
- NR₇R₈, wherein this substituent is on the whole:
- morpholino, thiomorpholino, thiomorpholino-S,S-dioxide, pyrrolidino, piperidino, piperazin-1-yl, 1-homopiperazinyl, 4-C₁₋₆ alkyl piperazin-1-yl, 4-aryl-1-piperazin-1-yl, 4-Bn-piperazin-1-yl (substituted, where appropriate, with R^{§a} at the heterocycloaliphatic ring),
- further amino residues of secondary, monocyclic or polycyclic amines having a total of 4 - 14 ring atoms, including the representatives substituted on the C skeleton with R^{§}
R₅ is
- a saturated or monounsaturated or polyunsaturated heterocyclic residue having a total of 4 - 8 ring atoms, among them 1 - 3 heteroatoms, which are preferably N, O and S, substituted, where appropriate, with R^{§b},
- C₁₋₆ alkoxy, C₁₋₆ alkylthio, each straight-chain, branched or cyclic and substituted, where appropriate, with R^{§a},
- a saturated or monounsaturated or polyunsaturated heterocyclic residue bound via an exocyclic atom selected from group O, N, S and having a total of 4 - 10 ring atoms among them 1 - 5 heteroatoms (preferably N, O and S) substituted, where appropriate, with R^{§b},
- a saturated or monounsaturated or polyunsaturated heterocyclic residue which is bound via an atom bound at the tricycle and selected from group O, N or S and a downstream C₁₋₆ alkylene group and which has a total of 4 - 10 ring atoms among them 1 - 5 heteroatoms (preferably N, O and S), substituted, where appropriate, with R^{§b},
- hydroxy, halogen (Cl, Br, I)
R₆ is
- hydrogen
- C₁₋₆ alkyl, straight-chain or branched as well as substituted, where appropriate, once, twice or thrice with residues R^{§a} selected independently from one another
- C₂₋₆ alkenyl (substituted, where appropriate, with R^{§a})
- carbonyl or sulfonyl, each substituted with C₁₋₆ alkyl (substituted, where appropriate, with R^{§a})
R^{§a} is
- OH, -O-C₁₋₈ alkyl, -S-C₁₋₄ alkyl, -SO₂-C₁₋₄ alkyl, -COOH, -COOC₁₋₈ alkyl, -CONH₂, -CONHC₁₋₆ alkyl, -CON(C₁₋₆ alkyl)₂, -NH₂, -NHC₁₋₆ alkyl, -N(C₁₋₆ alkyl)₂,
- halogen (-F, -Cl, -Br, -I)
- -CN,-SCN
R^{§b} is
- OH, -O-C₁₋₈ alkyl,
- SO₂-C₁₋₄ alkyl, -SO₃H, -OSO₂C₁₋₈ alkyl,
- COOH, -COOC₁₋₈ alkyl,
- CONH₂, -CONHC₁₋₆ alkyl, -CON(C₁₋₆ alkyl)₂,
- NH₂, -NHC₁₋₆ alkyl, -N(C₁₋₆ alkyl)₂,
- C₁₋₆ alkyl, -C₂₋₆ alkenyl, -C₂₋₆ alkynyl, each straight-chain, branched or cyclic as well as substituted, where appropriate, once, twice or thrice with halogen independently of one another,
- halogen (-F, -Cl, -Br, -I)
- CH₂CH₂OH, -CH₂CH₂SCH₃,
- sulfamoyl, alkyl sulfamoyl, dialkyl sulfamoyl with alkyl C₁₋₅ substituted, where appropriate, with methoxy,
- amidino, hydroxyamidino
- sulfo, phosphono,
- -CN, -NO₂ and -SCN
as well as pharmaceutically compatible salts, solvates, tautomers and prodrugs of these compounds,
with compounds wherein
- R₀=R₁=R₂=R₃=H
being excluded from protection.

3. Compounds according to claim 1, wherein the substituents have the following meanings:
Ro, R₁ and R₂ are independently selected from:
- chlorine, bromine, iodine, azido, hydrazino,
- L_{A}-A-L_{B}-B in which:
L_{A} is: - single bond, - NR^{#}, O,
A is:
- hydrogen
- C₁₋₆ alkyl (substituted, where appropriate, with R^{§a}),
- C₂₋₆ alkenyl (substituted, where appropriate, with R^{§a}),
- C₂₋₆alkynyl (substituted, where appropriate, with R^{§a}),
- phenyl, substituted, where appropriate, once, twice or thrice with residues R^{§b} independently selected from one another,
- a monocyclic or bicyclic, saturated or monounsaturated or polyunsaturated heterocycle having 4 - 10 ring atoms among them 1 - 5 heteroatoms (preferably N, O, S) which may be substituted, where appropriate, with a residue R^{§b} and/or one or more oxygen atoms,
and, in case A cannot be substituted any further, L_{B} and B are irrelevant,
L_{A}-A and L_{A}-A-L_{B} may jointly also be a single bond in each case,
L_{B} is: - single bond
- NR^{#}, O, S, S(O)₂, C=O,
- the following functional groups: 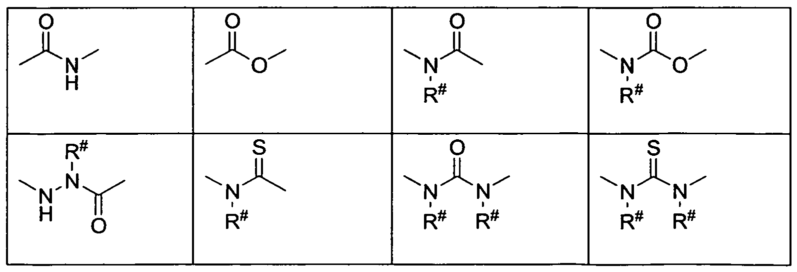
B is:
- hydrogen
- alkyl, substituted, where appropriate, with R^{§a},
- C₂₋₆ alkenyl (substituted, where appropriate, with R^{§a}),
- C₂₋₆ alkynyl (substituted, where appropriate, with R^{§a}),
- aryl, in particular phenyl, substituted, where appropriate, with R^{§b},
- a monocyclic or bicyclic, saturated or monounsaturated or polyunsaturated heterocycle having 4 - 14 ring atoms among them 1 - 5 heteroatoms (preferably N, O, S) which, where appropriate, may be substituted once, twice or thrice with residues R^{§b} independently selected from one another and/or one or more oxygen atoms,
R^{#} - hydrogen, alkyl (substituted, where appropriate, with R^{§})
R₃ is
- hydrogen,
- C₁₋₆ alkyl, straight-chain or branched as well as substituted, where appropriate, once, twice or thrice with halogen (F, Cl),
- C₁₋₆ alkoxy, straight-chain or branched or cyclic as well as substituted, where appropriate, with -OH, -OCH₃, -NH₂, -N(CH₃)₂, -COOH or once, twice or thrice with halogen (F, Cl),
- a monocyclic, saturated or monounsaturated or polyunsaturated heterocycle having 4 - 8 ring atoms among them 1 - 3 heteroatoms, preferably N, O and S,
R₄ is
- NR₇R₈, wherein this substituent is on the whole:
- morpholino, thiomorpholino, thiomorpholino-S,S-dioxide, pyrrolidino, piperidino, piperazin-1-yl, 1-homopiperazinyl, 4-C₁₋₆ alkyl piperazin-1-yl, 4-aryl-1-piperzin-1-yl, 4-Bn-piperazin-1-yl (substituted, where appropriate, with R^{§a} at the heterocycloaliphatic ring),
- further amino residues of secondary, monocyclic or polycyclic amines having a total of 4 - 14 ring atoms, including the representatives substituted on the C skeleton with R^{§}
R₅ is
- a saturated or monounsaturated or polyunsaturated heterocyclic residue having a total of 4 - 8 ring atoms among them 1 - 3 heteroatoms ( preferably N, O and S) substituted, where appropriate, with R^{§b},
- C₁₋₆ alkoxy, C₁₋₆ alkylthio, each straight-chain, branched or cyclic and substituted, where appropriate, with R^{§a}
- a saturated or monounsaturated or polyunsaturated heterocyclic residue bound via an exocyclic atom selected from group O, N, S and having a total of 4 - 10 ring atoms among them 1 - 5 heteroatoms (preferably N, O and S) substituted, where appropriate, with R^{§b},
- a saturated or monounsaturated or polyunsaturated heterocyclic residue which is bound via an atom bound at the tricycle and selected from group O, N or S and a downstream C₁₋₆alkylene group and which has a total of 4 - 10 ring atoms among them 1 - 5 heteroatoms (preferably N, O and S), substituted, where appropriate, with R^{§b},
- hydroxy, halogen (Cl, Br, I)
R₆ is
- hydrogen
- C₁₋₆ alkyl, straight-chain or branched as well as substituted, where appropriate, once, twice or thrice with residues R^{§a} selected independently from one another
- C₂₋₆ alkenyl
- carbonyl or sulfonyl, each substituted with C₁₋₆ alkyl and, where appropriate, with R^{§a},
R^{§a} is
- OH, -O-C₁₋₈ alkyl, -S-C₁₋₄ alkyl, -SO₂-C₁₋₄ alkyl, -COOH, -COOC₁₋₈ alkyl, -CONH₂, -CONHC₁₋₆ alkyl, -CON(C₁₋₆ alkyl)₂, -NH₂, -NHC₁₋₆ alkyl, -N(C₁₋₆ alkyl)₂,
- halogen (-F, -Cl, -Br, -I)
- -CN,-SCN
R^{§b} is
- OH, -O-C₁₋₈alkyl,
- SO₂-C₁₋₄ alkyl, -SO₃H, -OSO₂C₁₋₈ alkyl,
- COOH, -COOC₁₋₈ alkyl,
- CONH₂, -CONHC₁₋₆ alkyl, -CON(C₁₋₆ alkyl)₂,
- NH₂, ₋NHC₁₋₆ alkyl, -N(C₁₋₆ alkyl)₂,
- C₁₋₆ alkyl, -C₂₋₆ alkenyl, -C₂₋₆ alkynyl, each straight-chain, branched or cyclic as well as substituted, where appropriate, once, twice or thrice with halogen independently of one another,
- halogen (-F, -Cl, -Br, -I)
- sulfamoyl, alkyl sulfamoyl, dialkyl sulfamoyl with alkyl C₁₋₅ substituted, where appropriate, with methoxy,
- CN, -NO₂ and -SCN
as well as pharmaceutically compatible salts, solvates, tautomers and prodrugs of these compounds,
wherein compounds including
- R₀-R₁=R₂-R₃=H
are excluded from protection.

4. Compounds according to claim 1 selected from:
7-bromo-4-ethoxy-9-fluoro-2-(piperazin-1-yl)-5*H*-pyrimido[5,4-b]indole
7-bromo-4-ethoxy-2-(piperazin-1-yl)-5*H*-pyrimido[5,4-b]indol-9-ol
4-ethoxy-2-(piperazin-1-yl)-7-(pyridin-4-yl)-5*H*-pyrimido[5,4-b]indol-9-ol
4-ethoxy-7-(3,4-dimethoxyphenyl)-2-(piperazin-1-yl)-5*H*-pyrimido[5,4-b]indol-9-ol
7-(3,4-dimethoxyphenyl)-2-(piperazin-1-yl)-5*H*-pyrimido[5,4-b]indole-4,9-diol
4-ethoxy-8-(3,4,5-trimethoxyphenyl)-2-(piperazin-1-yl)-5*H*-pyrimido[5,4-b]indole
1-(7-(3,4-dimethoxyphenyl)-2-(piperazin-1-yl)-5*H*-pyrimido[5,4-b]indol-4-yl)piperidin-4-ol
7-(3,4-dimethoxyphenyl)-2-(piperazin-1-yl)-5*H*-pyrimido[5,4-b]indol-4-ol
4-((pyridin-2-yl)methoxy)-7-(3,4-dimethoxyphenyl)-2-(piperazin-1-yl)-5*H-*pyrimido[5,4-b]indole
4-((pyridin-4-yl)methoxy)-7-(3,4-dimethoxyphenyl)-2-(piperazin-1-yl)-5*H-*pyrimido[5,4-b]indole
4-((pyridin-3-yl)methoxy)-7-(3,4-dimethoxyphenyl)-2-(piperazin-1-yl)-5*H-*pyrimido[5,4-b]indole
4-methylthio-2-(piperazin-1-yl)-7-(pyridin-4-yl)-5*H*-pyrimido[5,4-b]indole
2-(7-(3,4-dimethoxyphenyl)-2-(piperazin-1-yl)-5*H*-pyrimido[5,4-b]indol-4-ylamino)ethanol
7-bromo-2-(piperazin-1-yl)-5*H*-pyrimido[5,4-b]indol-4-ol
4-(2-morpholinoethoxy)-7-(3,4-dimethoxyphenyl)-2-(piperazin-1-yl)-5*H-*pyrimido[5,4-b]indole
7-(3,4-dimethoxyphenyl)-4-morpholino-2-(piperazin-1-yl)-5*H*-pyrimido[5,4-b]indole
7-(3,4-dimethoxyphenyl)- 2-(piperazin-1-yl)-4-thiomorpholino-5*H*-pyrimido[5,4-b]indole
4-morpholino-2-(piperazin-1-yl)-7-(pyrindin-4-yl)-5*H*-pyrimido[5,4-b]indole
7-(3,4-dimethoxyphenyl)-*N*-(2-morpholinoethyl)-2-(piperazin-1-yl)-5*H-*pyrimido[5,4-b]indole-4-amine
7-(3,4-dimethoxyphenyl)-2-(piperazin-1-yl)-4-piperidino-5*H*-pyrimido[5,4-b]indole
4-cyclopropylmethoxy-7-(3,4-dimethoxyphenyl)-2-(piperazin-1-yl)-5*H-*pyrimido[5,4-b]indole
4-(1*H*-imidazol-1-yl)-7-(3,4-dimethoxyphenyl)-2-(piperazin-1-yl)-5*H-*pyrimido[5,4-b]indole
7-(3,4-dimethoxyphenyl)-2-(piperazin-1-yl)-4-(piperidin-4-yloxy)-5*H-*pyrimido[5,4-b]indole
4-cyclopropylmethoxy-2-(piperazin-1-yl)-7-(pyridin-4-yl)-5*H*-pyrimido[5,4-b]indole
4-ethoxy-2-morpholino-7-(pyridin-4-yl)-5*H*-pyrimido[5,4-b]indole
4-(4-ethoxy-2-(piperazin-1-yl)-5*H*-pyrimido[5,4-b]indol-7-yl)benzoic acid ethyl ester
4-(4-ethoxy-2-(piperazin-1-yl)-5*H*-pyrimido[5,4-b]indol-7-yl)benzoic acid hydrochloride
4-(4-ethoxy-2-(piperazin-1-yl)-5*H*-pyrimido[5,4-b]indol-7-yl)phenol hydrochloride
3-(4-ethoxy-2-(piperazin-1-yl)-5*H*-pyrimido[5,4-b]indol-7-yl)benzoic acid methyl ester
3-(4-ethoxy-2-(piperazin-1-yl)-5*H*-pyrimido[5,4-b]indol-7-yl)benzoic acid hydrochloride
4-ethoxy-7-(furan-2-yl)-2-(piperazin-1-yl)-5*H*-pyrimido[5,4-b]indole
tert-butyl-2-(4-ethoxy-2-(piperazin-1-yl)-5*H*-pyrimido[5,4-b]indol-7-yl)-1*H-*pyrrole-1-carboxylate
7-(benzo[*d*][1,3]dioxol-5-yl)-4-ethoxy-2-(piperazin-1-yl)-5*H*-pyrimido[5,4-b]indole
7-(3,4-dimethoxyphenyl)-2-(piperazin-1-yl)-N-(thiazol-2-yl)-5*H*-pyrimido[5,4-b]indole-4-amine
7-(3,4-dimethoxyphenyl)-2-(piperazin-1-yl)-4-(1*H*-pyrrol-1-yl)-5*H*-pyrimido[5,4-b]indole
7-(3,4-dimethoxyphenyl)-2-(piperazin-1-yl)-4-(1*H*-pyrazol-1-yl)-5*H-*pyrimido[5,4-b]indole
7-(3,4-dimethoxyphenyl)-2-(piperazin-1-yl)-4-(1*H*-1,2,3-triazol-1-yl)-5*H-*pyrimido[5,4-b]indole
7-(3,4-dimethoxyphenyl)-2-(piperazin-1-yl)-4-(4*H*-1,2,4-triazol-4-yl)-5*H-*pyrimido[5,4-b]indole
7-(3,4-dimethoxyphenyl)-2-(piperazin-1-yl)-4-(pyrrolidin-1-yl)-5*H*-pyrimido[5,4-b]indole
(4-(4-ethoxy-2-(piperazin-1-yl)-5*H*-pyrimido[5,4-b]indol-7-yl)piperazin-1-yl)(phenyl)methanone
4-ethoxy-2-(piperazin-1-yl)-7-(4-(pyrimidin-2-yl)piperazin-1-yl)-5*H-*pyrimido[5,4-b]indole
4-ethoxy-2-(piperidin-4-yl)-7-(pyridin-4-yl)-5*H*-pyrimido[5,4-b]indole
4-ethoxy-7-(3,4-dimethoxyphenyl)-2-(piperidin-4-yl)-5*H*-pyrimido[5,4-b]indole
2-(4-ethoxy-9-methoxy-2-(piperazin-1-yl)-5*H*-pyrimido[5,4-b] indol-7-yl)benzeneamine
2-(2-(piperazin-1-yl)-7-(pyridin-4-yl)-5*H*-pyrimido[5,4-b]indol-4-yloxy}ethanol
4-ethoxy-2-(4-methylpiperazin-1-yl)-7-(pyridin-4-yl)-5*H*-pyrimido[5,4-b]indole
4-ethoxy-2-(piperazin-1-yl)-6-(pyridin-4-yl)-5*H*-pyrimido[5,4-b]indole
4-ethoxy-2-(piperazin-1-yl)-7-(2-aminopyridin-5-yl)-5*H*-pyrimido[5,4-b]indole
4-ethoxy-2-(piperazin-1-yl)-7-(3,4-dimethoxy-phenyl)-5*H*-pyrimido[5,4-b]indole
4-ethoxy-2-(piperazin-1-yl)-7-(pyridin-3-yl)-5*H*-pyrimido[5,4-b]indole
4-ethoxy-2-(piperazin-1-yl)-7-(pyridin-4-yl)-5*H*-pyrimido[5,4-b]indole
4-ethoxy-2-(piperazin-1-yl)-8-(pyridin-4-yl)-5*H*-pyrimido[5,4-b]indole
4-ethoxy-7-morpholino-2-(piperazin-1-yl)-5*H*-pyrimido[5,4-b]indole
4-ethoxy-9-methoxy-2-(piperazin-1-yl)-7-(pyridin-4-yl)-5*H*-pyrimido[5,4-b]indole.

5. Pharmaceutical composition containing one or more compounds according to any of claims 1 to 4 as well as conventional excipients and carriers.

6. Pharmaceutical composition according to claim 5, further comprising one or more of the following substances:
- nucleoside analogues (e.g. fludarabine, cladribine)
- alkylating agents (e.g. chlorambucil, cyclophosphamide)
- ß₂ adrenoceptor agonists (e.g. terbutaline, salbutanol, salmetanol, fenoterole, formoterole)
- corticosteroids
- leukotriene antagonists (either enzyme inhibitors [such as 5-lipoxygenase inhibitors or arachidonic acid enzyme inhibitors] or receptor antagonists), e.g. pramkulaste, montelukaste, zafirlukast, zileuton
- antihistamines (preferably those having mast cell-stabilizing properties or leukotriene-antagonizing aspects, such as loratadine, astemizole, mizolastine, olopatadine
- theophylline
- PDE inhibitors
- (monoclonal) antibodies against TNF-alpha or other active substances which inhibit the formation or release of TNF-alpha or the activity of TNF-alpha (e.g. recombinant soluble receptor constructs)
- (monoclonal) antibodies (e.g. rituximab, TACl-Ig).

7. One or more of the compounds according to any of claims 1 to 4 for use in the treatment of non-solid malignant tumors of the hematopoietic system.

8. Compound according to claim 7 for use in the treatment of leukemias and lymphomas.

9. Compound according to claim 8 for use in the treatment of chronic lymphatic B cell type leukemia.

10. Compound according to claim 9 for use in the treatment of chronic lymphatic leukemia which is associated with chromosomal deletions.

11. Compound according to claim 10, wherein the chromosomal deletion is an 11q deletion.

12. Process for the production of compounds according to any of claims 1 to 4, comprising the following processing steps: 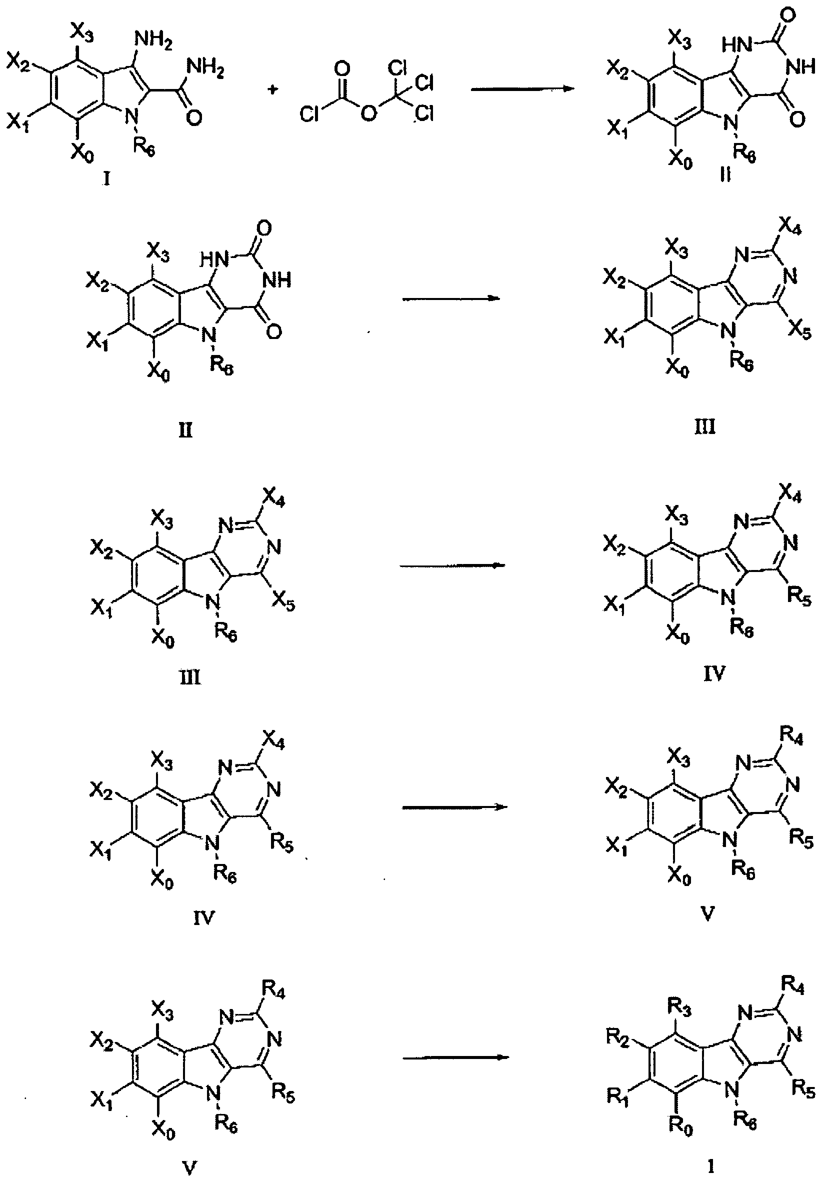 X₀-X₅ = independently like or unlike F, Cl, Br, I, H, wherein X₄, X₅ unlike H
• Reaction of the compounds of general formula I with phosgene derivatives, preferably diphosgene, in a suitable solvent, preferably dioxan or toluene, to give the compounds of general formula II,
• Reaction of the compounds of general formula II with a halogenating agent, preferably dichlorophenyl phosphine oxide, phosphorus trichloride, phosphorus pentachloride, phosphorus oxychloride or the mixtures thereof, each in the heat, to give the compounds of general formula III,
• Reaction of the compounds of general formula III with O, N, S or C nucleophiles, preferably alcoholates, amines and thiolates, in alkanols or, where appropriate, aprotic, dipolar solvents while heating, in exceptional cases also at room temperature, to give the tricyclic compounds of general formula IV,
• Reaction of the compounds of general formula IV with O, N, S or C nucleophiles, preferably alcoholates, amines and thiolates, in a suitable solvent, preferably toluene, mesitylene or dioxan while heating, in exceptional cases also at room temperature, to give the compounds of general formula V,
• Reaction of the compounds of general formula V with O, N, S or C nucleophiles in a suitable solvent, preferably toluene, dioxan or THF, while heating and using suitable catalysts, in exceptional cases with the use of a microwave oven, to give the compounds of general formula 1,
• Each of the residues R₀, R₁ R₂, R₄, R₅ und R₆ can be introduced in different synthesis stages by means of suitable reactions, in particular metal catalyzed C-C cross-coupling reactions, and, where appropriate, using suitable protecting groups.

## Revendications

1. Liaisons de la formule générale 1 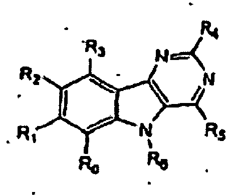 dans lesquelles les substituants ont la signification suivante:
R₀, R₁ ou R₂
- L_{A}-A-L_{B}-B, où signifient:
L_{A}: liaison simple,
-NR^{§}, O, S, S(O), S(O)₂, S(O)₂-O, O-S(O)₂, -CHR^{§}-, -CH₂- O-, -CH₂-CH₂-O-, -O-CH₂,-O-CH₂ -CH₂ -, C=O,
A:
- hydrogène,
- C₁₋₆alcoyle (éventuellement substitué par R^{§}),
- C₂₋₆alcényle (éventuellement substitué par R^{§}),
- C₂₋₆ alkinyle (éventuellement substitué par R^{§}),
- chlore, brome, iode,
- acide, hydrazino,
- phényle, éventuellement substitué une, deux ou trois fois par des restes R^{§} sélectionnés indépendamment l'un de l'autre,
- un hétérocycle saturé mono- ou bicycle ou non saturé une ou plusieurs fois avec de 4 à 14 atomes annulaires, parmi lesquels de 1 à 5 hétéroatomes (de préférence N, O et S) pouvant porter un ou plusieurs atomes d'oxygène sur C, N et/ou S, et éventuellement substitué par R^{§},
et au cas où A n'est plus substituable, L_{B} et B étant sans objet,
LA-A ainsi que L_{A}-A- L_{B} pouvant également signifier dans l'ensemble une liaison simple,
L_{B}:
- liaisons simple
- NR^{§}, O, S, S(O), S(O)₂, S(O)₂₋ O, O-S(O)₂, -CHR^{§}-, -CH₂ -O-, -CH₂- -CH₂ -O-, -O-CH₂ ,-O-CH₂,- CH₂-, C=O;
- les groupes fonctionnels suivants: 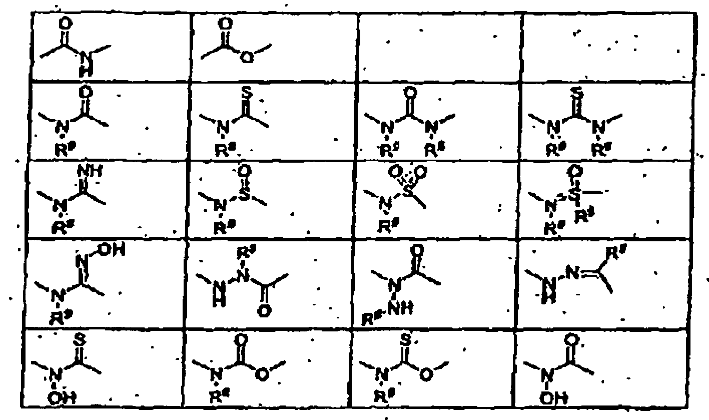
B:
- hydrogène
- alcoyle, éventuellement substitué par R^{§}
- C₂₋₆alcényle (éventuellement substitué par R^{§}),
- C₂₋₆alkinyle (éventuellement substitué par R^{§}),
- arylalkyl- avec C₆₋₁₂aryle et C₁₋₅alcoyle, (alcoyle et/ou aryle éventuellement substitué par R^{§}),
- alcoyle, une ou plusieurs fois substitué par des hétérocycles saturés mono- ou bicycliques ou non saturés une ou plusieurs fois avec de 4 à 14 atomes annulaires, parmi lesquels de 1 à 5 hétéroatomes (de préférence N, O, S) qui peuvent éventuellement être substitués une, deux ou trois fois par de restes R^{§} sélectionnés indépendamment l'un de l'autre et/ou un ou plusieurs atomes d'oxygène.
- aryle, en particulier phényle, éventuellement substitué par R^{§}
- un hétérocycle saturé mono- ou bicyclique ou non saturé une ou deux fois avec de 4 à 14 atomes annulaires, parmi lesquels de 1 à 5 hétéroatomes (de préférence N, O, S), pouvant éventuellement être substitués une, deux ou trois fois par des restes R^{§} sélectionnés indépendamment l'un de l'autre et/ou un ou plusieurs atomes d'oxygène.
R^{#} - hydrogène, alcoyle (éventuellement substitué par R^{§})
R₃
- hydrogène,
- C₁₋₆alcoyle, à chaine droite, ramifié ou C₃₋₆alcoyle également cyclique ainsi qu'une, deux ou trois fois substitué par des restes R^{§} sélectionnés indépendamment l'un de l'autre,
- R^{§}
- C₁₋₆alcoxy à chaîne droite, ramifié ou C₃₋₆alcoxy également cyclique ainsi qu'éventuellement substitué une, deux ou trois fois par des restes R^{§} sélectionnés indépendamment l'un de l'autre,
- un hétérocycle saturé monocyclique ou non saturé une ou plusieurs fois avec de 4 à 8 atomes annulaires, parmi lesquels de 1 à 3 hétéroatomes, de préférence N, O et S
R₄
- NR₇R₈, où ce substituant signifie dans l'ensemble:
- morpholino, thiomorpholino, thiomorpholino-S,S,-dioxyde, pyrrolidino, piperidino, piperazin-1-yl, 1-homopiperazinyl, 4-C₁₋₆-alkyl-piperazin-1-yl, 4-aryl-1-piperazin-1-yl, 4-Bn-piperazin-1-yl (éventuellement substitué par R^{§} à l'anneau hétérocyclo-aliphatique),
- d'autres restes amino d'amines secondaires, mono- ou polycycliques avec en tout de 4 à 14 atomes annulaires, y compris le représentant substitué par R^{§} au squelette C
R₅
- reste hétérocyclique saturé mono-, bi- ou tricyclique ou non saturé une ou deux fois avec en tout de 4 à 14 atomes annulaires, desquels de 1 à 5 hétéroatomes qui sont de préférence N, O et S, éventuellement substitués par R^{§},
- C₁₋₆alcoyle, C₁₋₆alcoxy, C₁₋₆alkilthio, chaque fois à chaîne droite, ramifié ou cyclique et éventuellement substitué par R^{§}.
- un reste hétérocyclique saturé ou non saturé une ou plusieurs fois lié par un atome exocyclique du groupe O, N ou S avec en tout de 4 à 10 atomes annulaires, parmi lesquels de 1 à 5 hétéroatomes qui sont de préférence N, O et S, éventuellement substitué par R^{§},
- un reste hétérocyclique saturé ou non saturé une ou plusieurs fois lié au tricycle par un atome du groupe O, N ou S et un groupe C₁₋₆alcoyle disposé après avec en tout de 4 à 10 atomes annulaires, parmi lesquels de 1 à 5 hétéroatomes (de préférence N, O et S), éventuellement substitués par R^{§},
- hydroxy, halogène (Cl, Br, I)
R₆
- hydrogène,
- C₁₋₆alcoyle, à chaine droite, ramifié ou C₃₋₆alcoyle également cyclique ainsi qu'éventuellement substitué une, deux ou trois fois par des restes R^{§} sélectionnés indépendamment l'un de l'autre
- C₂₋₆alcényle (éventuellement substitué par R^{§})
- alcoyle, substitué une ou plusieurs fois, avec des hétérocycles saturés mono- ou bicycliques ou non saturés une ou plusieurs fois avec de 4 à 14 atomes annulaires, parmi lesquels de 1 à 5 hétéroatomes qui sont de préférence N, O et S pouvant porter un ou plusieurs atomes d'oxygène sur C, N et/ou S et pouvant éventuellement être substitués une, deux ou trois fois par des restes R^{§} sélectionnés indépendamment l'un de l'autre, ainsi qu'éventuellement substitué par R^{§}
- carbonyle ou sulfonyle, chacun substitué par
- hydrogène, C₁₋₆alcoyle (éventuellement substitué par R^{§}),
- C₂₋₆alcényly (éventuellement substitué par R^{§}),
- C₂₋₆alkinyle (éventuellement substitué par R^{§}),
- aryle, en particulier phényle (éventuellement substitué par R^{§}),
- hétérocycle saturé mono- ou bicyclique ou non saturé une ou plusieurs fois avec de 4 à 14 atomes annulaires, parmi lesquels de 1 à 5 hétéroatomes qui sont de préférence N, O et S, pouvant porter un ou plusieurs atomes d'oxygène sur C, N et/ou S et pouvant éventuellement être substitués une, deux ou trois fois par des restes R^{§} sélectionnés indépendamment l'un de l'autre,
R^{§}
- OH, SH, -O-C₁₋₈alcoyle, -O-C₆₋₁₄aryle, -S-C₁₋₄alcoyle, -S-C₆₋₁₄aryle,
- SO-C₁₋₄ alcoyle, SO-C₆₋₁₄aryle, -SO₂₋C₁₋₄alcoyle, -SO₂-C₆₋₁₄aryle, -SO₃H, -OSO₂C₁₋₈alcoyle, -OSO₂C₆₋₁₄aryle. -COOH, -COOC₁₋₈alcoyle,
- (CO)C₁₋₈alcoyle,
- COOH, -CONH₂, -CONHC₁₋₈alcoyle, -CON(C₁₋₆alcoyle)₂,
- NH₂, -NHC₁₋₆alcoyle, -N(C₁₋₈alkyl)₂, -NHC₆₋₁₄aryle, -NH-Hétaryle,
- N(C₆₋₁₄ aryl)₂, -N(C₁₋₈alkyl)(C₆₋₁₄aryl),
- C₁₋₆-alcoyle, -C₂₋₁₂alcényle, -C₂₋₁₂alkinyle, chaque fois à chaîne droite, ramifié où cyclique ainsi qu'éventuellement substitués une, deux ou trois fois indépendamment l'un de l'autre par un halogène,
- halogène (-F, -CI. -Br, -I),
- CH₂CH₂OH, - CH₂CH₂SH, -CH₂CH₂SCH₃,
- sulfamoyle, alkylsulfamoyle, dialkylsulfamoyle avec alcoyle C₁₋₅ éventuellement substitué par méthoxy,
- amidino, hydroxyamidino
- sulfo, phosphono,
- CN, -NO₂, et -SCN
ainsi que des sels, des solvates, des tautomères et des prodrogues de ces liaisons acceptables pharmaceutiquement,
les liaisons avec:
-R₀ =R₁ =R₂ =R₃ =H
étant exclus de la protection.

2. Liaisons selon la revendication 1, dans lesquelles les substituants ont la signification ci-après:
R₀, R₁ au R₂ sont sélectionnés indépendamment l'un de l'autre parmi:
- chlore, brome, iode, azido, hydrazino
- L_{A}-A-L_{B}-B, où signifient:
LA: - liaison simple,
-NR^{§}, O, S, S(O)₂, C=0,
A:
- hydrogène,
- C₁₋₆alcoyle (éventuellement substitué par R^{§a}),
- C₂₋₆alcényle (éventuellement substitué par R^{§a}),
- C₂₋₆alkinyle (éventuellement substitué par R^{§a}),
- phényle, éventuellement substitué une, deux ou trois fois par des restes R^{§b} sélectionnés indépendamment l'un de l'autre un hétérocycle saturé mono- ou bicyclique ou non saturé une ou plusieurs fois avec de 4 à 10 atomes annulaires, parmi lesquels de 1 à 5 hétéroatomes (de préférence N, O et S), éventuellement substitués par un ou plusieurs atomes d'oxygène et/ou un reste R^{§b},
et au cas où A n'est plus substituable, L_{B} et B sont sans objet,
L_{A}-A ainsi que L_{A}-A-L_{B} peuvent également signifier chacun dans l'ensemble une liaison simple
L_{B}:
- liaison simple,
- NR^{§}, O, S, S(O)₂, C=O
- les groupes fonctionnels suivants: 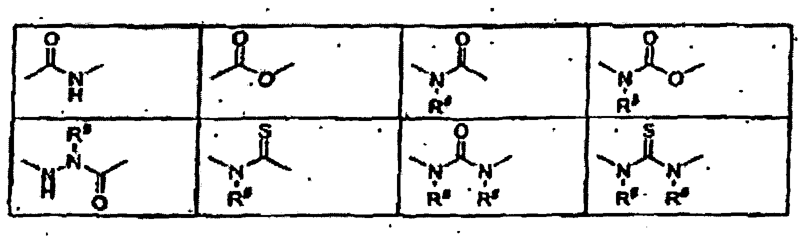
B:
- hydrogène
- alcoyle, éventuellement substitué par R^{§a}
- C₂₋₆alcényle (éventuellement substitué par R^{§a})
- C₂₋₆alkinyle (éventuellement substitué par R^{§a}),
- aryle, en particulier phényle, éventuellement substitué par R^{§b}
- un hétérocycle saturé mono- ou bicyclique ou non saturé une ou plusieurs fois avec de 4 à 10 atomes annulaires, parmi lesquels de 1 à 5 hétéroatomes (de préférence N, O, S), qui peuvent éventuellement être substitués une, deux ou trois fois par des restes R^{§b} sélectionnés indépendamment l'un de l'autre et/ou un ou plusieurs atomes d'oxygène
R^{#} - hydrogène, alcoyle (éventuellement substitué par R^{§})
R₃
- hydrogène,
- C₁₋₆alcoyle, à chaine droite, ramifié ainsi qu'éventuellement substitué une, deux ou trois fois par des restes R^{§a} sélectionnés indépendamment l'un de l'autre
- R^{§b}
- C₁₋₆alcoxy, à chaine droite, ramifié ou C₃₋₆alcoxy également cyclique ainsi qu'éventuellement substitué une, deux ou trois fois par des restes R^{§} sélectionnés indépendamment l'un de l'autre,
- un hétérocycle saturé monocyclique ou non saturé une ou plusieurs fois avec de 4 à 8 atomes annulaires, parmi lesquels de 1 à 3 hétéroatomes, de préférence N, O et S
R₄
- NR₇ R₈, où ce substituant signifie dans l'ensemble:
- morpholino, thiomorpholino, thiomorpholino-S,S,-dioxyde, pyrrolidino, piperidino, piperazin-1-yl, 1-homopiperazinyl, 4-C₁₋₆-alkyl-piperazin-1-yl, 4-aryl-1-piperazin-1-yl, 4-Bn-piperazin-1-yl (éventuellement substitué par R^{§a} à l'anneau hétérocycle-aliphatique),
- autres restes amino d'amines secondaires, mono- ou polycycliques avec en tout de 4 à 14 atomes annulaires, y compris le représentant substitué par R^{§} au squelette C
R₅
- un reste hétérocyclique saturé ou non saturé une ou plusieurs fois avec en tout de 4 à 8 atomes annulaires, parmi lesquels de 1 à 3 hétéroatomes, qui sont de préférence N, O et S, éventuellement substitués par R^{§b},
- C₁₋₆alcoxy, C₁₋₆alkylthio, chacun à chaîne droite, ramifié ou cyclique ainsi qu'éventuellement substitué par R^{§a},
- un reste hétérocyclique saturé ou non saturé une ou plusieurs fois, lié par un atome exocyclique du groupe O, N, S avec en tout de 4 à 10 atomes annulaires, parmi lesquels de 1 à 5 hétéroatomes (de préférence N, O et S), éventuellement substitués par R^{§b},
- un reste hétérocyclique saturé ou non saturé une ou plusieurs fois, lié au tricycle par un atome du groupe O, N ou S et un groupe alkylène C₁₋₆ disposé à la suite, ou non saturé une ou plusieurs fois avec en tout de 4 à 10 atomes annulaires, parmi lesquels de 1 à 5 hétéroatomes (de préférence N, O et S), éventuellement substitués par R^{§b},
- hydroxy, halogène (CI, Br, I)
R₆
- hydrogène,
- C₁₋₆alcoyle, à chaine droite ou ramifié ainsi qu'éventuellement substitué une, deux ou trois fois par des restes R^{§a} sélectionnés indépendamment l'un de l'autre,
- C₂₋₆alcényle (éventuellement substitué par R^{§a}),
- carbonyle ou sulfonyle, chacun substitué par C₁₋₈alcoyle (éventuellement substitué par R^{§a}),
R^{§a}
- OH, -OC₁₋₈alcoyle, -S-C₁₋₄alcoyle, -SO₂-C₁₋₄alcoyle, -COOH,
- COOC₁₋₈alcoyle, -CONH₂, -CONHC₁₋₆alcoyle, -CON(C₁₋₆alkyl)₂, -NH₂,
- NHC₁₋₆alcoyle, -N(C₁₋₆alkyl)₂,
- halogène (-F, -Cl, -Br, -I),
- CN, -SCN
R^{§b}
- OH, -O-C₁₋₈alcoyle,
- SO₂-C₁₋₄alcoyle, -SO₃H, -OSO₂C₁₋₆alcoyle,
- COOH, -COOC₁₋₈alcoyle,
- CONH₂, -CONHC₁₋₆alcoyle, -CON(C₁₋₆alkyl)₂,
- NH₂, -NHC₁₋₆alcoyle, -N(C₁₋₆alkyl)₂,
- C₁₋₆alcoyle, -C₂₋₆alcényle, -C₂₋₆alkinyl, chacun à chaîné droite, ramifié ou cyclique ainsi qu'éventuellement substitué une, deux ou trois fois indépendamment l'un de l'autre par un halogène,
- halogène (-F. -CI, -Br, -I),
- CH₂CH₂OH, -CH₂CH₂SCH3,
- sulfamoyle, alkylsulfamoyle, dialkylsulfamoyle, avec alcoyle C₁₋₅ éventuellement substitué par méthoxy,
- amidino, hydroxyamidino
- sulfo, phosphono,
- -CN, -N0₂, et -SCN
ainsi que des sels, solvates, tautomères et prodrogues de ces liaisons acceptables pharmaceutiquement,
les liaisons avec:
- R₀=R₁=R₂=R₃=H
étant exclus de la protection.

3. Liaisons selon la revendication 1, dans lesquelles les substituants ont la signification suivante:
R₀, R₁ ou R₂ sont sélectionnés indépendamment l'un de l'autre parmi:
- chlore, brome, iode. azido, hydrazino
- L_{A-}A-L_{B}-B, où signifient :
- L_{A}: - liaison simple, - NR^{§}, O
- A:
- hydrogène,
- C₁₋₆alcoyle (éventuellement substitué par R^{§a})
- C₂₋₆alcényle (éventuellement substitué par R^{§a}),
- C₂₋₆alkinyle (éventuellement substitué par R^{§a}),
- phényle, éventuellement substitué une, deux ou trois fois par des restes R^{§b} sélectionnés indépendamment l'un de l'autre
- un hétérocycle saturé mono- ou bicyclique ou non saturé une ou plusieurs fois avec de 4 à 10 atomes circulaires, parmi lesquels de 1 à 5 hétéroatomes (de préférence N, O, S), qui peut éventuellement être substitué par un reste R^{§b} et/ou un ou plusieurs atomes d'oxygène,
et au cas où A n'est plus substituable, L_{B} et B sont sans objet,
L_{A}-A ainsi que L_{A}-A-L_{B} peuvent chacun également signifier dans l'ensemble une liaison simple
L_{B}:
- liaison simple,
- NR^{#}, O, S, S(O)₂,C=O
- les groupes fonctionnels suivants: 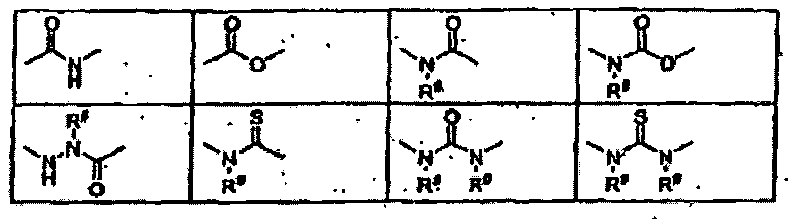
B:
- hydrogène
- alcoyle, éventuellement substitué par R^{§a}
- C₂₋₆alcényle (éventuellement substitué par R^{§a}),
- C₂₋₆alkinyle (éventuellement substitué par R^{§a}),
- aryle, en particulier phényle, éventuellement substitué par R^{§b},
- un hétérocycle saturé mono- ou bicyclique ou non saturé une ou plusieurs fois avec de 4 à 14 atomes annulaires, parmi lesquels de 1 à 5 hétéroatomes (de préférence N, O, S), qui peut éventuellement être substitué une, deux ou trois fois par des restes R^{§b} sélectionnés indépendamment l'un de l'autre et/ou un ou plusieurs atomes d'oxygène.
R^{#} -hydrogène, alcoyle (éventuellement substitué par R^{§}),
R₃
- hydrogène,
- C₁₋₆alcoyle, à chaîne droite, ou ramifié ainsi qu'éventuellement substitué une, deux ou trois fois par un halogène (F, CI),
- C₁₋₆alcoxy, à chaine droite, ou ramifié ou cyclique ainsi qu'éventuellement substitué par -OH, -OCH₃. -NH₂. -N(CH₃)₂, -COOH ou une, deux ou trois fois substitué par un halogène (F, Cl),
- un hétérocycle saturé monocyclique ou non saturé une ou plusieurs fois avec de 4 à 8 atomes annulaires, parmi lesquels de 1 à 3 hétéroatomes, de préférence N, O et S,
R₄
- NR₇ R₈, où ce substituant signifie dans l'ensemble:
- morpholino, thiomorpholino, thiomorpholino-S,S,-dioxyde, pyrrolidino, piperidino, piperazin-1-yl, 1-homopiperazinyl, 4-C₁₋₆alkyl-piperazin-1-yl, 4-aryl-1-piperazin-1-yl, 4-Bn-piperazin-1-yl (éventuellement substitué par R^{§a} à l'anneau hétérocyclo-aliphatique),
- autres restes amino d'amines secondaires, mono- ou polycycliques avec en tout de 4 à 14 atomes annulaires, y compris le représentent substitué par R^{§} au squelette C
R₅
- un reste hétérocyclique saturé ou non saturé une ou plusieurs fois avec en tout de 4 à 8 atomes annulaires, parmi lesquels de 1 à 3 hétéroatomes (de préférence N, O et S), éventuellement substitué par R^{§a},
- C₁₋₆alcoxy, C₁₋₆alkylthio, chacun à chaîne droite, ramifié ou cyclique ainsi qu'éventuellement substitué par R^{§a},
- un reste hétérocyclique saturé ou non saturé une ou plusieurs fois lié par un atome exocyclique du groupe O, N ou S avec en tout de 4 à 10 atomes annulaires, parmi lesquels de 1 à 5 hétéroatomes (qui sont de préférence N, O et S), éventuellement substitué par R^{§b},
- un reste hétérocyclique saturé ou non saturé une ou plusieurs fois lié au tricycle par un atome du groupe O, N ou S et un groupe C₁₋₆alcoyle disposé après avec en tout de 4 à 10 atomes annulaires, parmi lesquels de 1 à 5 hétéroatomes (de préférence N, O et S), éventuellement substitués par R^{§b},
- hydroxy, halogène (CI, Br, I)
R₆
- hydrogène,
- C₁₋₆alcoyle, a chaîne droite ou ramifié ainsi qu'éventuellement substitué une, deux ou trois fois par des restes R^{§a} sélectionnés indépendamment l'un de l'autre,
- C₂-₆alcényle
- carbonyle ou sulfonyle, chacun substitué par C₁₋₆alcoyle et éventuellement substitué par R^{§a},
R^{§a}
- OH, -O-C₁₋₈alcoyle, -S-C₁₋₄alcoyle, -SO₂-C₁₋₄alcoyle, -COOH, -COOC₁₋₈ alcoyle, -CONH₂, -CONHC₁₋₆alcoyle, -CON(C₁₋₆alkyl)₂, -NH₂,
- NHC₁₋₆alcoyle, -N (C₁₋₆alkyl)₂,
- halogène (-F, -Cl, -Br, -I),
- CN, -SCN
R^{§b}
- OH, -O-C₁₋₈alcoyle
- SO₂-C₁₋₄alcoyle, -SO₃H, - OSO₂ C₁₋₈alcoyle,
- COOH, -COOC₁₋₈alcoyle,
- CONH₂, -CONHC₁₋₆alcoyle, -CON(C₁₋₆alkyl)₂,
- NH₂, -NHC₁₋₆alcoyle, -N(C₁₋₆alkyl)₂,
- C₁₋₆alcoyle, -C₂₋₆alcényle, -C₂₋₆alkinyle, chacun à chaîne droite, ramifié ou cyclique ainsi qu'éventuellement substitué une, deux ou trois fois indépendamment l'un de l'autre par un halogène,
- halogène (-F. -Cl, -Br, -I),
- sulfamoyle, alkylsulfamoyle, dialkylsulfamoyle avec alcoyle C₁₋₆ éventuellement substitué par méthoxy,
- CN, -NO₂, et -SCN
ainsi que des sels, solvates, tautomères et prodrogues de ces liaisons acceptables pharmaceutiquement,
les liaisons avec:
-R₀=R₁= R₂= R₃= H
étant exclus de la protection.

4. Liaisons selon la revendication 1, sélectionnées parmi:
7-brom-4-éthoxy-9-fluor-2-(piperazin-1-yl)-5H-pyrimido[5,4-b]indol
7-brom-4-éthoxy-2-(piperazin-1-yl)-5H-pyrimido[5,4-b]indol-9-ol
4-éthoxy-2-(piperazin-1-yl)-7-(pyridin-4-yl)-5H-pyrimido[5,4-b]indol-9-ol
4-éthoxy-7-(3,4-diméthoxyphényl)-2-(piperazin-1-yl)-5H-pyrimido[5,4-b]indol-9-ol
7-(3,4-diméthoxyphényl)-2-(piperazin-1-yl)-5H-pyrimido[5,4-b]indol-4,9-diol
4-éthoxy-8-(3,4,5-triméthoxyphényl)-2-(piperazin-1-yl)-5H-pyrirnido[5,4-b]indol
1-(7-(3,4-diméthoxyphényl)-2-(piperazin-1-yl)-5H-pyrimido[5,4-b]indol-4-yl)piperidin-4-ol
7-(3.4-diméthoxyphényl)-2-(piperazin-1-yl)-5H-pyrimido[5,4-b]indol-4-ol
4-((pyridin-2-yl)méthoxy))-7-(3,4-dimethoxyphényl)-2-(piperazin-1-yl)-5H-pyrimido [5,4-b]indol
4-((pyridin-4-yl)méthoxy))-7-(3,4-diméthoxyphényl)-2-(piperazin-1-yl)-5H-pyrimido[5,4-b]indol
4-((pyridin-3-yl)méthoxy))-7-(3,4-diméthoxyphényl)-2-(piperazin-1-yl)-5H-pyrimido[5,4-b]indol
4-méthylthio-2-(piperazin-1-yl)-7-(pyridin-4-yl)-5H-pyrimido[5,4-b]indol
2-(7-(3,4-dimethoxyphényl)-2-(piperazin-1-yl)-5H-pyrimido[5,4-b]indol-4-ylamino) éthanol
7-brom-2-(piperazin-1-yl)-5H-pyrirnido[5,4-b]indol-4-ol
4-(2-morpholinoéthoxy))-7-(3,4-diméthoxyphényl)-2-(piperazin-1-yl)-5H-pyrimido [5,4-b]indol
7-(3,4-diméthoxyphényl)-4-morpholino-2-(piperazin-1-yl)-5H-pyrimido[5,4-b]indol
7-(3,4-diméthoxyphényl)-2-(piperazin-1-yl)-4-thiomorpholino-5H-pyrimido [5,4-b]indol
4-Morpholino-2-(piperazin-1-yl)-7-(pyrindin-4-yl)-5H-pyrimido[5,4-b]indol
7-(3,4-diméthoxyphényl)-N-(2-morpholinoéthyl)-2-(piperazin-1-yl)-5H-pyrimido[5,4-b]indol-4-amine
7-(3,4-diméthoxyphényl)-2-(piperazin-yl)-4-piperidino-5H-pyrimido[5,4-b]indol
4-cyclopropylméthoxy-7-(3,4-diméthoxyphényl)-2-(piperazin-1-yl)-5H-pyrimido[5,4-b]indol
4-(1H-imidazol-1-yl)-7-(3,4-diméthoxyphényl)-2-(piperazin-1-yl)-5H-pyrimldo[5,4-b]indol
7-(3,4-diméthoxyphényl)-2-(piperazin-1-yl)-4-(piperidin-4-yloxy)-5H-pyrimido[5,4-b]indol
4-cyclopropylméthoxy-2-(piperazin-1-yl)-7-(pyridine-4-yl)-5H-pyrimido[5,4-b]indol
4-éthoxy-2-morpholino-7-(pyridine-4-yl)-5H-pyrimido[5,4-b]indol
4-(4-éthoxy-2-(piperazin-1-yl)-5H-pyrimido[5,4-b]indol-7-yl)benzoate d'éthyle
4-(4-éthoxy-2-(piperazin-1-yl)-5H-pyrimido[5,4-b]indol-7-yl)hydrochlorure de benzoyle
4-(4-éthoxy-2-(piperazin-1-yl)-5H-pyrimido[5,4-b]indol-7-yl)hydrochlorure phénolique
3-(4-éthoxy-2-(piperazin-1-yl)-5H-pyrimido[5,4-b]indol-7-yl)benzoate de méthyle
3-(4-éthoxy-2-(piperazin-1-yl)-5H-pyrimido[5,4-b]indol-7-yl) hydrochlorure de benzoyle
4-éthoxy-7-(furan-2-yl)-2-(piperazin-1-yl)-5H-pyrimido[5,4-b]indol
tert-butyl-2-(4-éthoxy-2-(piperazin-1-yl)-5H-pyrimido[5,4-b]indol-7-yl)-1H-pyrrol-1-carboxylate
7-(benzo[d][1,3]dioxol-5-yl)-4-éthoxy-2-(piperazin-1-yl)-5H-pyrimido[5,4-b]indol
7-(3,4-diméthoxyphényl)-2-(piperazin-1-yl)-N-(thiazol-2-yl)-5H-pyrimido[5,4-b]indol-4-amine
7-(3,4-diméthoxyphényl)-2-(piperazin-1-yl)-4-(1H-pyrrol-1-yl)-5H-pyrimido[5,4-b]indol
7-(3,4-diméthoxyphényl)-2-(piperazin-1-yl)-4-(1H-pyrazol-1-yl)-5H-pyrimido[5,4-b]indol
7-(3,4-diméthoxyphényl)-2-(piperazin-1-yl)-4-(1H-1,2,3-triazol-1-yl)-5H-pyrimido[5,4-b]indol
7-(3,4-diméthoxyphényl)-2-(piperazin-1-yl)-4-(4H-1,2,4-triazol-4-yl)-5H-pyrimido[5,4-b]indol
7-(3,4-diméthoxyphényl)-2-(piperazin-1-yl)-4-(pyrrolidin-1-yl)-5H-pyrimido[5,4-b]indol
(4-(4-éthoxy-2-(piperazin-l-yl)-5H-pyrimido[5,4-b]indol-7-yl)piperazin-1-yl)(phényl)méthanone
4-éthoxy-2-(piperazin-1-yl)-7-(4-(pyrimidin-2-yl)piperazin-1-yl)-5H-pyrimido[5,4-b]indol
4-éthoxy-2-(piperidin-4-yl)-7-(pyridin-4-yl)-5H-pyrimido[5,4-b]indol
4-éthoxy-7-(3,4-diméthoxyphényl)-2-(piperidin-4-yl)-5H-pyrimido[5,4-b]indol
4-éthoxy-7-(3,4-diméthoxyphényl)-2-(piperidin-3-yl)-5H-pyrimido[5,4-b]indol
2-(4-éthoxy-9-méthoxy-2-(piperazin-1-yl)-5H-pyrimido[5,4-b]indol-7-yl)benzèneamine
2-(2-(piperazin-1-yl)-7-(pyridin-4-yl)-5H-pyrimido[5,4-b]indol-4-yloxy)éthanol
4-éthoxy-2(4-méthylpiperazin-1-yl)-7-(pyridin-4-yl)-5H-pyrimido[5,4-b]indol
4-éthoxy-2-(piperazin-1-yl)-6-(pyridin-4-yl)-5H-pyrimido[5,4-b]indol
4-éthoxy-2-(piperazin-1-yl)-7-(2-aminopyridin-5-yl)-5H-pyrimido[5,4-b]indol
4-éthoxy-2-(piperazin-1-yl)-7-(3,4-diméthoxy-phényl)-5H-pyrimido[5,4-b]indol
4-éthoxy-2-(piperazin-1-yl)-7-(pyridin-3-yl)-5H-pyrimido[5,4-b]indol
4-éthoxy-2-(piperazin-1-yl)-7-(pyridin-4-yl)-5H-pyrimido[5,4-b]indol
4-éthoxy-2-(piperazin-1-yl)-8-(pyridin-4-yl)-5H-pyrimido[5,4-b]indol
4-éthoxy-7-morpholino-2-(piperazin-1-yl)-5H-pyrimido[5,4-b]indol
4-éthoxy-9-méthoxy-2-(piperazin-1-yl)-7-(pyridin-4-yl)-5H-pyrimido[5,4-b]indol

5. Composition pharmaceutique contenant une ou plusieurs liaisons selon l'une des revendications 1 à 4 ainsi que des agents auxiliaires et porteurs habituels.

6. Composition pharmaceutique selon la revendication 5, présentant par ailleurs une ou plusieurs des substances actives suivantes:
- analogues nucléosides (par exemple fludarabine, cladribine)
- agents alkylants (par exemple chlorambucil, cyclophosphamide)
- agonistes de β₂-adrénocepteurs (par exemple terbutaline, salbutanol, salmétanol, fénotérol, formotérol)
- corticostéroïdes
- antagonistes de leucotriènes (soit des inhibiteurs d'enzymes [tels qu'inhibiteurs de lipoxygénase ou inhibiteurs d'enzyme d'acide arachidonique], soit des antagonistes de récepteurs), par exemple pranlukast, montelukast, zafirlukast, zileutone
- antihistaminiques (de préférence ceux avec des propriétés de stabilisation de mastocytes ou à aspect d'antagonisation de leucotriènes, tels que par exemple loratadine, astemizol, mizolastine, olopatadine
- théophylline
- inhibiteurs de PDE
- anticorps (monoclonaux) contre les TNF-alpha ou autres substances actives qui inhibent la formation ou la libération de TNF-alpha ou l'activité de TNF-alpha (par exemple constructs de récepteurs solvants recombinants)
- anticorps (monoclonaux) (par exemple rituximab, TACl-lg)

7. Une ou plusieurs liaisons selon l'une des revendications 1 à 4 destinées à être utilisées dans le traitement de tumeurs malignes non solides du système de formation sang.

8. Liaison selon la revendication 7 destinée à être utilisée dans le traitement de leucémies et de lymphomes.

9. Liaison selon la revendication 8 destinée à être utilisée dans le traitement de leucémie lymphatique chronique de type de cellule B.

10. Liaison selon la revendication 9 destinée à être utilisée dans le traitement de leucémie lymphatique chronique qui est accompagnée de délétions chromosomiques.

11. Liaison selon la revendication 10, la délétion chromosomique étant une délétion 11q.

12. Procédé pour la préparation de liaisons selon l'une des revendications 1 à 4, présentent les étapes de procédé suivantes: 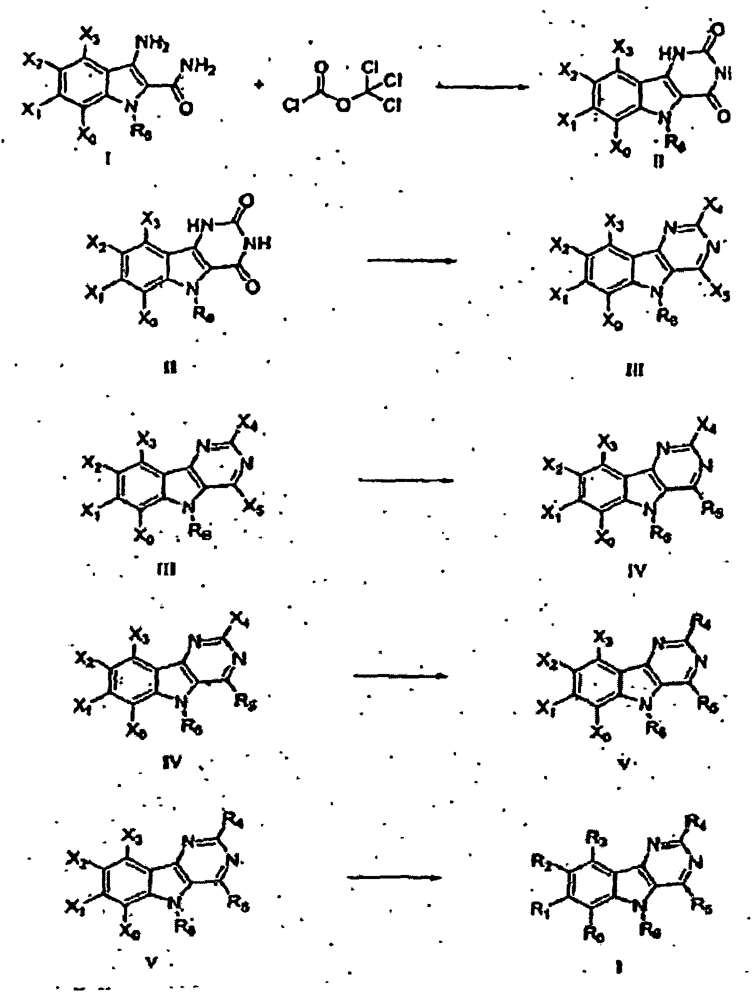 X₀-X₅= indépendamment égaux ou inégaux à F, Cl, Br, I, H, X₄, X₅ étant inégaux à H
• Transformation des liaisons de la formule générale 1 par des dérivés de phosgène, de préférence disphosgène, dans un solvant approprié, de préférence dioxane ou toluol, pour obtenir des liaisons de la formule générale II.
• Transformation des liaisons de la formule générale II par un agent d'halogénisation, de préférence oxyde de dichlorphénylphosphine, trichlorure de phosphore, pentachlorure de phosphore, oxychlorure de phosphore ou leurs mélanges, chaque fois à la chaleur, pour obtenir des liaisons de la formule générale III
• Transformation des liaisons de la formule générale III par des nucléophyles d'O, N, S ou C, de préférence des alcoolates, amines et thiolates, dans des alcanols ou éventuellement des solvants aprotiques, dipolaires sous échauffement, dans des cas exceptionnels également à température ambiante, pour obtenir des liaisons tricycliques de la formule générale IV
• Transformation des liaisons de la formule générale IV par des nucléophyles d'O, N, S ou C, de préférence des alcoolates, amines et thiolates dans un solvant approprié, de préférence toluol, mésitylène ou dioxane sous échauffement, dans des cas exceptionnels également à température ambiante, pour obtenir des liaisons de la formule générale V
• Transformation des liaisons de la formule générale V par des nucléophyles d'O, N, S ou C dans un solvant approprié, de préférence toluol, dioxane ou THF, sous échauffement et à l'aide de catalyseurs appropriés, dans des cas exceptionnels à l'aide d'une micro-onde, pour obtenir des liaisons de la formule générale 1
• L'introduction des restes R₀, R₁, R₂, R₄, R₅ et R₆ peut chaque fois avoir lieu à différents étages de synthèse, au moyen de réactions appropriées, en particulier de réactions de couplage croisé C-C catalysées par un métal, et éventuellement à l'aide de groupes de protection appropriés.
